# EUROPEAN PATENT SPECIFICATION

(11) **EP 3 710 032 B1**
(45) Date of publication and mention of the grant of the patent: **17.12.2025**
(21) Application number: 18878173.6
(22) Date of filing: 15.11.2018
(51) Int. Cl.: C12Q 1/6881, C12Q 1/6883, C12Q 1/6886, A61K 38/00, C07K 14/705, G01N 33/49

(54) **BIOMARKER PROXY TESTS AND METHODS FOR STANDARD BLOOD CHEMISTRY TESTS**
BIOMARKER-PROXY-TESTS UND VERFAHREN FÜR STANDARD-BLUTANALYSE
TESTS DE BIOMARQUEURS DE SUBSTITUTION ET PROCÉDÉS DE TESTS DE CHIMIE SANGUINE STANDARD

(30) Priority: 15.11.2017 US 201762586301 P
(43) Date of publication of application: 23.09.2020
(73) Proprietor: The Translational Genomics Research Institute, Phoenix, Arizona 85004 (US)
(72) Inventor: HUENTELMAN, Matthew, Phoenix Arizona 85004 (US); MCDANIEL, Timothy, Phoenix Arizona 85004 (US); NAYMIK, Marcus, Phoenix Arizona 85004 (US)
(74) Representative: Barker Brettell LLP
(86) International application number: PCT/US2018/061394
(87) International publication number: WO 2019/099753

(56) References cited:
- WO-A1-2015/179952
- WO-A2-2004/072265
- WO-A2-2011/132872
- WO-A2-2013/100822
- US-A- 5 858 649
- US-A1- 2016 265 056
- PALMER CHANA ET AL: "Cell-type specific gene expression profiles of leukocytes in human peripheral blood", BMC GENOMICS, BIOMED CENTRAL, vol. 7, no. 1, 16 May 2006 (2006-05-16), pages 115, XP021014482, ISSN: 1471-2164, DOI: 10.1186/1471-2164-7-115
- WATKINS NICHOLAS A. ET AL: "A HaemAtlas: characterizing gene expression in differentiated human blood cells", vol. 113, no. 19, 7 May 2009 (2009-05-07), US, pages e1 - e9, XP055818703, ISSN: 0006-4971, Retrieved from the Internet <URL:http://ashpublications.org/blood/article-pdf/113/19/e1/1485741/zh8019090000e1.pdf> DOI: 10.1182/blood-2008-06-162958
- JOEHANES ROBY ET AL: "Gene expression analysis of whole blood, peripheral blood mononuclear cells, and lymphoblastoid cell lines from the Framingham Heart Study", PHYSIOLOGICAL GENOMICS, vol. 44, no. 1, 1 November 2011 (2011-11-01), US, pages 59 - 75, XP055819625, ISSN: 1094-8341, DOI: 10.1152/physiolgenomics.00130.2011
- MCDADE THOMAS W. ET AL: "Genome-Wide Profiling of RNA from Dried Blood Spots: Convergence with Bioinformatic Results Derived from Whole Venous Blood and Peripheral Blood Mononuclear Cells", vol. 62, no. 2, 3 May 2016 (2016-05-03), pages 182 - 197, XP055818819, ISSN: 1948-5565, Retrieved from the Internet <URL:https://www.ncbi.nlm.nih.gov/pmc/articles/PMC4972449/pdf/nihms804587.pdf> DOI: 10.1080/19485565.2016.1185600
- BYBJERG-GRAUHOLM JONAS ET AL: "RNA sequencing of archived neonatal dried blood spots", MOLECULAR GENETICS AND METABOLISM REPORTS, vol. 10, 24 December 2016 (2016-12-24), pages 33 - 37, XP055819459, ISSN: 2214-4269, DOI: 10.1016/j.ymgmr.2016.12.004
- RUSSOM AMAN ET AL: "Microfluidic Leukocyte Isolation for Gene Expression Analysis in Critically Ill Hospitalized Patients", vol. 54, no. 5, 1 May 2008 (2008-05-01), US, pages 891 - 900, XP055819709, ISSN: 0009-9147, Retrieved from the Internet <URL:https://academic.oup.com/clinchem/article-pdf/54/5/891/32671693/clinchem0891.pdf> DOI: 10.1373/clinchem.2007.099150

## Description

### FIELD OF THE INVENTION

The present disclosure relates to blood tests and proxy methods of conducting standard blood tests using genetic markers, for example, a complete blood count, comprehensive metabolic panel, chemistry panel, and thyroid-related blood tests (thyroxine, T3, and TSH levels).

### BACKGROUND OF THE INVENTION

Blood tests offer a variety of information for the diagnosis of diseases or conditions or maintenance of a subject's health. A well-chosen complement of blood tests, such as a complete blood count panel, comprehensive metabolic panel, or chemistry panel, can thoroughly assess one's overall state of health, as well as detect the silent warning signals that precede the development of serious diseases such as diabetes and heart disease. However, the current technology for conducting blood tests requires more than a few drops of blood. These tests require venipuncture to obtain cells and extracellular fluid (plasma) from the body for analysis. Although minimally invasive, venipuncture still requires a technician, and thus these tests cannot be performed without visiting a laboratory, whether one within a hospital or clinic or a standalone testing site. Another limitation of these tests is that for each test conducted, often at least one tube of blood collection required. For example, if a patient has orders for a complete blood count panel, comprehensive metabolic panel, and thyroid-related tests, it can require the collection of four tubes of blood. With the increased frequency of blood test monitoring, the subject can develop iatrogenic anemia, which is low red blood cell counts due to too much removal of blood. The amount of blood collected and the need to visit a laboratory for blood collection are significant obstacles for greater use of these tests as monitors of one's state of health. Thus, more convenient alternatives for obtaining the same results as standard blood tests are needed.

McDade et al. (2016), Biodem Soc Biol 62(2):182-197, and Bybjerg-Grauholm et al. (2017), Mol Gen Metab Rep 10(1):33-37, both relate to analysis of RNA from dried blood spots, whereas WO2011/132872 extracts RNA from a small blood volume and analyses the mRNA level of IFN-gamma in the blood sample.

### SUMMARY OF THE INVENTION

One aspect of the invention is directed to a method of performing a blood test according to claim 1. The method of performing the blood test generally includes extracting RNA from a blood sample; determining an mRNA level associated with a predictive gene in the blood sample; and converting the mRNA level into a blood test result for a target blood component, wherein the mRNA level of the predictive gene in the blood sample relates to the target blood component. In certain aspects, the method further includes selecting the predictive gene.

In an exemplary embodiment, the method comprises: extracting an RNA from a blood sample; selecting a predictive gene, wherein an mRNA level of the predictive gene in the blood sample relates a target blood component; determining the mRNA level of the predictive gene in the blood sample; and converting the mRNA level into a blood test result of the target blood component.

In the method of claim 1, the blood sample is plasma, or dried blood spot. In those embodiments wherein the blood sample is a dried blood spot, the quality of the dried blood spot may be determined by assessing quality of the extracted RNA.

Other exemplary aspects of the invention, the blood sample has a volume in the range of: 10 µl - 3 ml, 10 µl-2.5 ml, 15 µl-2.5 ml, 15 µl-2 ml, 20 µl-2 ml, 25 µl-2 ml, 25 µl-1.5 ml, 30 µl-1.5 ml, 30 µl-1 ml, 10-300 µl, 10-250 µl, 15-250 µl, 15-200 µl, 20-200 µl, 25-200 µl, 25-150 µl, 30-150 µl, or 30-100 µl. In further particular aspects, the blood sample has a volume of between 10 µl and 1 ml or a volume of between 10-100 µl.

The mRNA level can be determined using many methods, for example, RNA sequencing, quantitative PCR, and hybridization. In certain preferred embodiments, the mRNA level is determined using next-generation sequencing and normalized using DESeq2 algorithm or edgeR algorithm.

In an exemplary embodiment, the blood test is reported as an amount of the target blood component; a concentration of the target blood component; a volume of the target blood component; a distribution of the target blood component; a ratio of the target blood component to a second blood component; or combinations thereof.

In one specific embodiment, the blood test is reported as a volume ratio of red blood cells to total blood (hematocrit level). In other aspects, the blood test is reported as a volume ratio of mean corpuscular hemoglobin (MCH) to mean corpuscle (cell) (MCV) (mean corpuscular hemoglobin concentration (MCHC)).

The method of claim 1 targets the blood component Prostate-Specific Antigen (PSA_total). Further examples of targeted blood components, which are herein disclosed but not part of the invention, include: Absolute Basophils, Absolute Eosinophil, Absolute Lymphocyte, Absolute Monocyte, Absolute Neutrophil, Alanine Aminotransferase, Albumin, Alkaline Phosphatase, Anion Gap, Aspartate Aminotransferase, Total Bilirubin, Blood Urea Nitrogen (BUN), Calcium, Chloride, Cholesterol, CO2, Creatinine, Eosinophils, Gamma-Glutamyl Transferase (GGT), Globulin, Glucose, HDL Cholesterol, Hemoglobin, Immature Granulocyte, Lactic Dehydrogenase, LDL Cholesterol, Lymphocytes, mean corpuscular hemoglobin (MCH), mean corpuscle (cell) volume (MCV), Monocytes, mean platelet volume (MPV), Non-HDL Cholesterol, Osmolality, Inorganic Phosphorus, Platelet Count, Potassium, Total Protein, Red Blood Cell (RBC), red cell distribution width (RDW), Segmented Neutrophils, Sodium, Total T3, T3 Uptake, T7 Index, Thyroxine (T4), Triglycerides, Thyroid Stimulating Hormone (TSH), Uric Acid, VLDL Cholesterol, and White Blood Cell (WBC).

In disclosed embodiments, the blood sample is whole blood, plasma, dried blood spot, or combinations thereof, and the target blood component is selected from the group consisting of: Segmented Neutrophils, Eosinophils, Prostate-Specific Antigen, red blood cells, monocytes, creatinine, lymphocytes, eosinophil, alanine aminotransferase, electrolytes, and non-HDL cholesterol.

In other embodiments, the blood test includes: Prostate-Specific Antigen (PSA_total), Red Blood Cell count (RBC_m.mm3), Absolute Eosinophil, Anion Gap (AG), red cell distribution width (RDW_sd), Thyroid Index (T7), or combinations thereof.

In a particular non-limiting embodiment, converting the mRNA level into a blood test result uses the following formula: blood test result = C + C₁*(gene), C and C₁ are constants, and (gene) represents the mRNA level of the predictive gene. In particular preferred embodiment, the mRNA level is normalized gene count.

In a disclosed but not claimed embodiment, the target blood component is Segmented Neutrophils and the predictive gene is: MNDA, STX3, TNFRSF1A, MSL1, or TLR1. In a specific exemplary aspects, for MNDA, C is: 21.7-40.3, 21.7-37.2, 24.8-37.2, 24.8-34.1, and 27.9-34.1; and C₁ is: 21.4-39.7, 21.4-36.6, 24.4-36.6, 24.4-33.6, and 27.5-33.6; for STX3: C is 23.1-43.0, 23.1-39.7, 26.4-39.7, 26.4-36.4, and 29.8-36.4; and C₁ is: 19.9-36.9, 19.9-34.1, 22.7-34.1, 22.7-31.3, and 25.6-31.3; for TNFRSF1A: C is: 20.8-38.6, 20.8-35.7, 23.8-35.7, 23.8-32.7, and 26.8-32.7, and C₁ is: 22.2-41.3, 22.2-38.1, 25.4-38.1, 25.4-35.0, and 28.6-35.0; for MSL1: C is: 20.1-37.3, 20.1-34.5, 23.0-34.5, 23.0-32.7, and 25.9-31.6, and C₁ is: 22.9-42.5, 22.9-39.3, 26.2-39.3, 26.2-36.0, and 29.5-36.0; for TLR1: C is: 24.9-46.3, 24.9-42.8, 28.5-42.8, 28.5-39.2, and 32.1-39.2, and C₁ is: 18.2-33.7, 18.2-31.1, 20.8-31.1, 20.8-28.5, and 23.3-28.5.

In a disclosed but not claimed embodiment, the blood sample is whole blood, the target blood component is Eosinophils, the predictive gene is: SLC29A1, SIGLEC8, IL5RA, TMIGD3, or SMPD3. In a further specific exemplary embodiment, for SLC29A1: C is: between -0.57 and -0.31, between -0.52 and -0.31, between -0.52 and -0.35, between -0.48 and -0.35, and between -0.48 and -0.39, and C₁ is: 2.19-4.07, 2.19-3.75, 2.50-3.75, 2.50-3.44, and 2.81-3.44; for SIGLEC8: C is: 0.34-0.62, 0.34-0.57, 0.38-0.57, 0.38-0.53, and 0.43-0.53; and C₁ is: 1.6-2.9, 1.6-2.7, 1.8-2.7, 1.8-2.5, and 2.0-2.5; for IL5RA: C is: between -0.124 and -0.067, between -0.115 and -0.067, between -0.115 and -0.076, between -0.105 and -0.076, and between -0.105 and -0.086, *etc.,* and C₁ is: 2.0-3.7, 2.0-3.4, 2.2-3.4, 2.2-3.1, and 2.5-3.1; for TMIGD3: C is: between -0.00104 and -0.00056, between -0.00096 and -0.00056, between -0.00096 and -0.00064, between -0.00088 and -0.00064, and between -0.00088 and -0.00072, and C₁ is: 1.8-3.4, 1.8-3.2, 2.1-3.2, 2.1-2.9, and 2.4-2.9; for SMPD3: C is: 0.11-0.20, 0.11-0.18, 0.12-0.18, 0.12-0.17, and 0.14-0.17, and C₁ is: 1.8-3.3, 1.8-3.1, 2.0-3.1, 2.0-2.8, and 2.3-2.8.

In the method of claim 1, the blood sample may be dried blood spot, the target blood component is PSA_total, the predictive gene is: CTC-265F19.1, ADAM9, RAB11FIP5, SNAPC4, or LMNA. In a further specific exemplary embodiment, for CTC-265F19.1: C is: 0.30-0.56, 0.30-0.52, 0.35-0.52, 0.35-0.48, and 0.39-0.48, and C₁ is: 0.37-0.68, 0.37-0.63, 0.42-0.63, 0.42-0.58, and 0.47-0.58; for ADAM9, C is: 0.30-0.56, 0.30-0.52, 0.35-0.52, 0.35-0.48, and 0.39-0.48, and C₁ is: 1.2-2.2, 1.2-2.0, 1.3-2.0, 1.3-1.9, and 1.5-1.9; for RAB11FIP5: C is: 0.31-0.58, 0.31-0.53, 0.36-0.53, 0.36-0.49, and 0.40-0.49, and C₁ is: 0.42-0.77, 0.42-0.71, 0.48-0.71, 0.48-0.65, and 0.53-0.65; for SNAPC4: C is: 0.31-0.58, 0.31-0.53, 0.36-0.53, 0.36-0.49, and 0.40-0.49, and C₁ is: 0.43-0.80, 0.43-0.74, 0.49-0.74, 0.49-0.67, and 0.55-0.67; for LMNA, C is: 0.29-0.53, 0.29-0.49, 0.33-0.49, 0.33-0.45, and 0.37-0.45, and C₁ is: 0.24-0.45, 0.24-0.42, 0.28-0.42, 0.28-0.38, and 0.31-0.38.

In disclosed but not claimed embodiments, the blood sample is dried blood spot, the target blood component is Eosinophils, the predictive gene is: SCARNA22, SNORA36C, SNORA11, RN7SL4P, or SNHG15. In a further specific exemplary embodiment, for SCARNA22: C is: 0.9-1.7, 0.9-1.6, 1.0-1.6, 1.0-1.4, and 1.2-1.4, and C₁ is: 1.1-2.0, 1.1-1.8, 1.2-1.8, 1.2-1.7, and 1.4-1.7; for SNORA36C: C is: 0.9-1.7, 0.9-1.6, 1.1-1.6, 1.1-1.5, and 1.2-1.5, and C₁ is: 1.0-1.9, 1.0-1.8, 1.2-1.8, 1.2-1.6, and 1.3-1.6, for SNORA11: C is: 0.9-1.6, 0.9-1.5, 1.0-1.5, 1.0-1.4, and 1.1-1.4, and C₁ is: 1.0-1.9, 1.0-1.7, 1.2-1.7, 1.2-1.6, and 1.3-1.6; for RN7SL4P: C is: 0.7-1.4, 0.7-1.3, 0.8-1.3, 0.8-1.2, and 1.0-1.2, and C₁ is: 1.1-2.0, 1.1-1.9, 1.3-1.9, 1.3-1.7, and 1.4-1.7; for SNHG15, C is: 1.0-1.8, 1.0-1.7, 1.1-1.7, 1.1-1.5, and 1.3-1.5, and C₁ is: 1.0-1.8, 1.0-1.6, 1.1-1.6, 1.1-1.5, and 1.2-1.5.

In the method of claim 1, the blood sample may be plasma, the target blood component is PSA_total, the predictive gene is: HNRNPA3P3, GTF3A, RP11-342M1.6, HNRNPLP2, and RPS11P5. In a further specific exemplary embodiment, for HNRNPA3P3: C is: 0.15-0.27, 0.15-0.25, 0.17-0.25, 0.17-0.23, and 0.19-0.23, and C₁ is: 0.33-0.61, 0.33-0.56, 0.38-0.56, 0.38-0.52, and 0.42-0.52; for GTF3A: C is: between -0.48 and -0.26, between -0.45 and -0.26, between -0.45and -0.30, between -0.41 and -0.30, and between - 0.41 and -0.34, C₁ is: 0.7-1.3, 0.7-1.2, 0.8-1.2, 0.8-1.1, and 0.9-1.1; for RP11-342M1.6: C is: 0.28-0.52, 0.28-0.48, 0.32-0.48, 0.32-0.44, and 0.36-0.44; and C₁ is: 0.23-0.42, 0.23-0.39, 0.26-0.39, 0.26-0.36, and 0.29-0.36. In further aspects, for HNRNPLP2: C is: 0.23-0.43, 0.23-0.39, 0.26-0.39, 0.26-0.36, and 0.30-0.36; and C₁ is: 0.22-0.41, 0.22-0.38, 0.25-0.38, 0.25-0.35, and 0.29-0.35. In yet further aspects, for RPS11P5: C is: 0.17-0.32, 0.17-0.29, 0.20-0.29, 0.20-0.27, and 0.22-0.27; and C₁ is: 0.34-0.64, 0.34-0.59, 0.39-0.59, 0.39-0.54, and 0.44-0.54.

In disclosed but not claimed embodiments, the blood sample is plasma, the blood test is Red Blood Cell count (RBC_m.mm3), the predictive gene is: UTY, DDX3Y, ZFY, TXLNGY, and RPS4Y1. In a further specific exemplary embodiment, for UTY: C is: 3.1-5.8, 3.1-5.4, 3.6-5.4, 3.6-4.9, and 4.0-4.9, and C₁ is: 0.24-0.45, 0.24-0.41, 0.28-0.41, 0.28-0.38, and 0.31-0.38; for DDX3Y: C is: 3.1-5.8, 3.1-5.4, 3.6-5.4, 3.6-4.9, and 4.0-4.9, and C₁ is: 0.23-0.43, 0.23-0.40, 0.27-0.40, 0.27-0.37, and 0.30-0.37; for ZFY: C is: 3.1-5.8, 3.1-5.4, 3.6-5.4, 3.6-4.9, and 4.0-4.9, and C₁ is: 0.23-0.43, 0.23-0.40, 0.26-0.40, 0.26-0.36, and 0.30-0.36; for TXLNGY: C is: 3.2-5.9, 3.2-5.4, 3.6-5.4, 3.6-5.0, and 4.1-5.0; and C₁ is: 0.23-0.42, 0.23-0.39, 0.26-0.39, 0.26-0.36, and 0.29-0.36; for RPS4Y1: C is: 3.2-5.9, 3.2-5.4, 3.6-5.4, 3.6-5.0, and 4.1-5.0, and C₁ is: 0.22-0.42, 0.22-0.38, 0.26-0.38, 0.26-0.35, and 0.29-0.35.

In yet another example, converting the mRNA level into the blood test result uses the following formula: blood test result = C + C₁*(gene₁) + C₂*(gene₂) + ... + Cₙ*(geneₙ), n is 1, 2, 3, 4, or 5, C, C₁, C₂, ... and Cₙ are constants, and (gene₁), (gene₂), ..., and (geneₙ) represent the mRNA level of gene₁, gene₂, ..., and geneₙ. In particular embodiments, the mRNA level is the normalized gene count.

In a disclosed but not claimed embodiment, the blood sample is whole blood, the target blood component is Segmented Neutrophils, gene₁ is RNF24, gene₂ is MNDA, and gene₃ is WIPF1. In some aspects, C is: 19.7-36.6, 19.7-33.8, 22.5-33.8, 22.5-31.0, and 25.4-31.0; C₁ is: 4.6-8.6, 4.6-7.9, 5.3-7.9, 5.3-7.3, and 5.9-7.3; C₂ is: 7.4-13.8, 7.4-12.7, 8.5-12.7, 8.5-11.7, and 9.5-11.7; and C₃ is: 11.6-21.5, 11.6-19.8, 13.2-19.8, 13.2-18.2, and 14.9-18.2.

In yet another disclosed but not claimed embodiment, the blood sample is whole blood, the target blood component is Lymphocytes, gene₁ is GRB2, gene₂ is MNDA, and gene₃ is NFAM1, C is: 43.0-79.8, 43.0-73.6, 49.1-73.6, 49.1-67.5, and 55.2-67.5; C₁ is: between -18.8 and -10.1, between -17.3 and -10.1, between -17.3 and -11.5, between -15.9 and -11.5, and between - 15.9 and -13.0; C₂ is: between -11.1 and -6.0, between -10.2 and -6.0, between -10.2 and -6.8, between -9.4 and -6.8, and between -9.4 and -7.7; and C₃ is: between -13.0 and -7.0, between -12.0 and -7.0, between -12.0 and -8.0, between -11.0 and -8.0, and between -11.0 and -9.0.

In further disclosed but not claimed embodiments, the blood sample is whole blood, the target blood component is Monocytes, gene₁ is NAGA, gene₂ is RIN2, gene₃ is ADA2, gene₄ is PLXNB2, and gene₃ is ANXA2, C is: between -1.9 and -1.0, between -1.8 and -1.0, between -1.8 and - 1.2, between -1.6 and -1.2, and between -1.6 and -1.3, *etc;* C₁ is: 1.8-3.4, 1.8-3.2, 2.1-3.2, 2.1-2.9, and 2.4-2.9; C₂ is: 2.2-4.2, 2.2-3.8, 2.6-3.8, 2.6-3.5, and 2.9-3.5; C₃ is: 2.9-5.5, 2.9-5.0, 3.4-5.0, 3.4-4.6, and 3.8-4.6; C₄ is: between -3.9 and -2.1, between -3.6 and -2.1, between - 3.6 and -2.4, between -3.3 and -2.4, and between -3.3 and -2.7; and C₅ is: 1.2-2.2, 1.2-2.0, 1.4-2.0, 1.4-1.9, and 1.5-1.9.

In still further disclosed but not claimed embodiments, the blood sample is plasma, the target blood component is Absolute Eosinophil, gene₁ is CLC, gene₂ is ADAT1, gene₃ is SNRPEP4, and gene₄ is GPC6, C is: 0.0021-0.0039, 0.0021-0.0036, 0.0024-0.0036, 0.0024-0.0033, and 0.0027-0.0033; C₁ is: 0.041-0.075, 0.041-0.070, 0.046-0.070, 0.046-0.064, and 0.052-0.064; C₂ is: 0.078-0.144, 0.078-0.133, 0.089-0.133, 0.089-0.122, and 0.100-0.122; C₃ is: between - 0.035 and -0.019, between -0.032 and -0.019, between -0.032 and -0.022, between -0.030 and -0.022, and between -0.030 and -0.024; and C₄ is: 0.012-0.022, 0.012-0.020, 0.014-0.020, 0.014-0.019, and 0.015-0.019.

In another disclosed but not claimed emboidment, the blood sample is plasma, the blood test is Anion Gap (Anion.Gap, AG), gene₁ is DHX40, gene₂ is SLC1A4, gene₃ is IMPA2, gene₃ is KATNA1, and gene₃ is MEIS3P1, C is: 5.9-11.0, 5.9-10.2, 6.8-10.2, 6.8-9.3, and 7.6-9.3; C₁ is: 1.7-3.2, 1.7-2.9, 1.9-2.9, 1.9-2.7, and 2.2-2.7; C₂ is: between -1.3 and -0.7, between -1.2 and -0.7, between -1.2 and -0.8, between -1.1 and -0.8, and between -1.1 and -0.9; C₃ is: 0.9-1.6, 0.9-1.5, 1.0-1.5, 1.0-1.4, and 1.1-1.4; C₄ is: 1.2-2.2, 1.2-2.0, 1.3-2.0, 1.3-1.8, and 1.5-1.8; and C₅ is: 0.35-0.66, 0.35-0.61, 0.40-0.61, 0.40-0.56, and 0.46-0.56.

In some disclosed but not claimed embodiments, the blood sample is plasma, the target blood component is Segmented Neutrophils, gene₁ is RXFP1, gene₃ is POLR3GL, gene₃ is FOXK2, and gene₄ is LAMB1, C is: 41.0-76.1, 41.0-70.2, 46.8-70.2, 46.8-64.4, and 52.7-64.4; C₁ is: 1.5-2.8, 1.5-2.5, 1.7-2.5, 1.7-2.3, and 1.9-2.3; C₂ is: between -7.1 and -3.8, between -6.5 and -3.8, between -6.5 and -4.4, between -6.0 and -4.4, and between -6.0 and -4.9; C₃ is: 3.6-6.6, 3.6-6.1, 4.1-6.1, 4.1-5.6, and 4.6-5.6; and C₄ is: 1.6-2.9, 1.6-2.7, 1.8-2.7, 1.8-2.4, and 2.0-2.4.

In other disclosed but not claimed embodiments, the blood sample is whole blood or plasma, the blood test is red blood cell distribution width (RDW_sd), gene₂ is CHCHD2P6 from plasma, gene₃ is SEC63P1 from plasma, gene₃ is DNAL1 from whole blood, and gene₄ is ENSG00000197262 from whole blood, C is: 26.2-48.7, 26.2-44.9, 30.0-44.9, 30.0-41.2, and 33.7-41.2; C₁ is: 1.0-1.9, 1.0-1.8, 1.2-1.8, 1.2-1.6, and 1.3-1.6; C₂ is: 1.0-1.9, 1.0-1.8, 1.2-1.8, 1.2-1.6, and 1.3-1.6; C₃ is: 2.3-4.2, 2.3-3.9, 2.6-3.9, 2.6-3.6, and 2.9-3.6; and C₄ is: 0.8-1.6, 0.8-1.5, 1.0-1.5, 1.0-1.3, and 1.1-1.3.

In yet other disclosed but not claimed embodiments, the blood sample is whole blood or plasma, the blood test is Thyroid Index (T7.Index), gene₁ is IGHV3-33 from whole blood, gene₂ is ZNF266 from whole blood, gene₃ is CCDC183-AS1 from whole blood, gene₄ is ENSG00000232745 from plasma, C is: 1.9-3.5, 1.9-3.2, 2.2-3.2, 2.2-3.0, and 2.4-3.0; C₁ is: between -0.20 and - 0.11, between -0.18 and -0.11, between -0.18 and -0.12, between -0.17 and -0.12, and between -0.17 and -0.14; C₂ is: between -0.99 and -0.53, between -0.91 and -0.53, between - 0.91 and -0.61, between -0.84 and -0.61, and between -0.84 and -0.69; C₃ is: 0.21-0.38, 0.21-0.36, 0.24-0.36, 0.24-0.33, and 0.27-0.33; and C₄ is: between -0.16 and -0.09, between -0.15 and -0.09, between -0.15 and -0.10, between -0.14 and -0.10, and between -0.14 and -0.11.

In further disclosed but not claimed embodiments, the blood sample is dried blood spot, the target blood component is Alaine Aminotransferase, gene₁ is EIF1AY, gene₂ is SRXN1, gene₃ is NDUFAF2, and gene₄ is TBCE, C is: 13.3-24.7, 13.3-22.8, 15.2-22.8, 15.2-20.9, and 17.1-20.9; C₁ is: 2.6-4.8, 2.6-4.5, 3.0-4.5, 3.0-4.1, and 3.3-4.1; C₂ is: 2.1-3.9, 2.1-3.6, 2.4-3.6, 2.4-3.3, and 2.7-3.3; C₃ is: 3.0-5.6, 3.0-5.2, 3.4-5.2, 3.4-4.7, and 3.9-4.7; and C₄ is: between -7.2 and -3.9, between -6.6 and -3.9, between -6.6 and -4.4, between -6.1 and -4.4, and between - 6.1 and -5.0.

In some disclosed but not claimed embodiments, the blood sample is dried blood spot, the target blood component is Eosinophils, gene₁ is SCARNA22, and gene₂ is TET3, C is: 0.60-1.11, 0.60-1.02, 0.68-1.02, 0.68-0.94, and 0.77-0.94; C₁ is: 0.66-1.22, 0.66-1.13, 0.75-1.13, 0.75-1.04, and 0.85-1.04; and C₂ is: 0.61-1.13, 0.61-1.04, 0.69-1.04, 0.69-0.95, and 0.78-0.95.

In other disclosed but not claimed embodiments, the blood sample is dried blood spot, the target blood component is Segmented Neutrophils, gene₁ is HMGB1P1, gene₂ is CSRNP1, and gene₃ is CCNJL, C is: 39.1-72.5, 39.1-67.0, 44.6-67.0, 44.6-61.4, and 50.2-61.4; C₁ is: 2.0-3.7, 2.0-3.4, 2.3-3.4, 2.3-3.1, and 2.5-3.1; C₂ is: 2.0-3.7, 2.0-3.4, 2.3-3.4, 2.3-3.1, and 2.5-3.1; and C₃ is: 1.7-3.2, 1.7-2.9, 2.0-2.9, 2.0-2.7, and 2.2-2.7.

In yet other disclosed but not claimed embodiments, the blood sample is high-quality dried blood spot, the target blood component is non-HDL cholesterol, gene₁ is BMT2, gene₂ is PKD1P5, and gene₃ is ARIH1, C is: 133-247, 133-228, 152-228, 152-209, and 171-209; C₁ is: between -52 and - 28, or any number range in between, e.g., between -48 and -28, between -48 and -32, between -44 and -32, and between -44 and -36; C₂ is: 17.4-32.2, 17.4-29.8, 19.8-29.8, 19.8-27.3, and 22.3-27.3; and C₃ is: between -47 and -25, between -44 and -25, between -44 and -29, between -40 and -29, and between -40 and -33.

In further disclosed but not claimed embodiments, the blood sample is high-quality dried blood spot, the target blood component is Eosinophils, gene₁ is NDUFA5, and gene₂ is MCM8, C is: 1.1-2.1, 1.1-2.0, 1.3-2.0, 1.3-1.8, and 1.5-1.8; C₁ is: 0.46-0.85, 0.46-0.78, 0.52-0.78, 0.52-0.72, and 0.59-0.72; and C₂ is: between -1.2 and -0.6, between -1.1 and -0.6, between -1.1 and -0.7, between -1.0 and -0.7, and between -1.0 and -0.8.

In yet further disclosed but not claimed embodiments, the blood sample is high-quality dried blood spot, the target blood component is Segmented Neutrophils, gene₁ is AKAP12, and gene₂ is APP, C is: 2.4-4.5, 2.4-4.2, 2.8-4.2, 2.8-3.8, and 3.1-3.8; C₁ is: 1.0-1.9, 1.0-1.7, 1.1-1.7, 1.1-1.6, and 1.3-1.6; and C₂ is: 1.6-3.0, 1.6-2.8, 1.9-2.8, 1.9-2.6, and 2.1-2.6.

In some disclosed but not claimed embodiments, the blood sample is whole blood, the target blood component is Lymphocytes, gene₁ is EVI2B, and gene₂ is NFAM1, C is: 39.7-73.7, 39.7-68.1, 45.4-68.1, 45.4-62.4, and 51.1-62.4; C₁ is: between -20.6 and -11.1, between -19.1 and - 11.1, between -19.1 and -12.7, between -17.5 and -12.7, and between -17.5 and -14.3; and C₂ is: between -16.1 and -8.7, between -14.8 and -8.7, between -14.8 and -9.9, between -13.6 and -9.9, and between -13.6 and -11.1.

In other disclosed but not claimed embodiments, the blood sample is whole blood, the target blood component is Monocytes, gene₁ is RIN2, and gene₂ is ADA2, C is: between -0.21 and -0.11, between -0.19 and -0.11, between -0.19 and -0.13, between -0.17 and -0.13, and between - 0.17 and -0.14; C₁ is: 2.8-5.1, 2.8-4.7, 3.1-4.7, 3.1-4.3, and 3.5-4.3; and C₂ is: 2.5-4.6, 2.5-4.3, 2.8-4.3, 2.8-3.9, and 3.2-3.9.

In yet other disclosed but not claimed embodiments, the blood sample is whole blood, the target blood component is Segmented Neutrophils, gene₁ is RNF24, gene₂ is MNDA, and gene₃ is TLR1, C is: 25.0-46.4, 25.0-42.8, 28.6-42.8, 28.6-39.3, and 32.1-39.3; C₁ is: 6.2-11.5, 6.2-10.6, 7.1-10.6, 7.1-9.7, and 8.0-9.7; C₂ is: 6.8-12.7, 6.8-11.7, 7.8-11.7, 7.8-10.7, and 8.8-10.7; and C₃ is: 5.2-9.7, 5.2-9.0, 6.0-9.0, 6.0-8.2, and 6.7-8.2.

Herein the inventors also disclose a blood test, which is not part of the invention. Typically, the blood test comprises a positive control plasmid, a first reagent, and a second reagent. The positive control plasmid comprising an exon of a predictive gene selected from Tables 1-9, wherein an mRNA level of the predictive gene in the blood sample relates to a blood test result of a target blood component. The first reagent detects the mRNA level of the predictive gene, comprises at least a primer or a probe hybridizing to the exon of the predictive gene. The second reagent detects an mRNA level of a housekeeping gene, for example, a primer or a probe hybridizing to the exon of the housekeeping gene.

Non-limiting examples of the housekeeping genes include glyceraldehyde-3-phosphate dehydrogenase (GAPDH), ACTB actin, beta2-microglobulin (B2M), Porphobilinogen deaminase (HMBS), or Peptidylprolyl Isomerase B (PPIB), *etc.*

Non-limiting examples of the target blood component include Segmented Neutrophils, Eosinophils, Prostate-Specific Antigen (PSA_total), Red Blood Cell count (RBC _m.mm3), Monocytes, Creatinine, Lymphocytes, Absolute Eosinophil, Anion Gap (AG), red cell distribution width (RDW_sd), Thyroid Index (T7), Alanine Aminotransferase, or non-HDL cholesterol, *etc.*

In some disclosed but not claimed embodiments, the target blood component is Segmented Neutrophils, and the predictive gene is: MNDA, STX3, TNFRSF1A, MSL1, TLR1, RNF24, WIPF1, RXFP1, POLR3GL, FOXK2, LAMB1, HMGB1P1, CSRNP1, CCNJL, AKAP12, or APP. In other disclosed but not claimed embodiments, the target blood component is Eosinophils, and the predictive gene is: SLC29A1, SIGLEC8, IL5RA, TMIGD3, SMPD3, SCARNA22, SNORA36C, SNORA11, RN7SL4P, SNHG15, TET3, NDUFA5, or MCM8. In yet other embodiments, the target blood component is PSA_total, and the predictive gene is: CTC-265F19.1, ADAM9, RAB11FIP5, SNAPC4, LMNA, HNRNPA3P3, GTF3A, RP11-342M1.6, HNRNPLP2, or RPS11P5. In further disclosed but not claimed embodiments, the target blood component is Red Blood Cell count (RBC _m.mm3), and the predictive gene is: UTY, DDX3Y, ZFY, TXLNGY, or RPS4Y1. In yet further disclosed but not claimed embodiments, the target blood component is Lymphocytes, and the predictive gene is: GRB2, MNDA, NFAM1, or EVI2B.

In some disclosed but not claimed aspects, the target blood component is Monocytes, and the predictive gene is: NAGA, RIN2, ADA2, PLXNB2, or ANXA2. In other disclosed but not claimed aspects, the target blood component is Absolute Eosinophil, and the predictive gene is: CLC, ADAT1, SNRPEP4, or GPC6. In yet other disclosed but not claimed aspects, target blood component is Anion Gap (AG), and the predictive gene is: DHX40, SLC1A4, IMPA2, KATNA1, or MEIS3P1. In further disclosed but not claimed aspects, the target blood component is red blood cell distribution width (RDW_sd), and the predictive gene is: CHCHD2P6, SEC63P1, DNAL1, or ENSG00000197262. In yet further disclosed but not claimed aspects, the target blood component is Thyroid Index (T7.Index), and the predictive gene is: IGHV3-33, ZNF266, CCDC183-AS1, or ENSG00000232745.

In some disclosed but not claimed embodiments, the target blood component is Alaine Aminotransferase, and the predictive gene is: EIF1AY, SRXN1, NDUFAF2, or TBCE. In other disclosed but not claimed embodiments, the target blood component is non-HDL cholesterol, and the predictive gene is: BMT2, PKD1P5, or ARIH1.

Additional objectives, advantages and novel features will be set forth in the description which follows or will become apparent to those skilled in the art upon examination of the drawings and detailed description which follows.

### BRIEF DESCRIPTION OF THE DRAWINGS

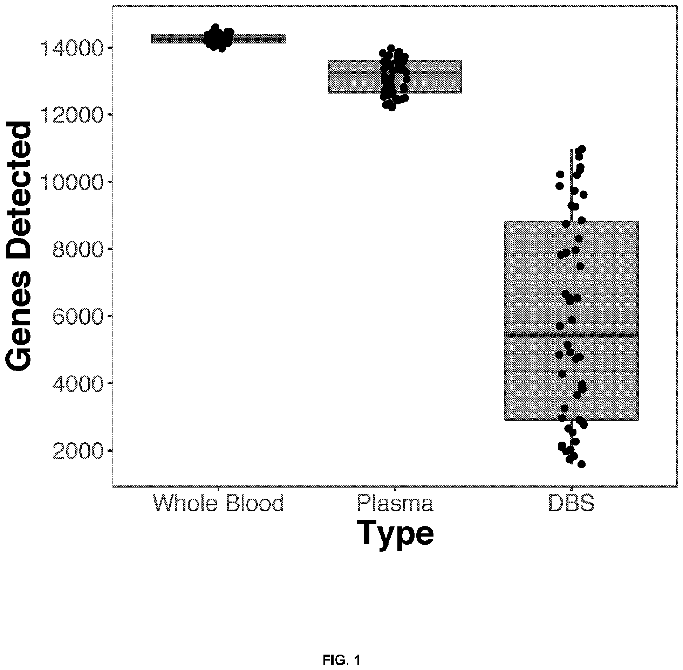

FIGs. 1 and 2 show the range in the number of genes detected whole blood samples, plasma samples, and dried blood spot samples.

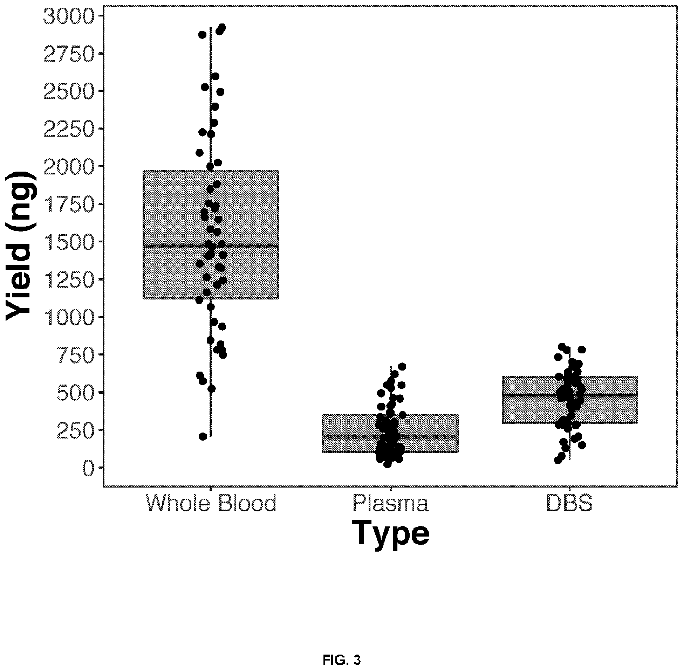

FIG. 3 depicts the spread of RNA yield from whole blood samples, plasma samples, and dried blood spot samples.

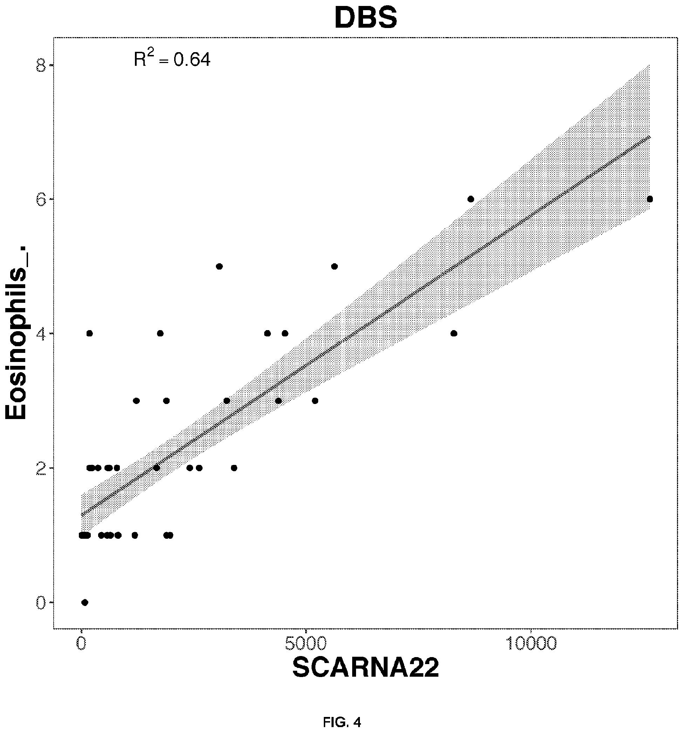

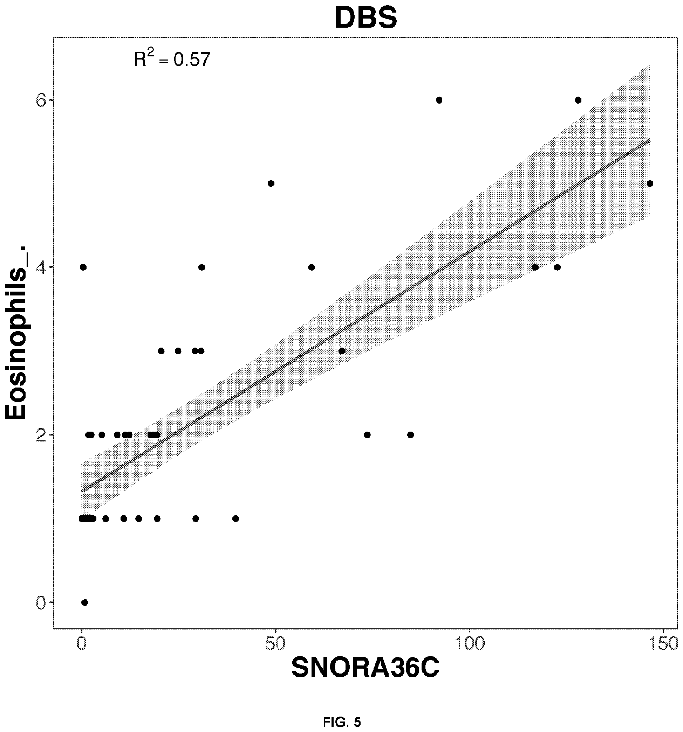

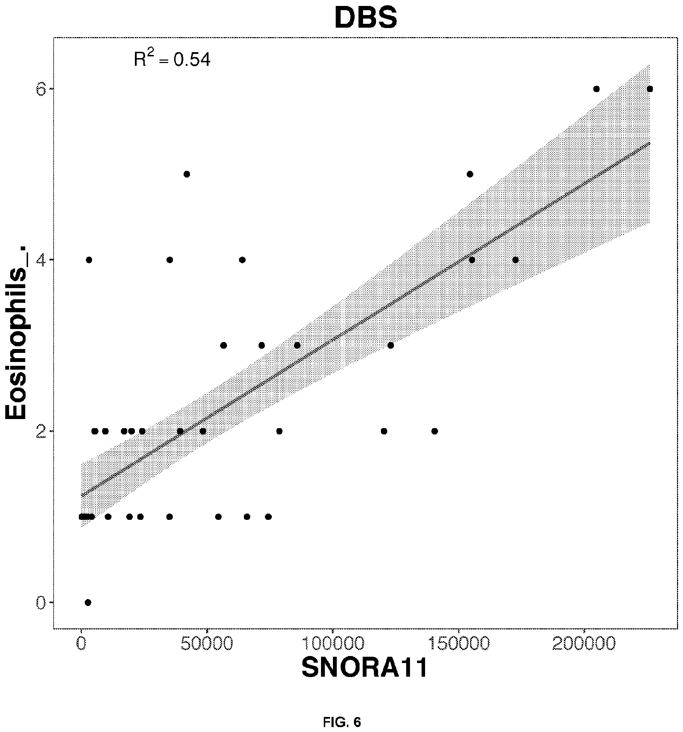

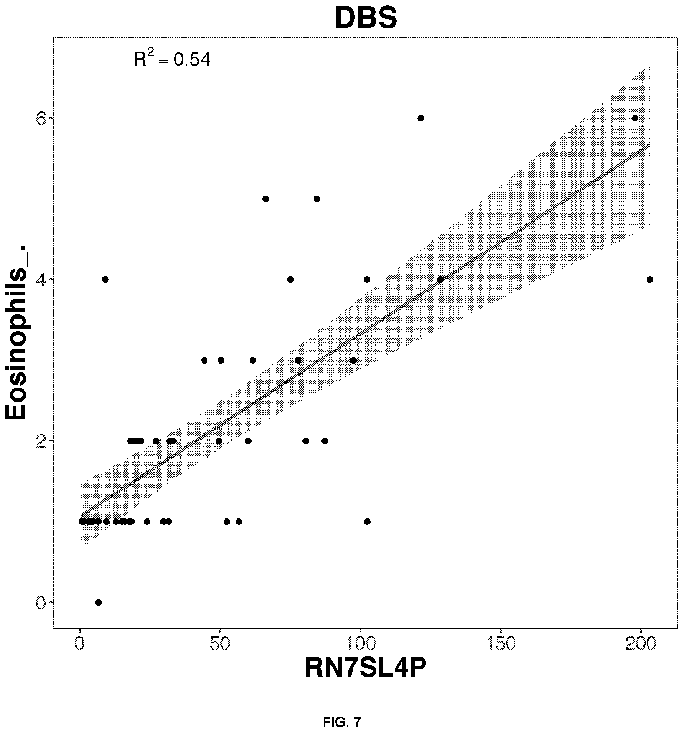

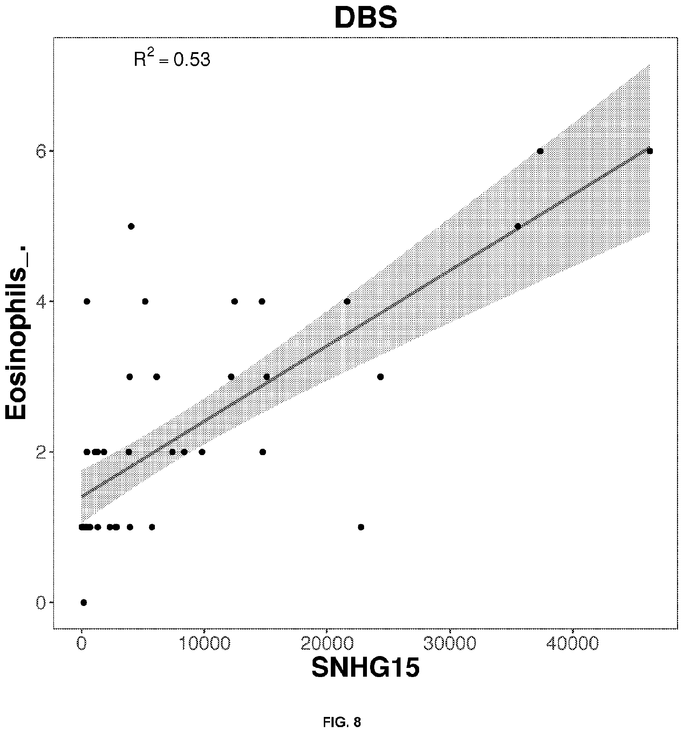

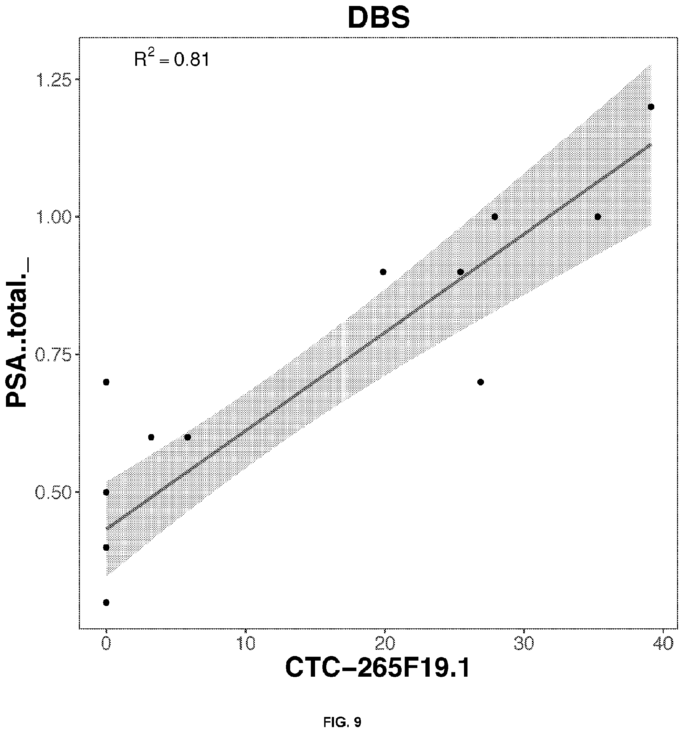

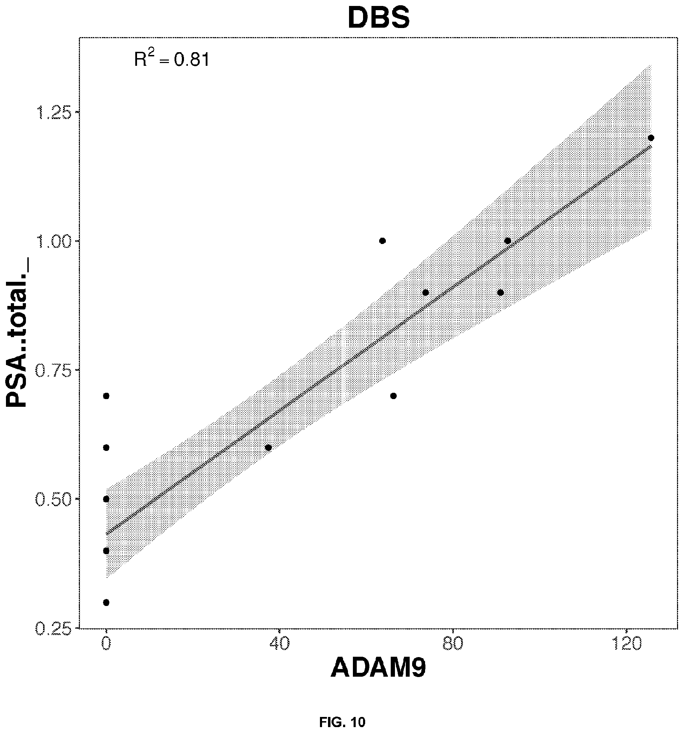

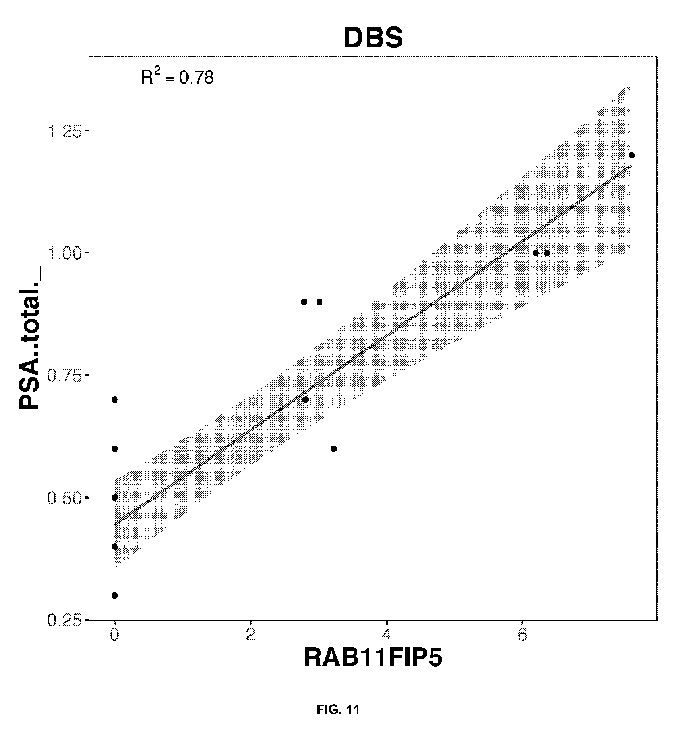

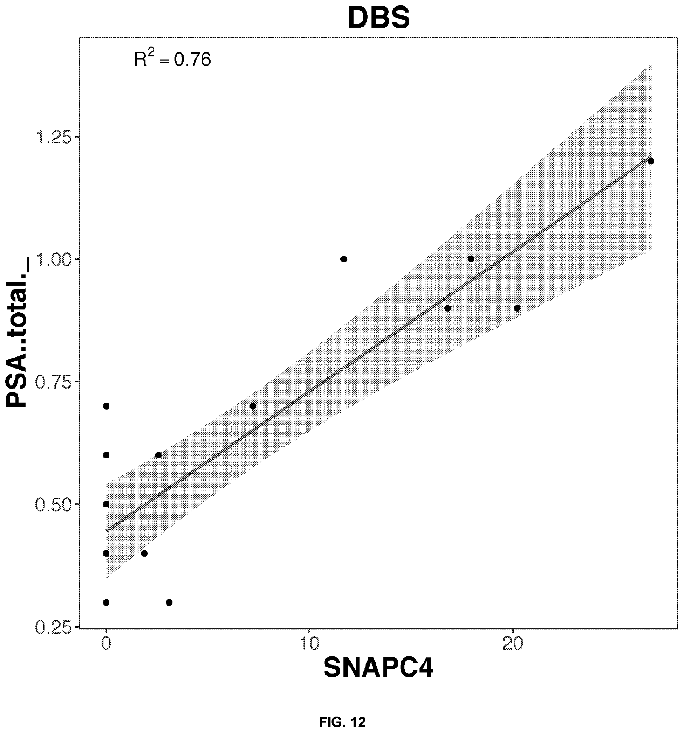

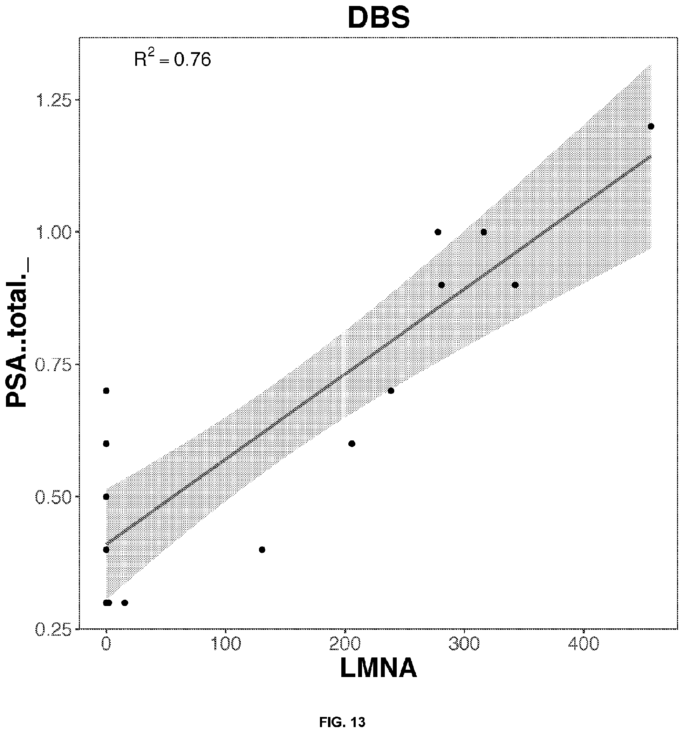

FIGs. 4-13 depict the simple regression graphs of the RNA expression of gene in dried blood spot samples with the results of a blood test for highly predictive single genes.

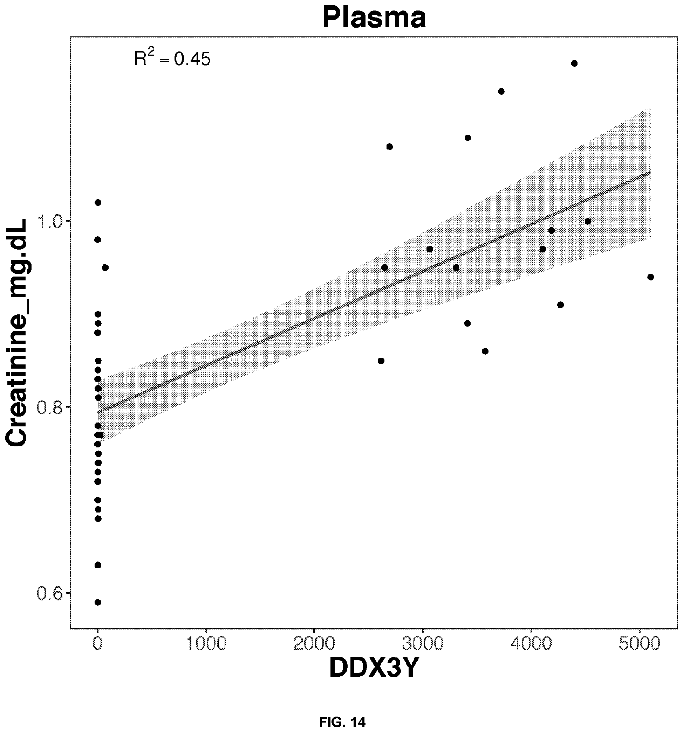

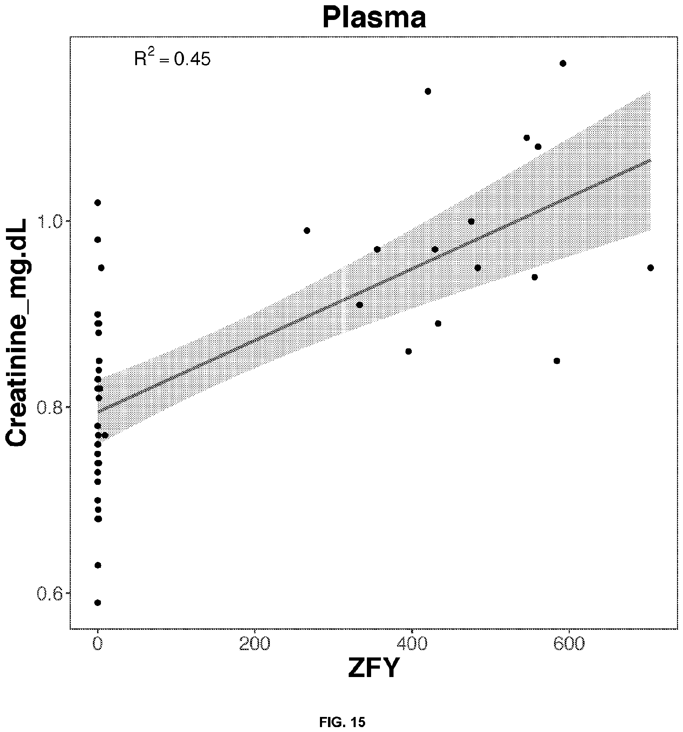

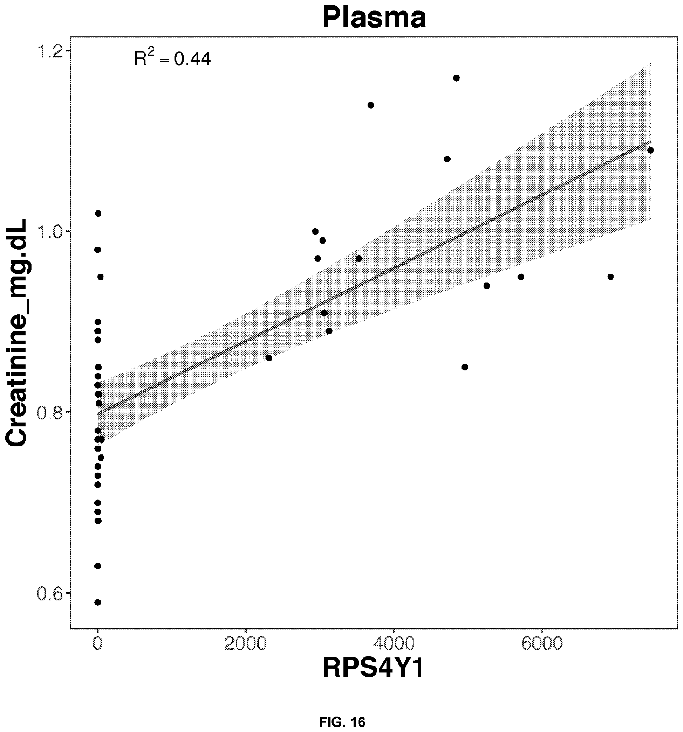

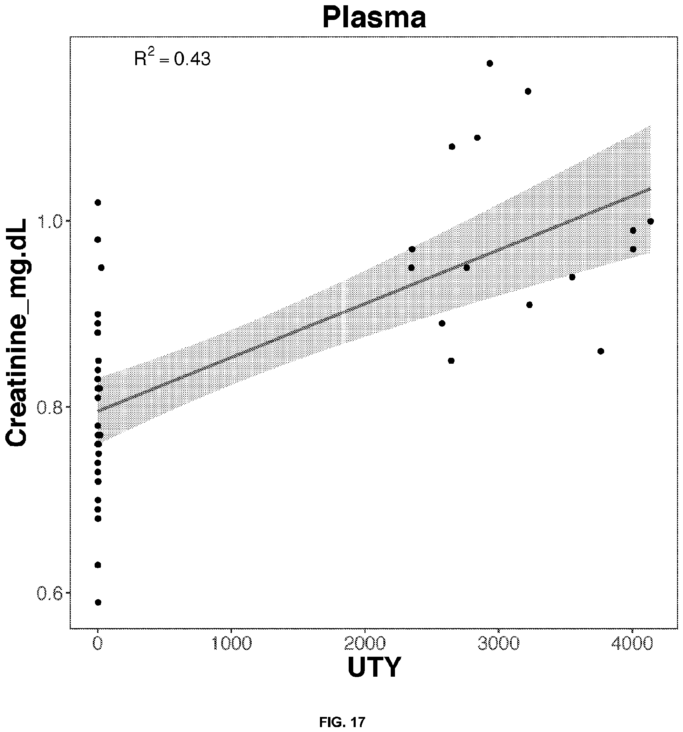

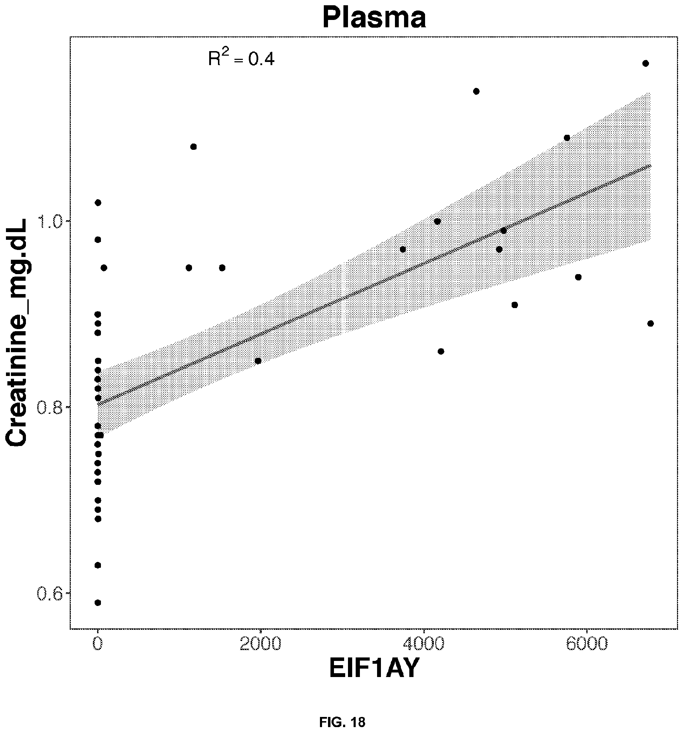

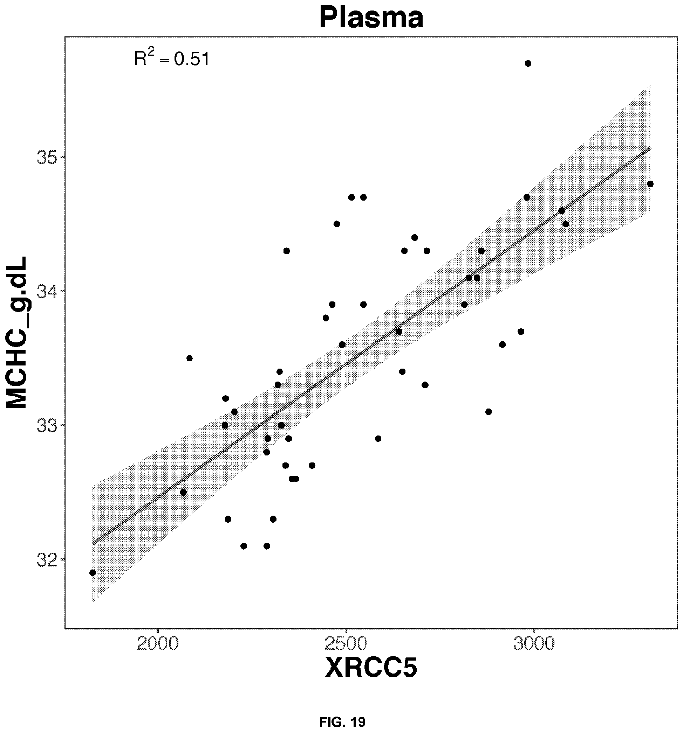

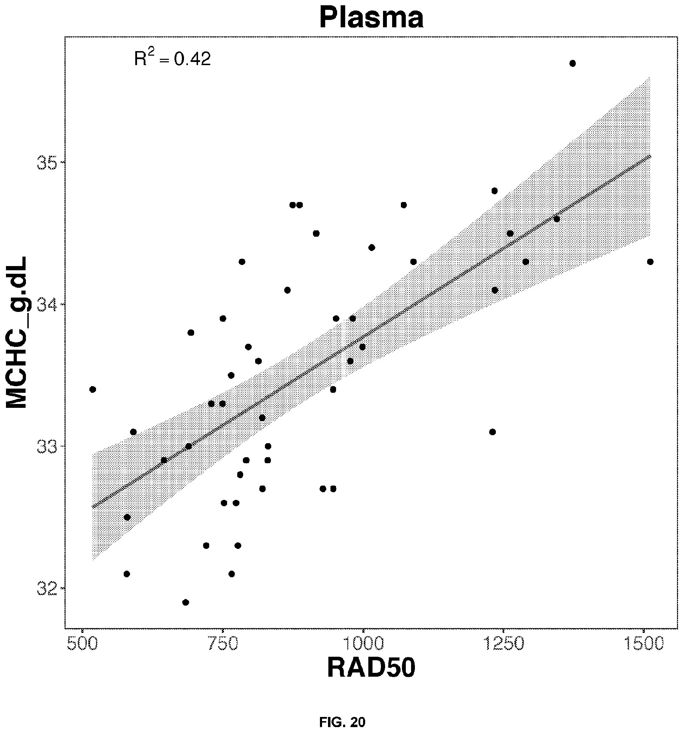

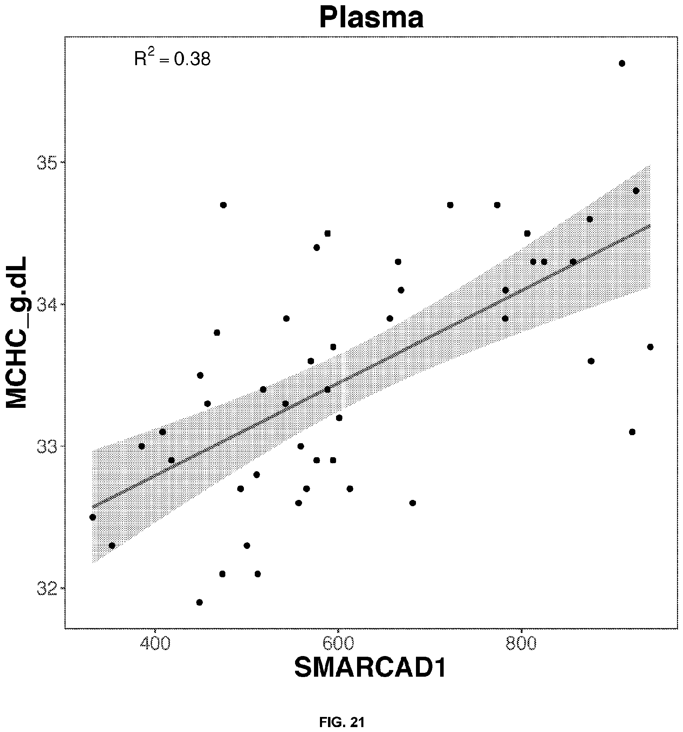

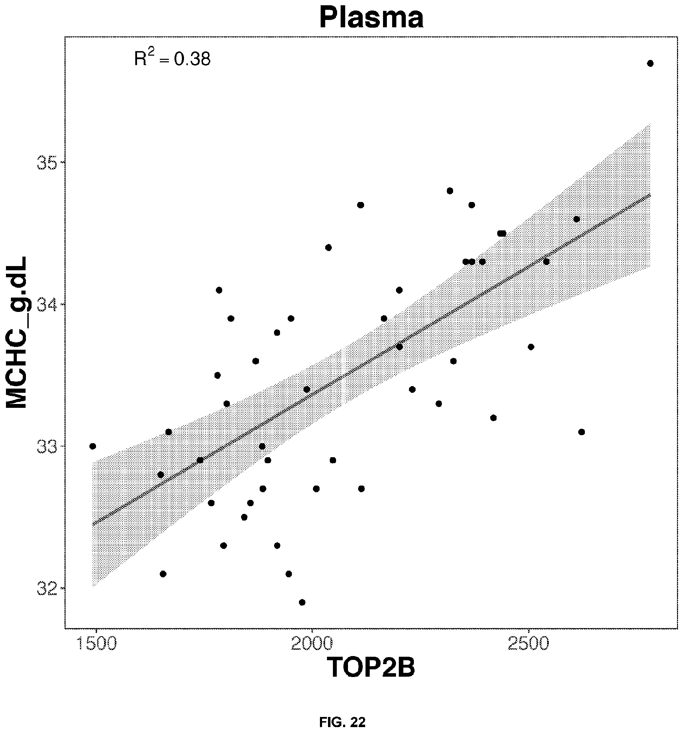

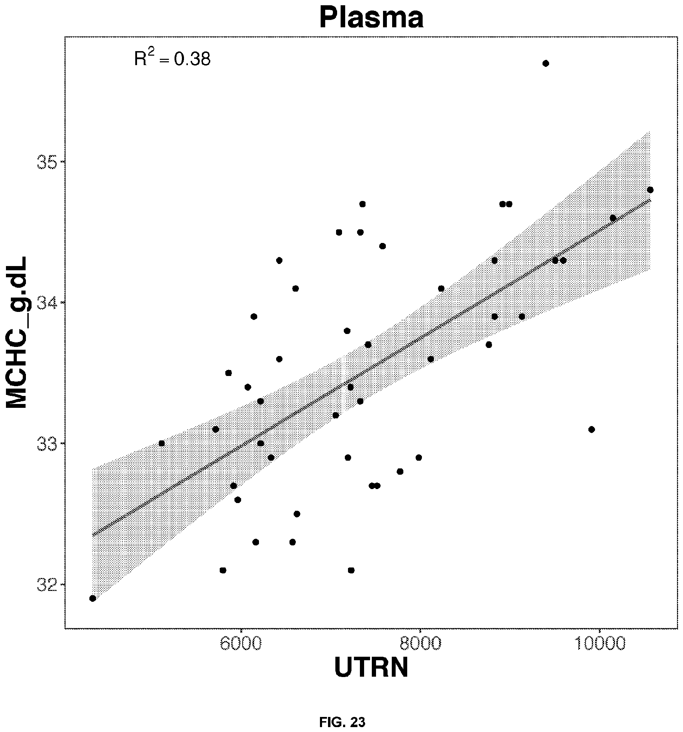

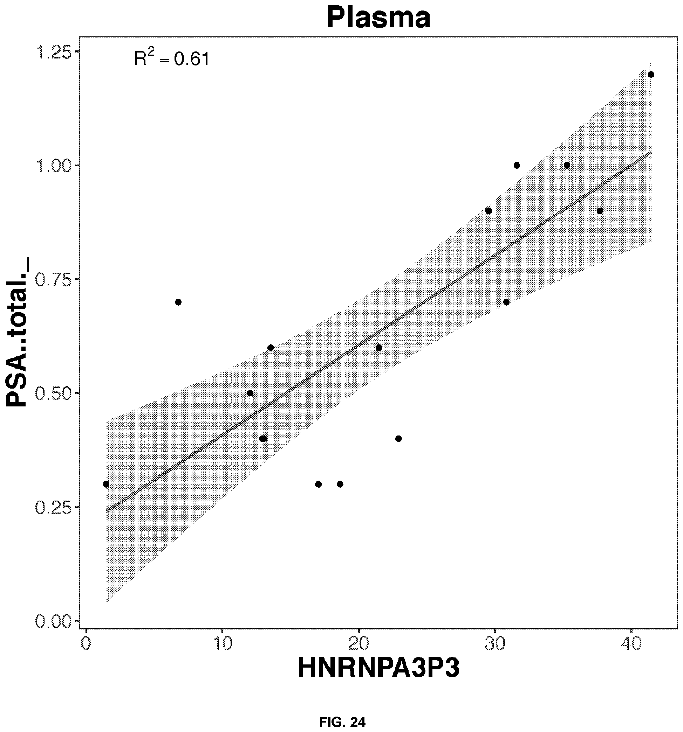

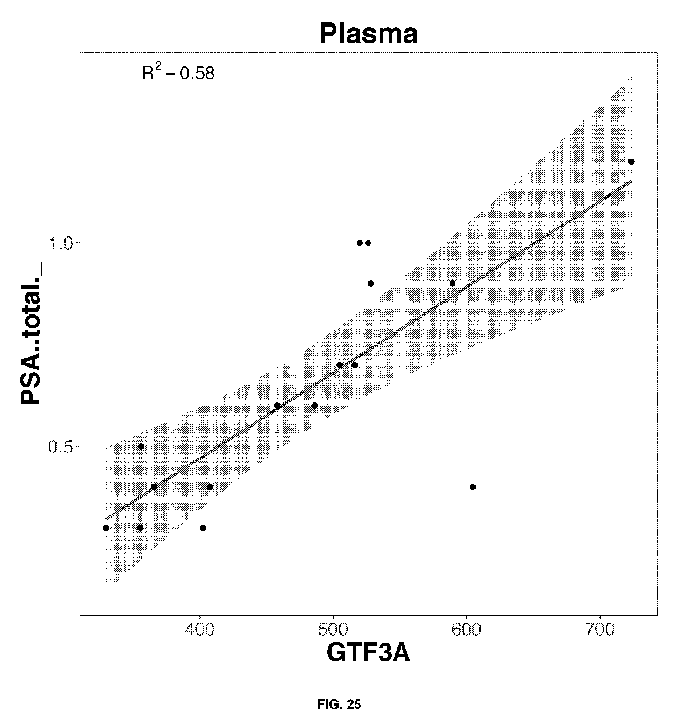

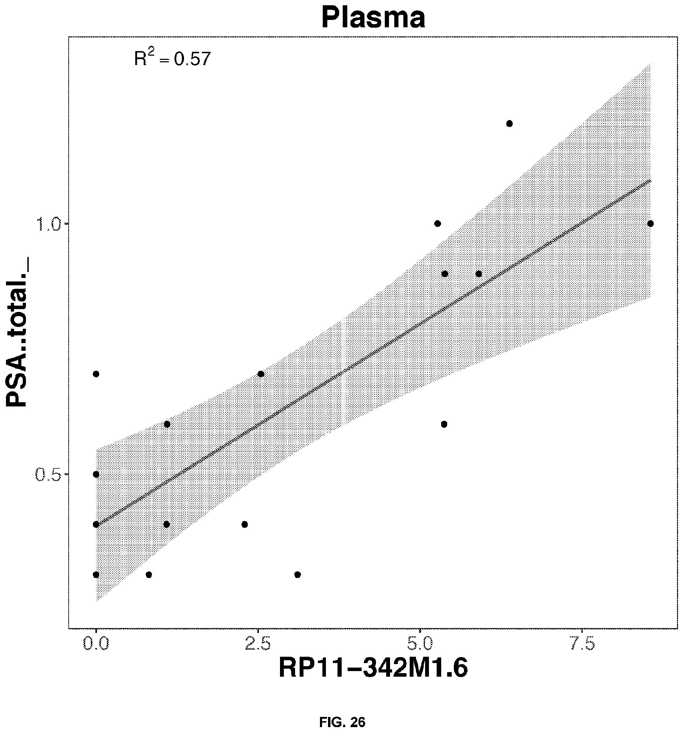

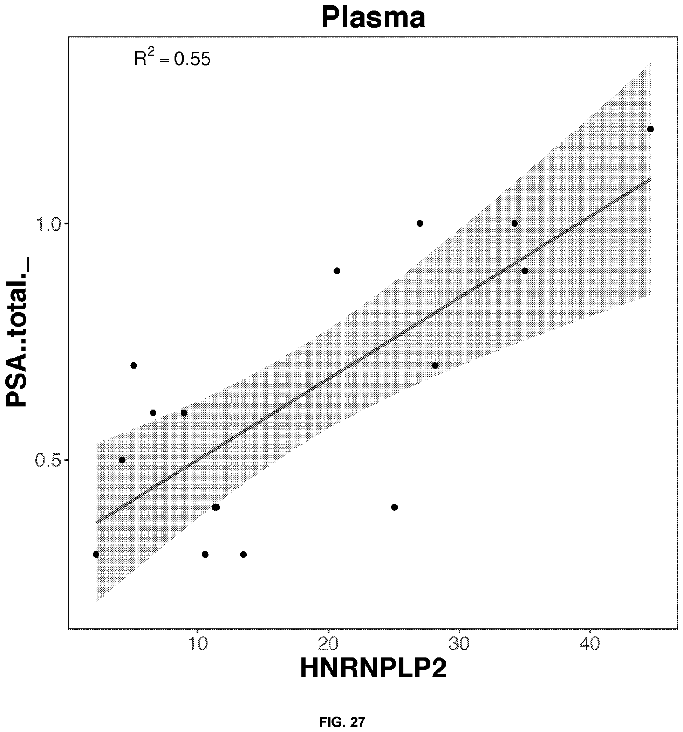

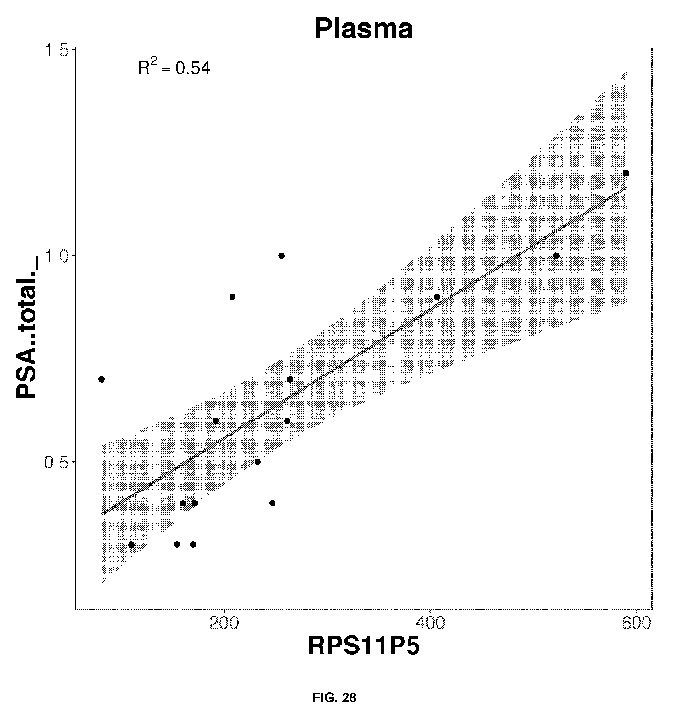

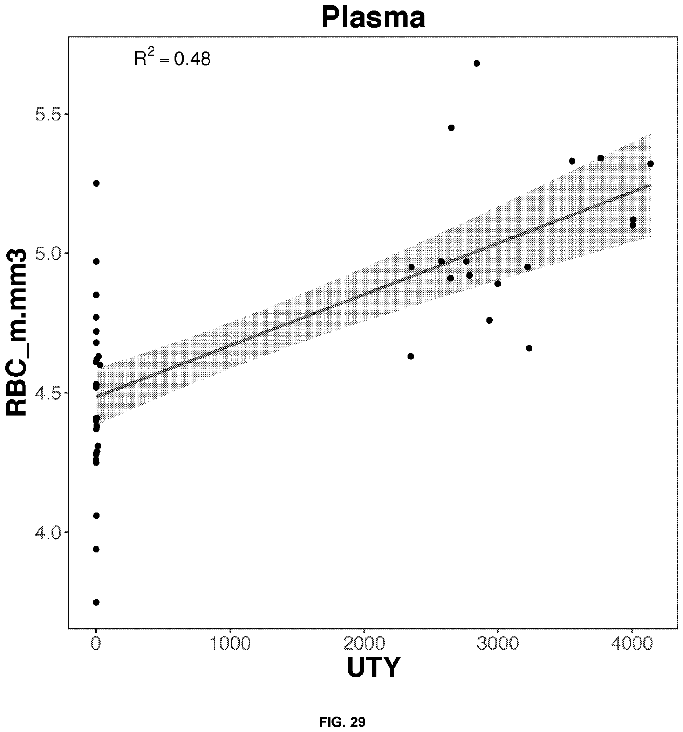

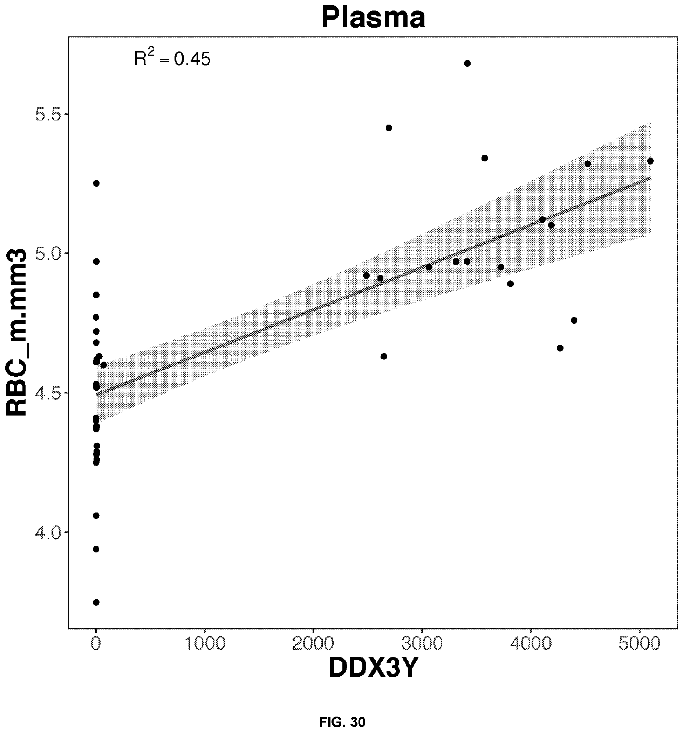

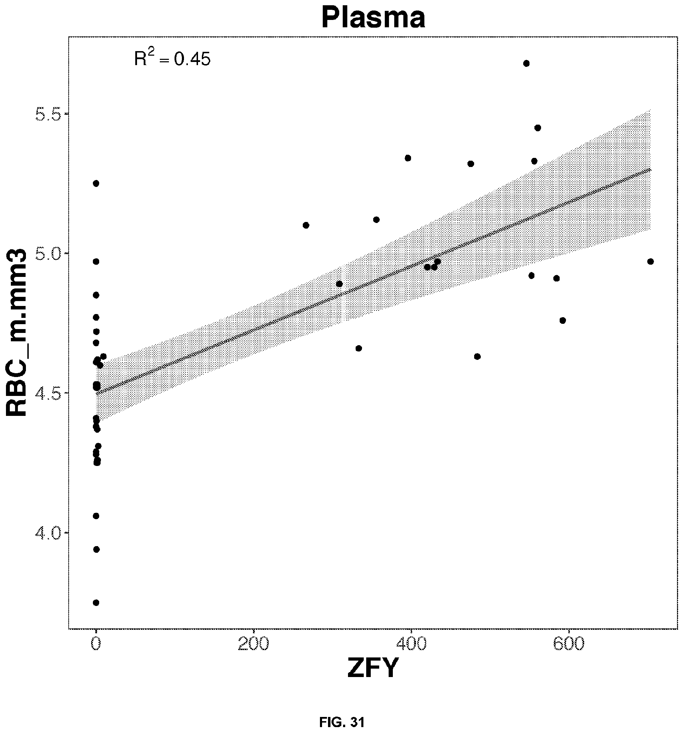

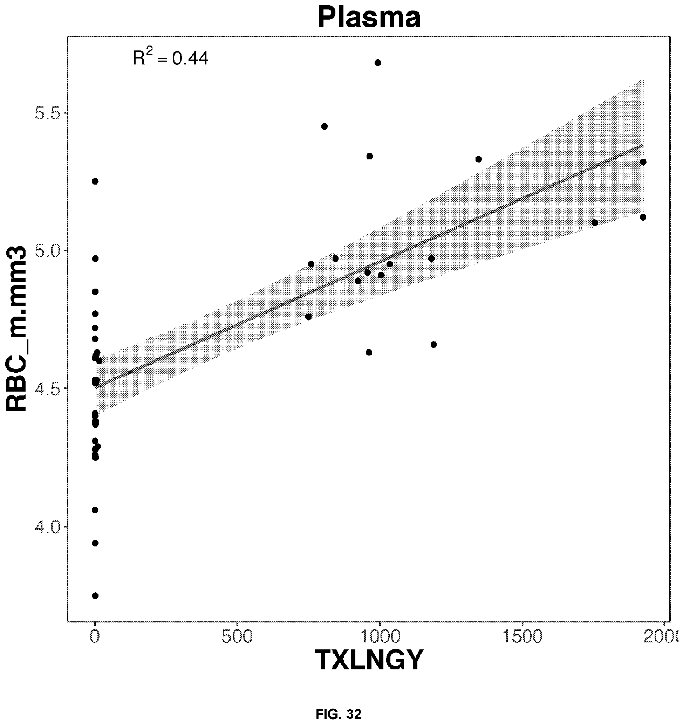

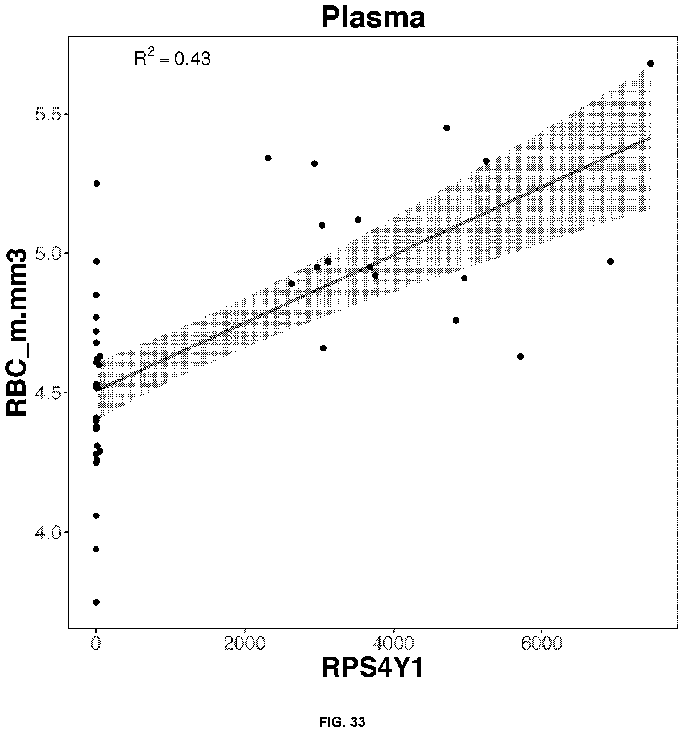

FIGs. 14-33 depict the simple regression graphs of the RNA expression of a gene in plasma samples with the results of a blood test for highly predictive single genes.

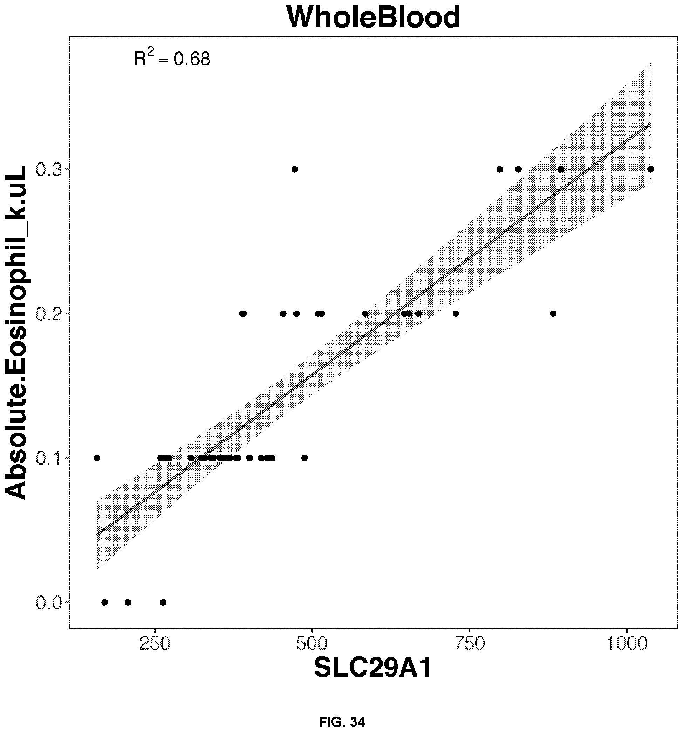

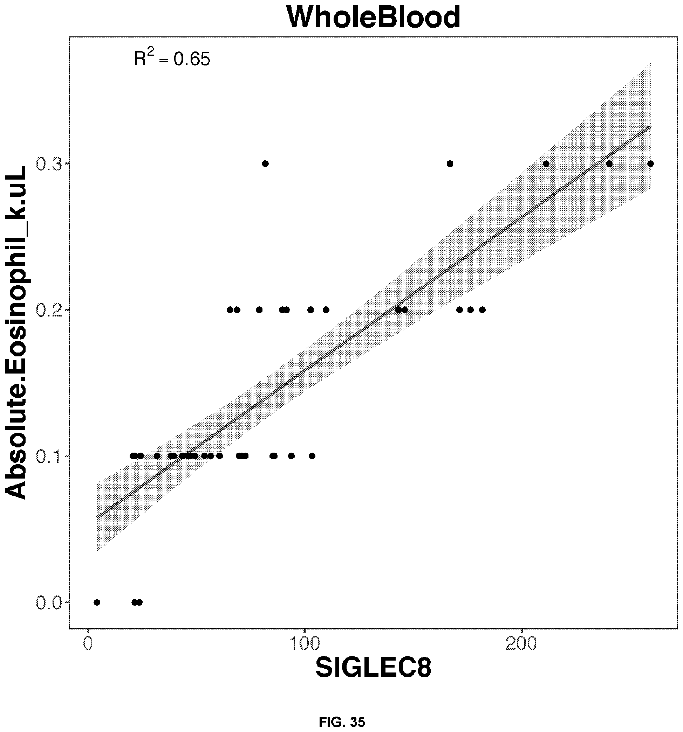

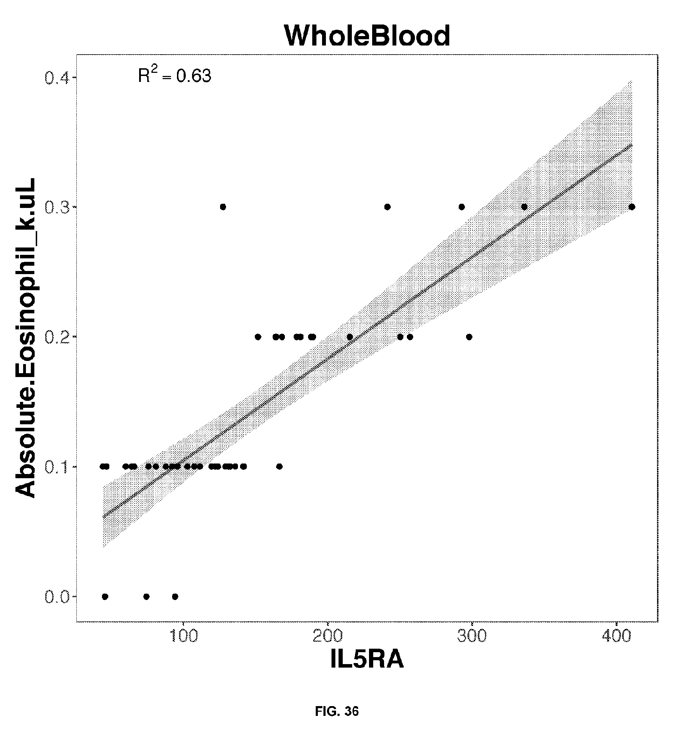

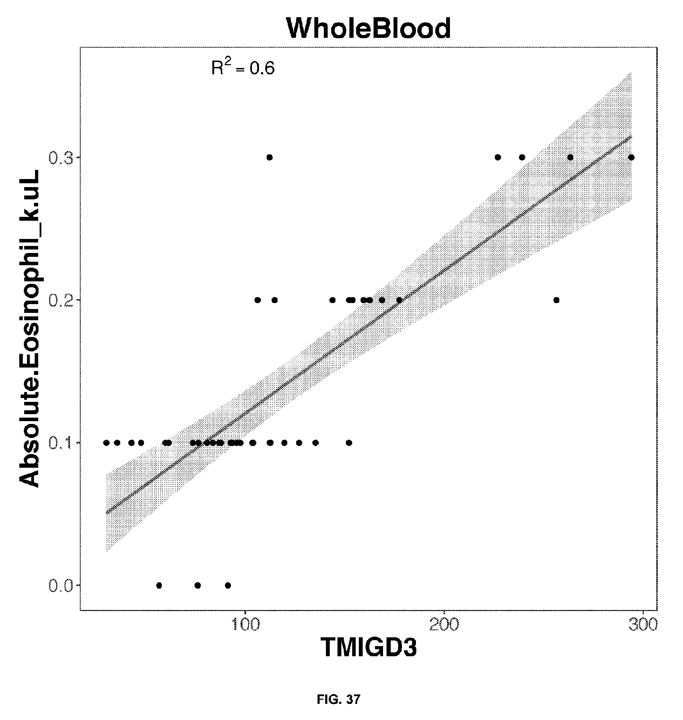

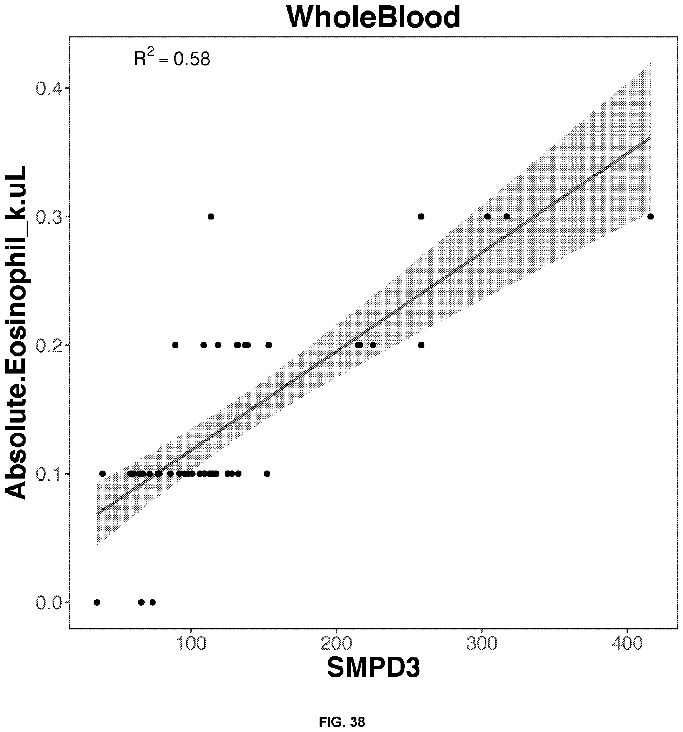

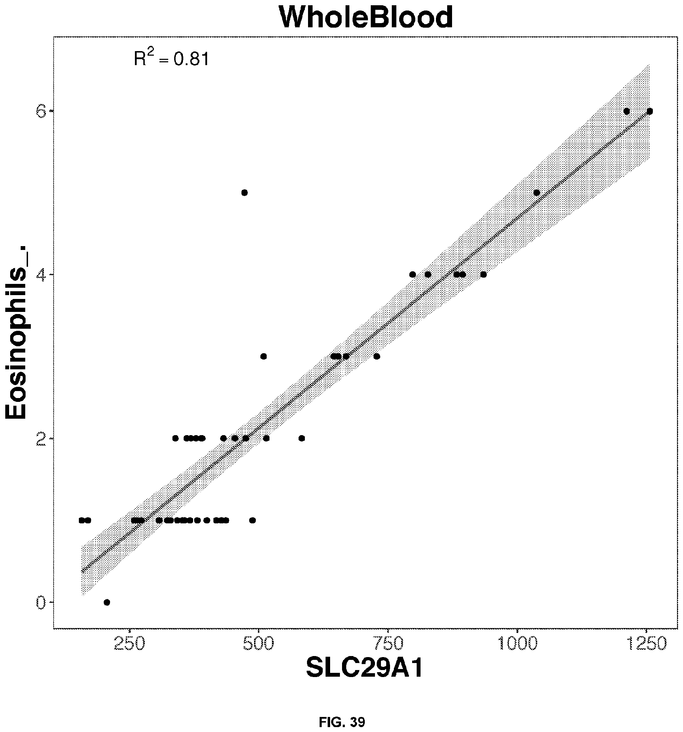

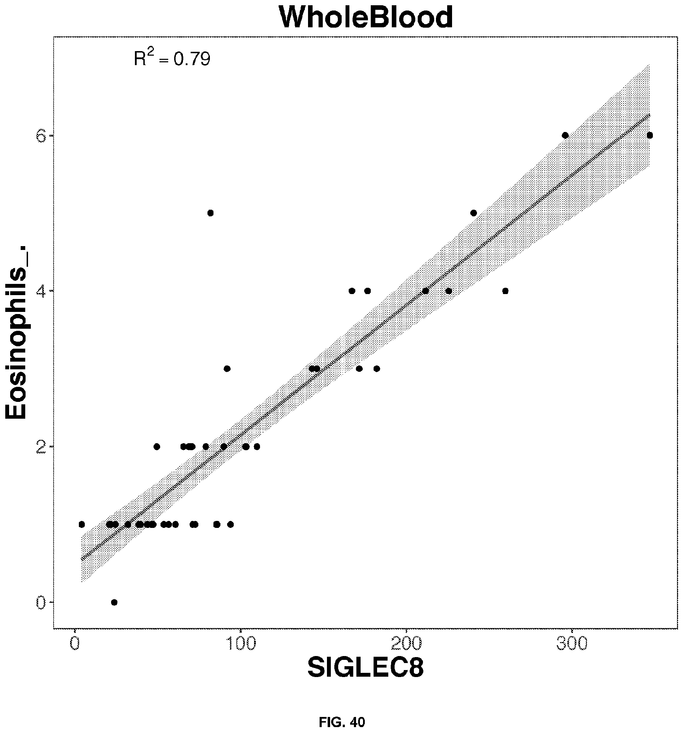

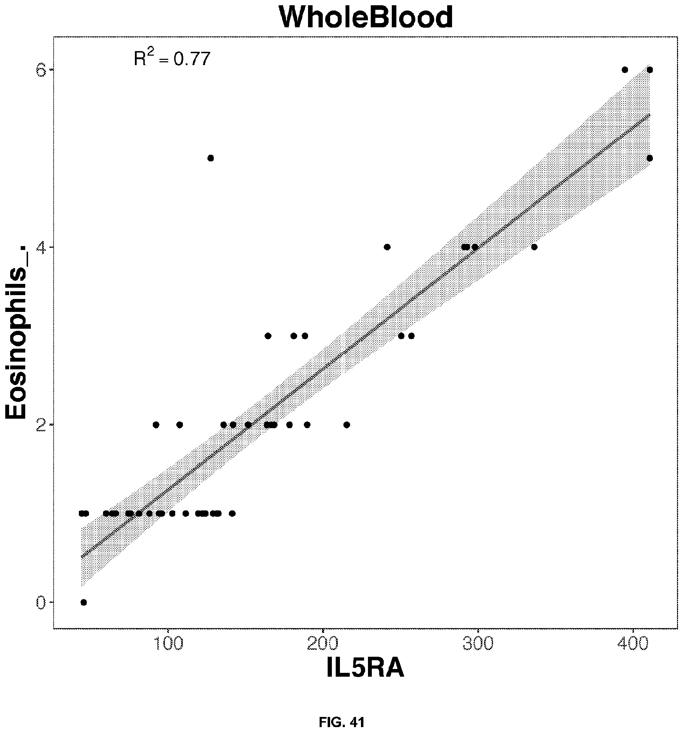

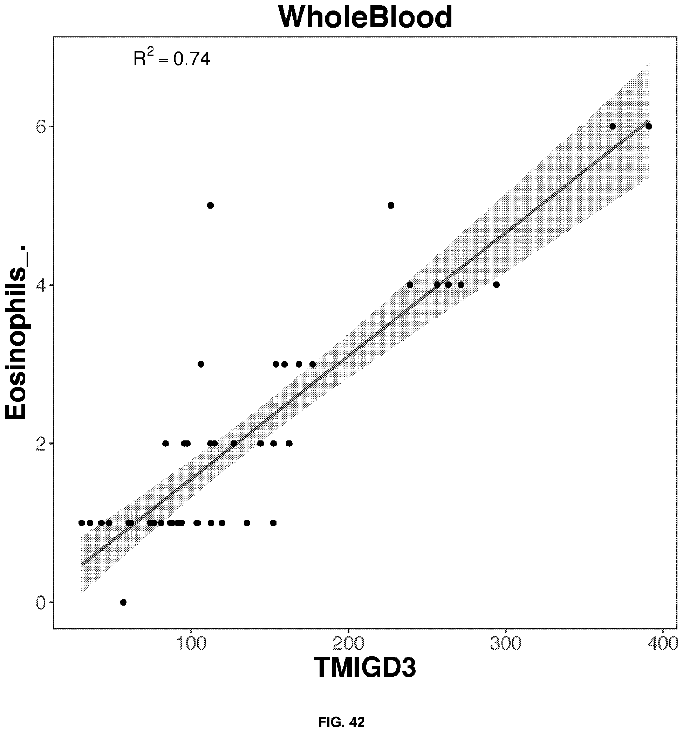

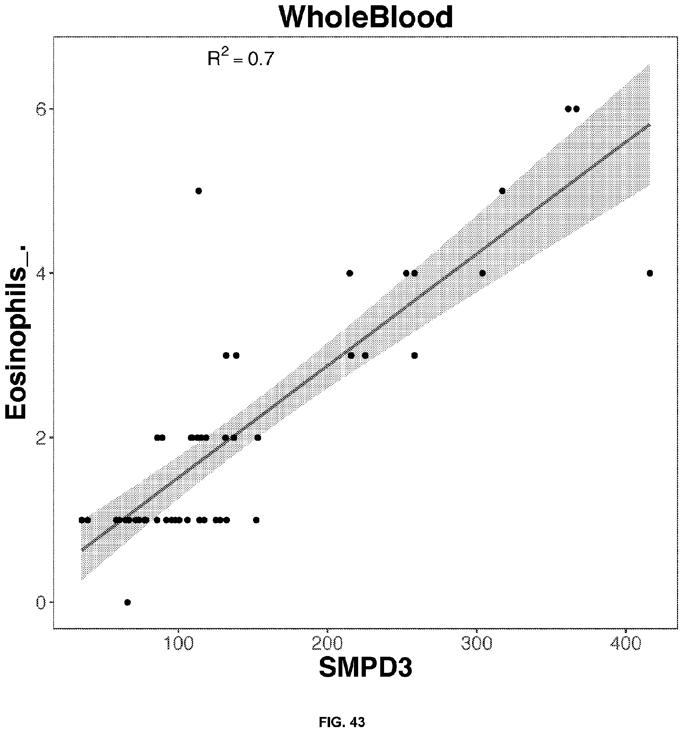

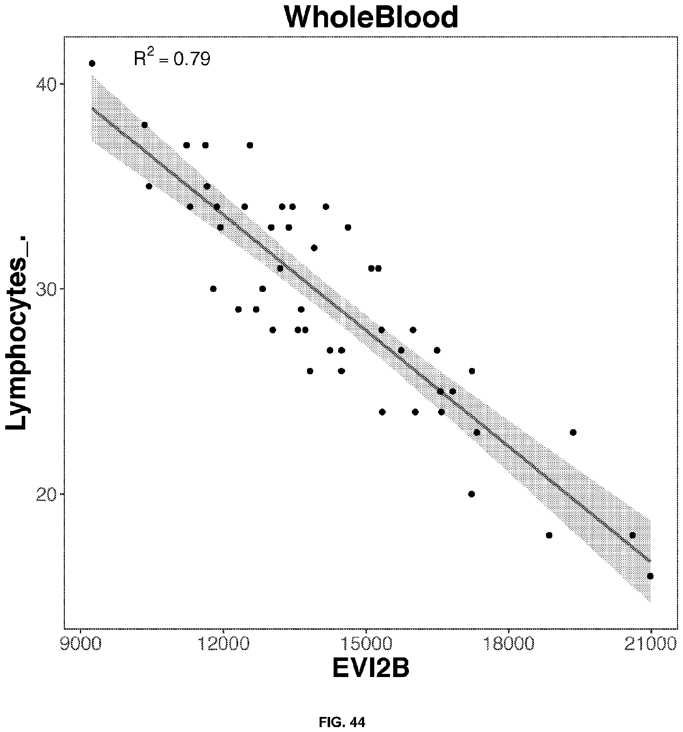

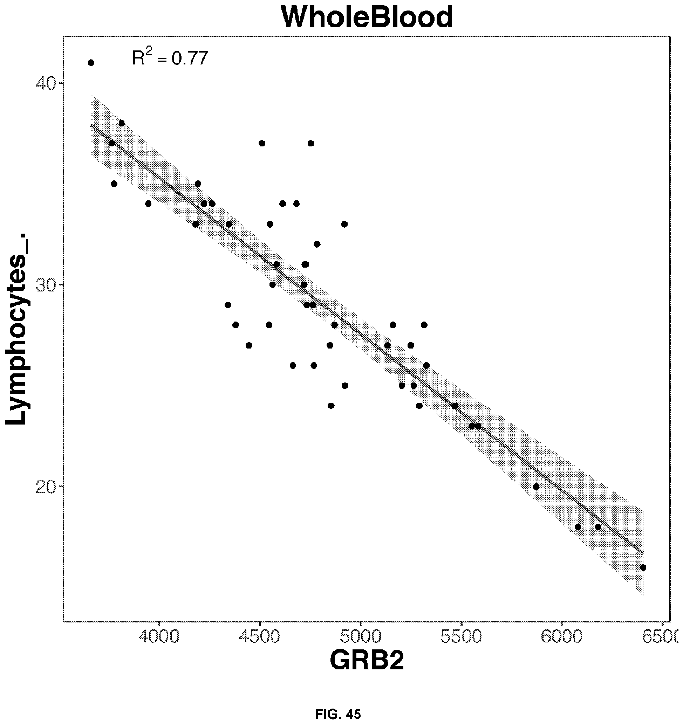

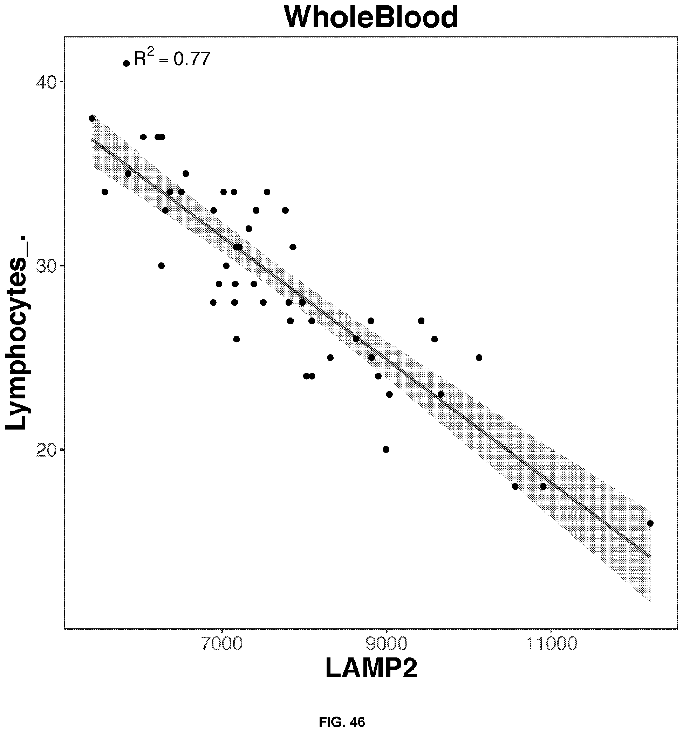

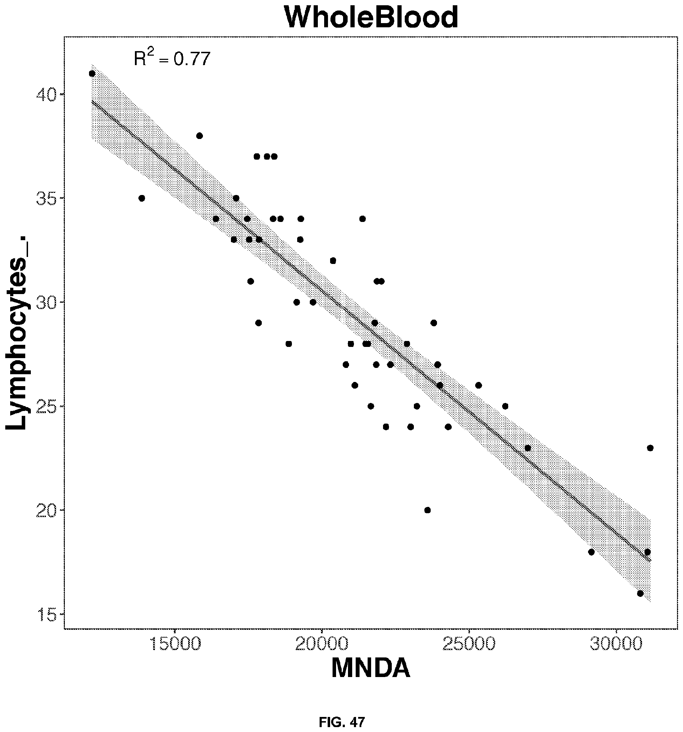

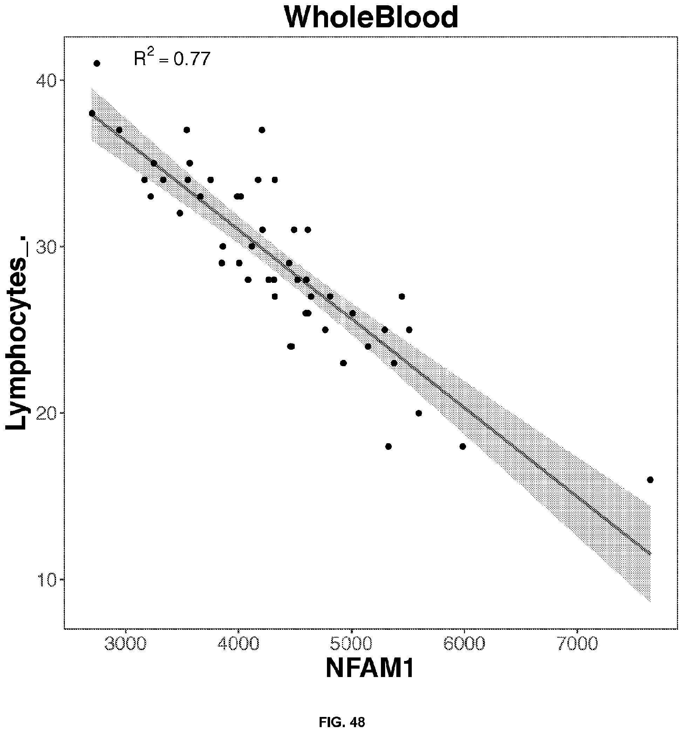

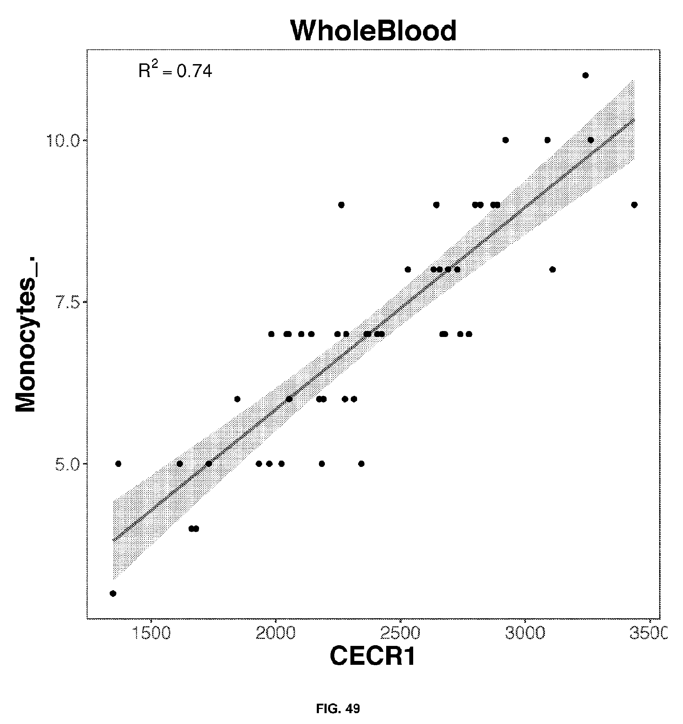

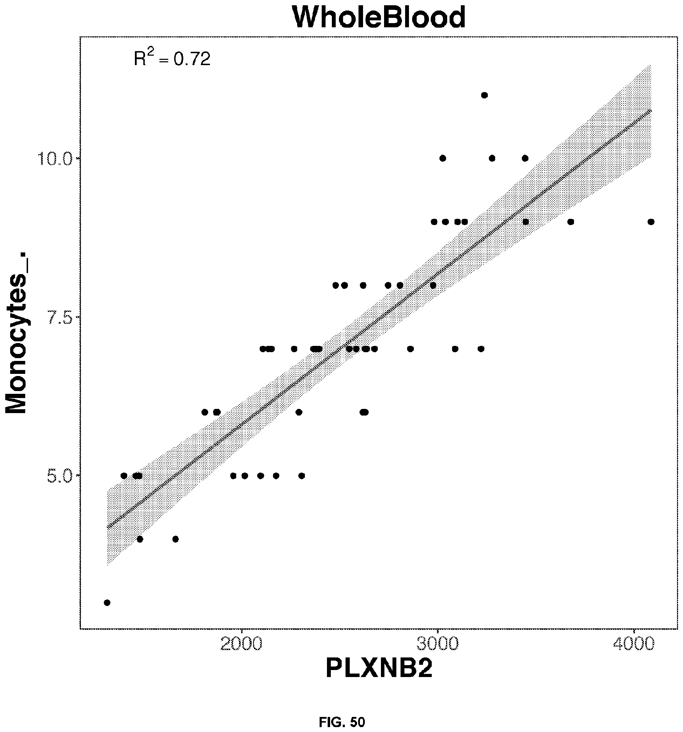

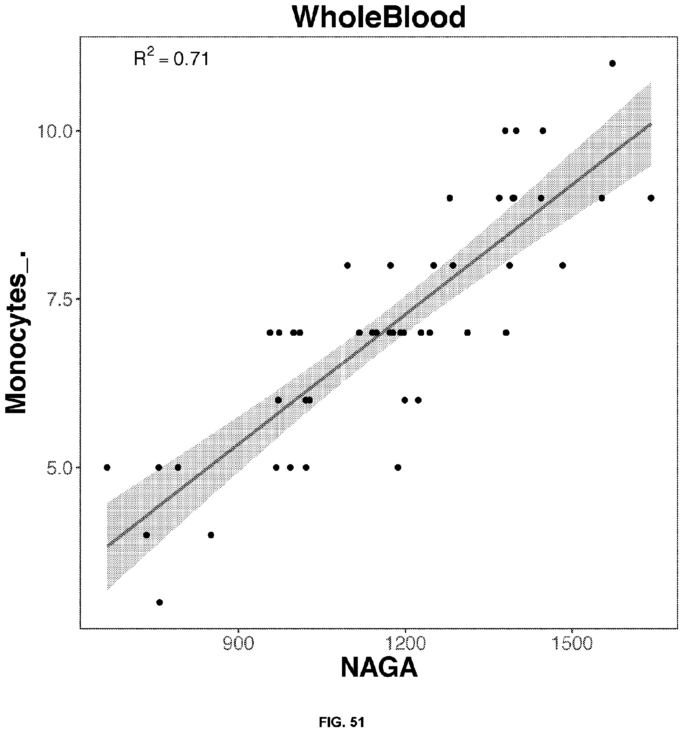

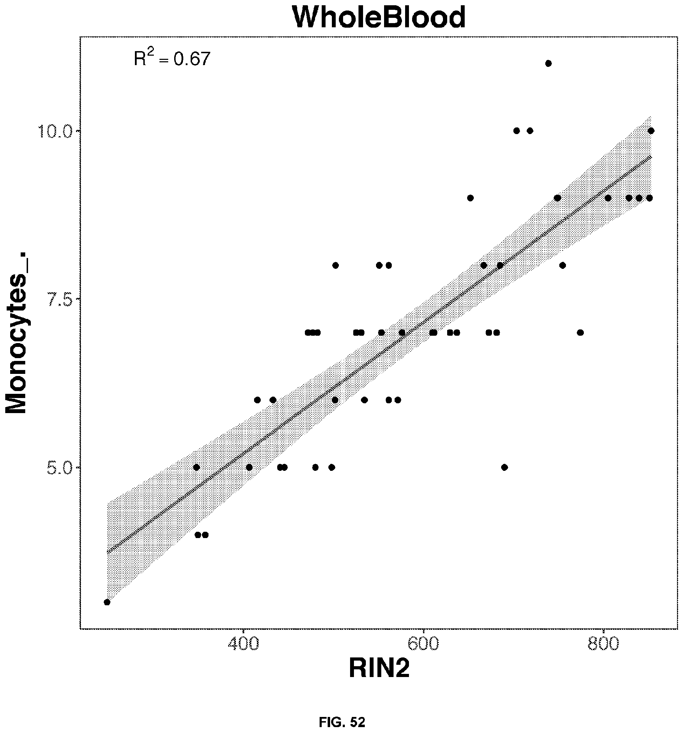

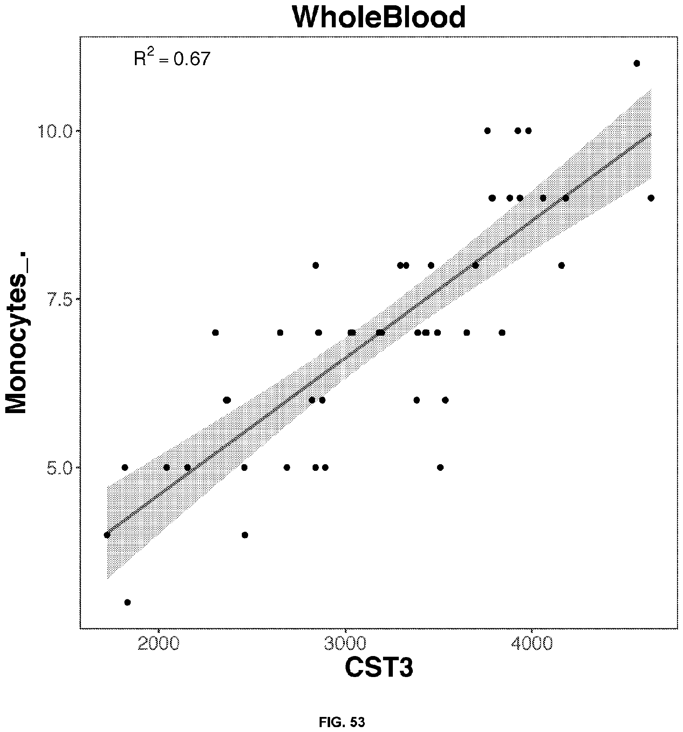

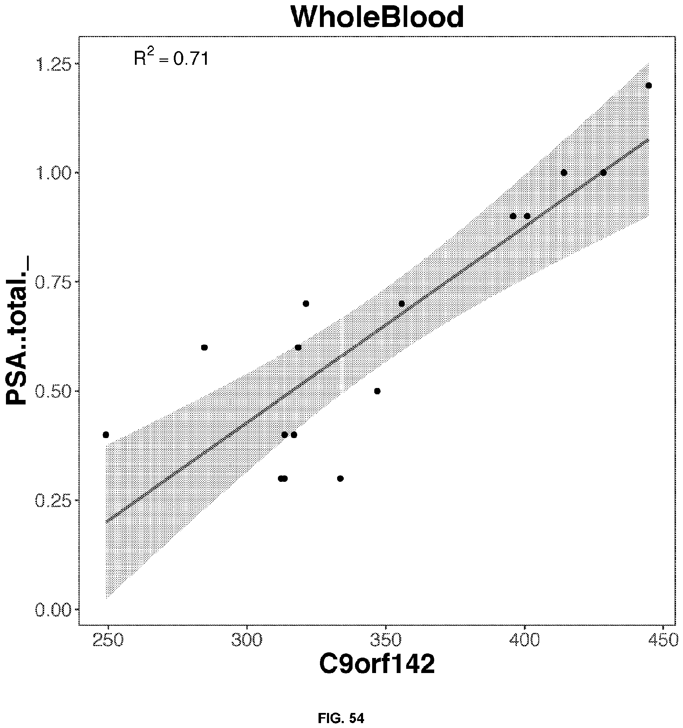

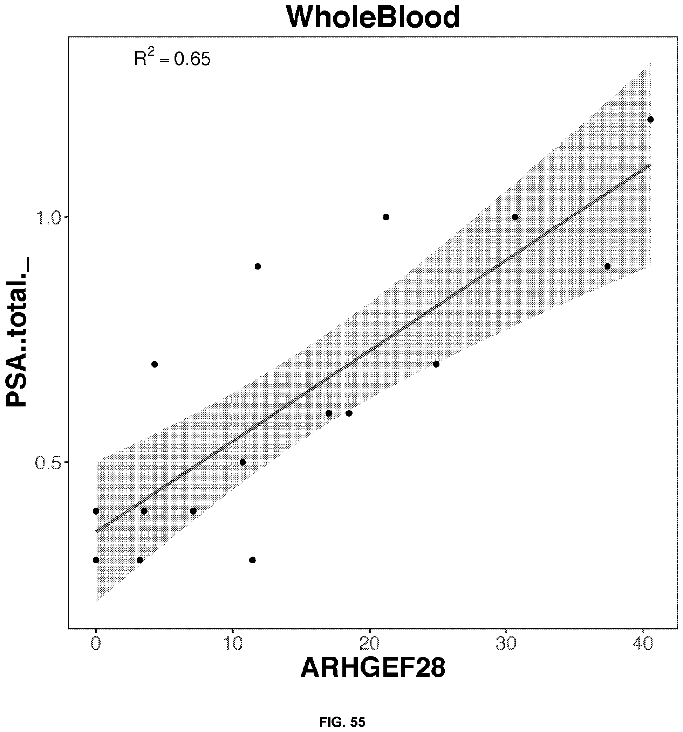

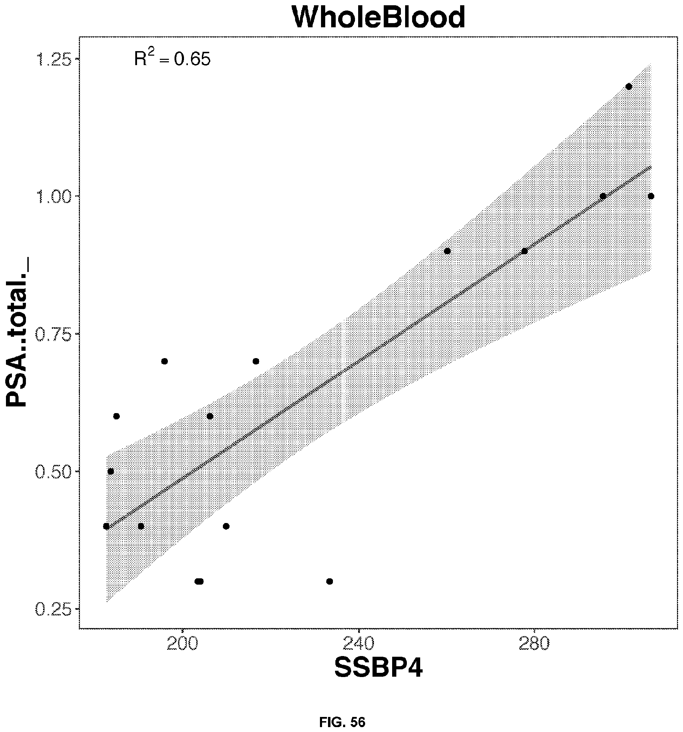

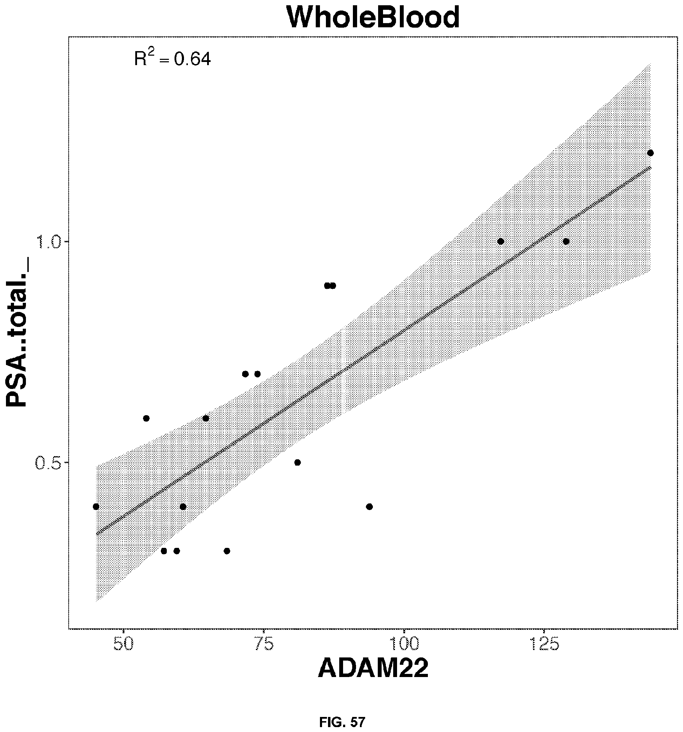

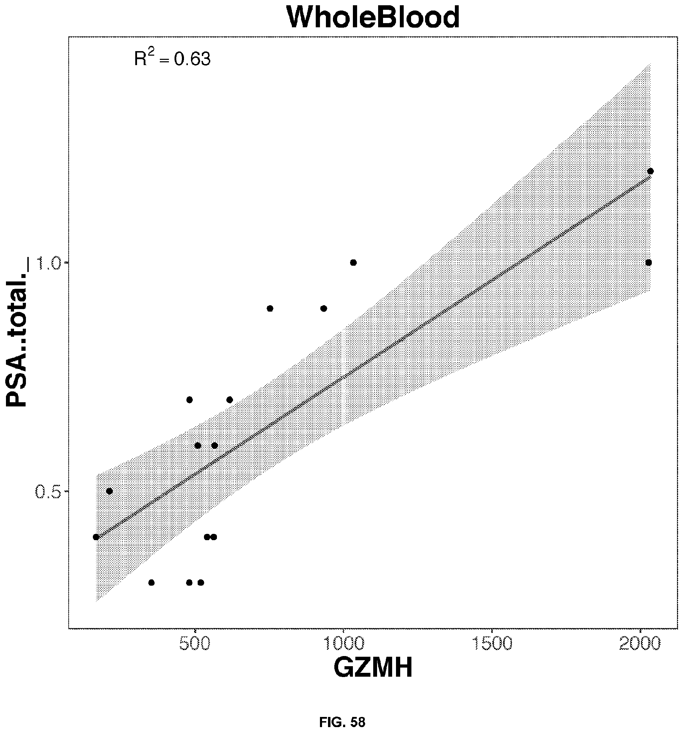

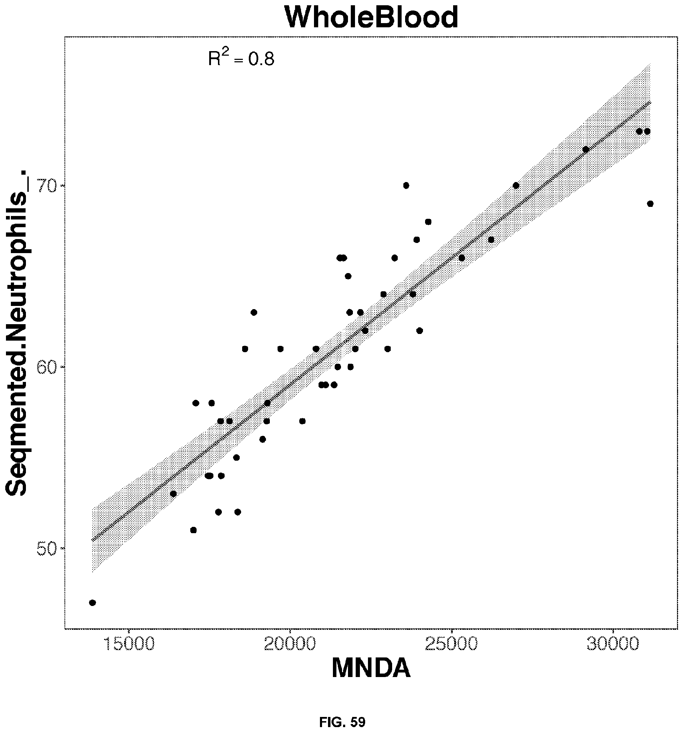

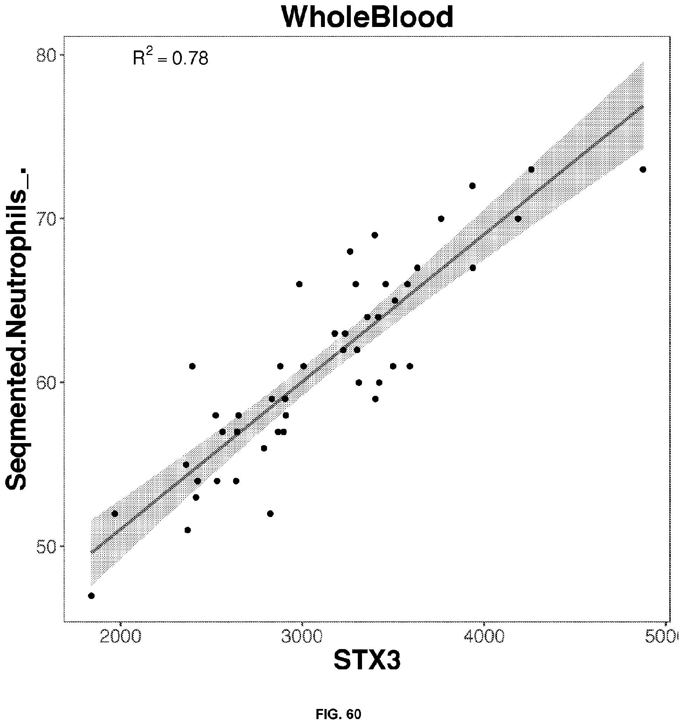

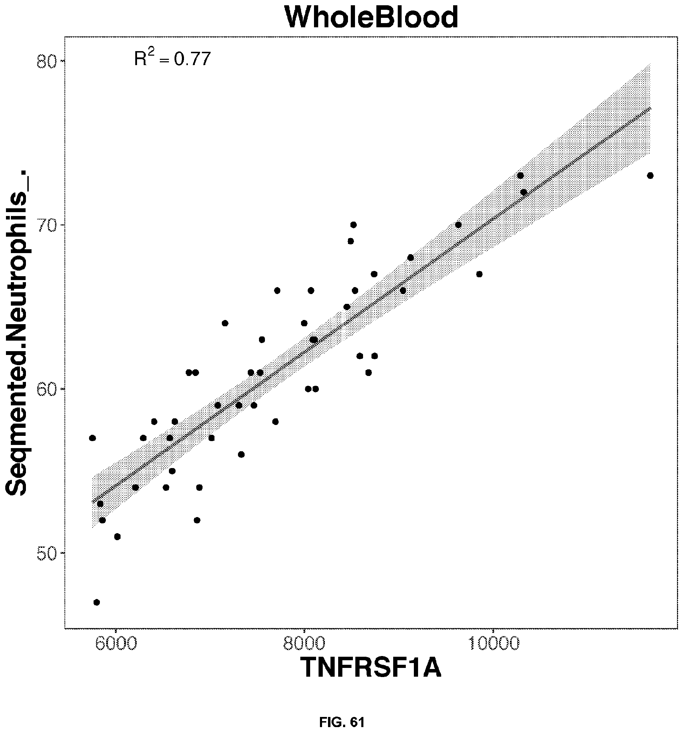

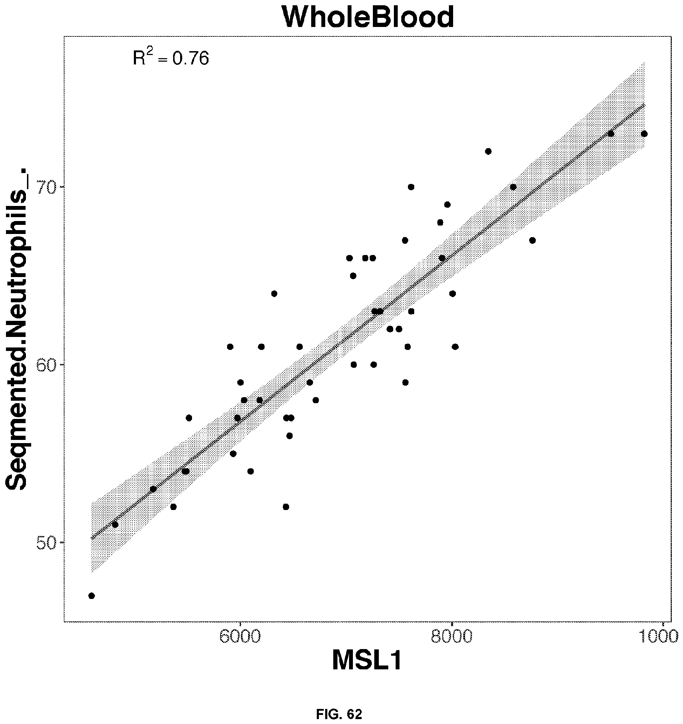

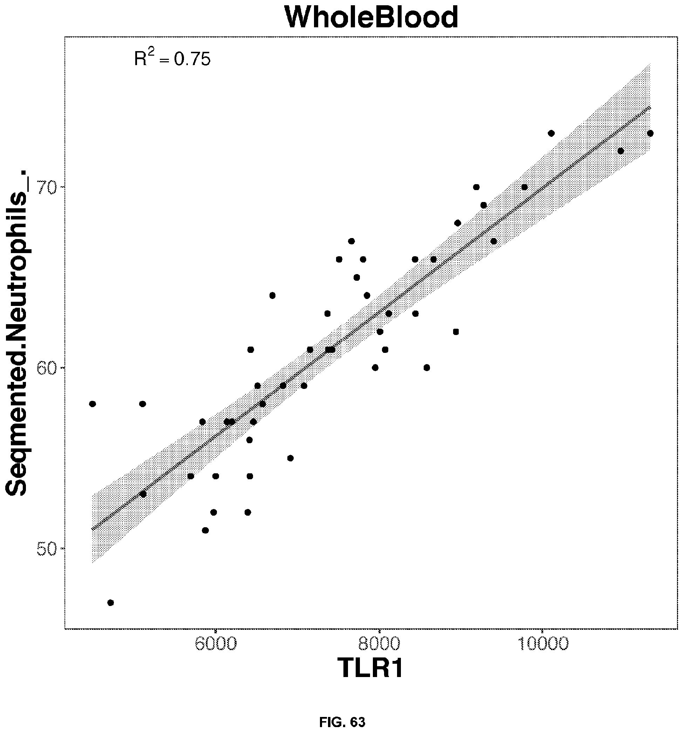

FIGs. 34-63 depict the simple regression graphs of the RNA expression of a gene in whole blood samples with the results of a blood test for highly predictive single genes.

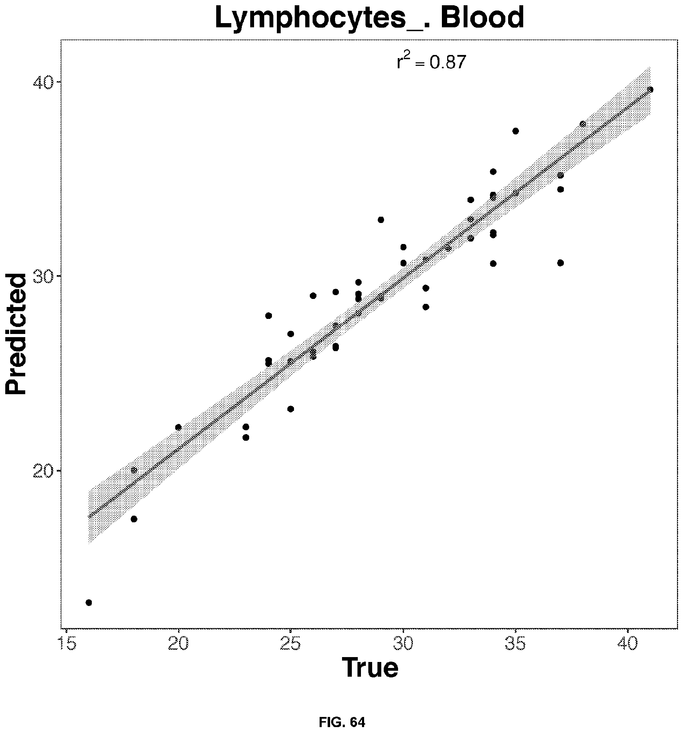

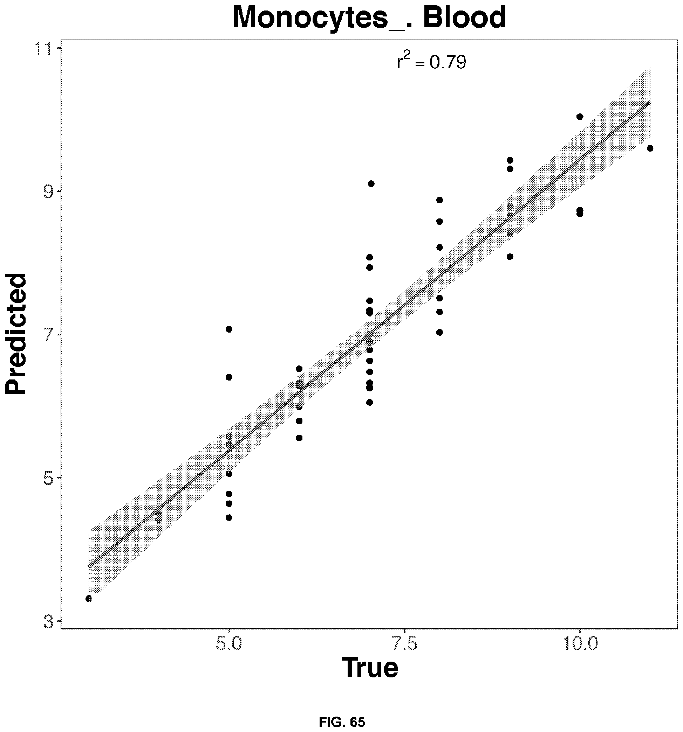

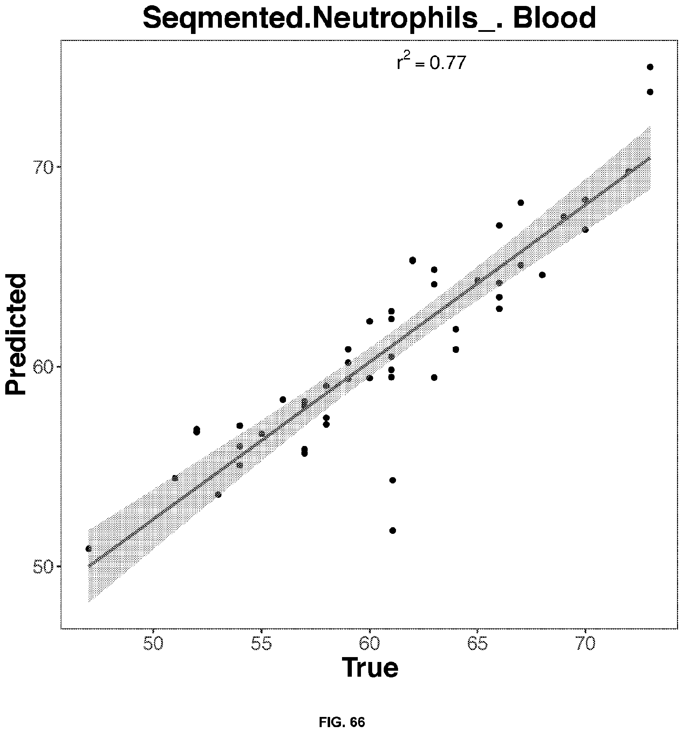

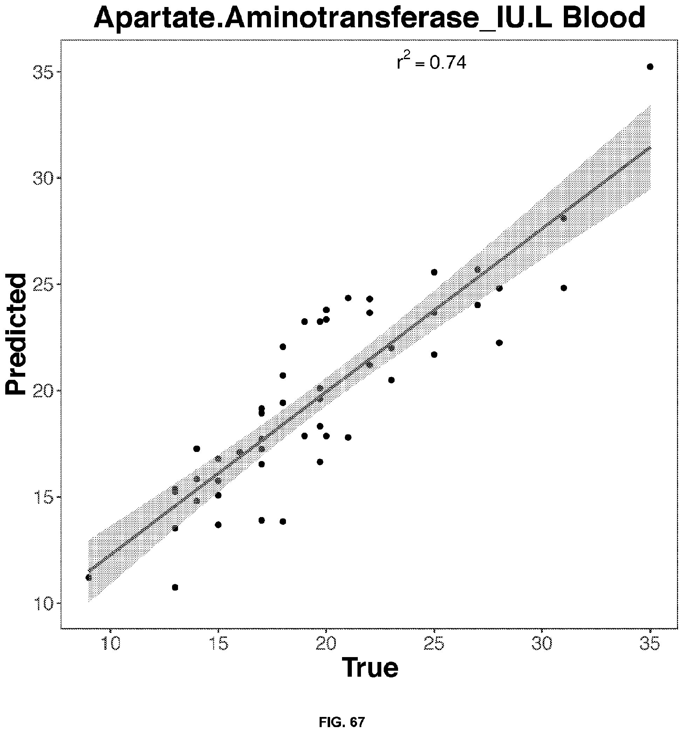

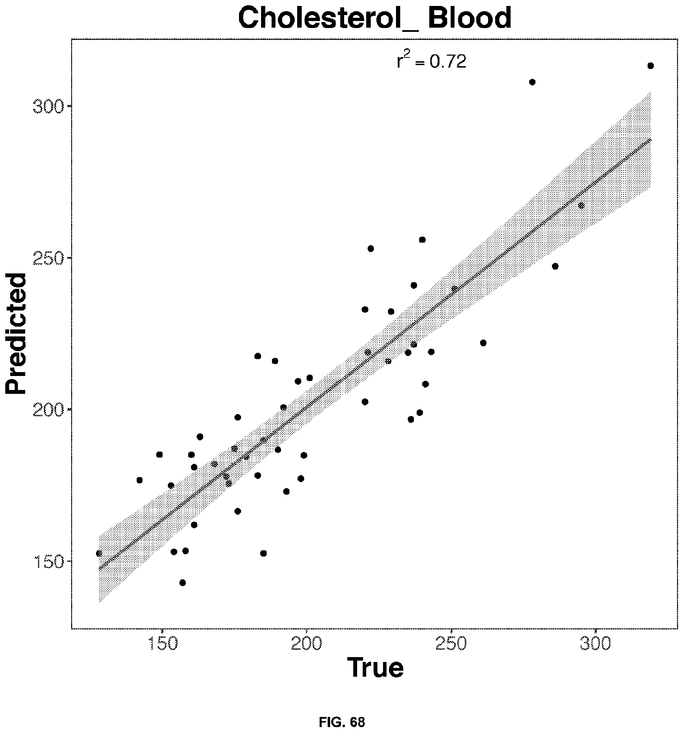

FIGs. 64-68 depict the 2D representation of the multiple regression graphs of the RNA expression of a combination of genes in whole blood samples with the results of a blood test. The R2 value (correlations score) shown are for the real analysis rather than the line of best fit for the 2D representation. The genes used in the multiple regression analysis for each blood result test is identified in Table 4.

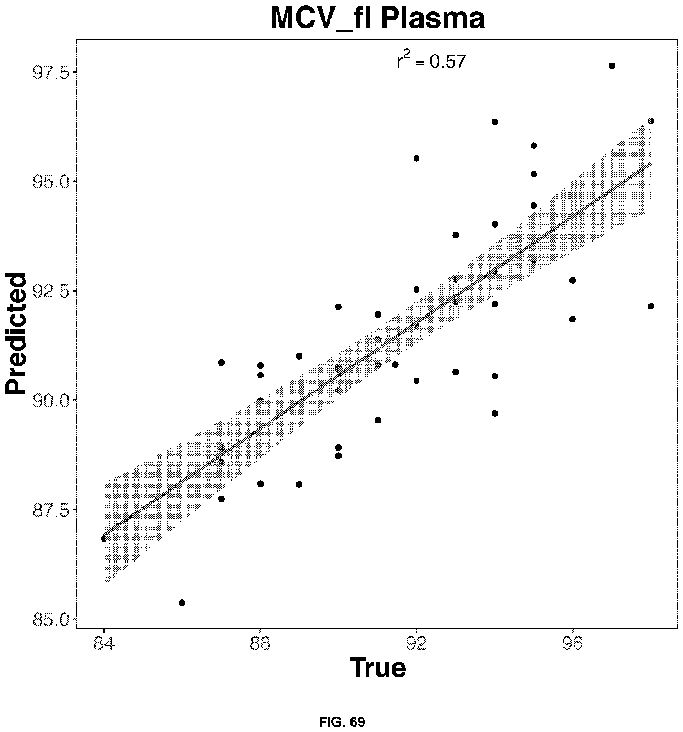

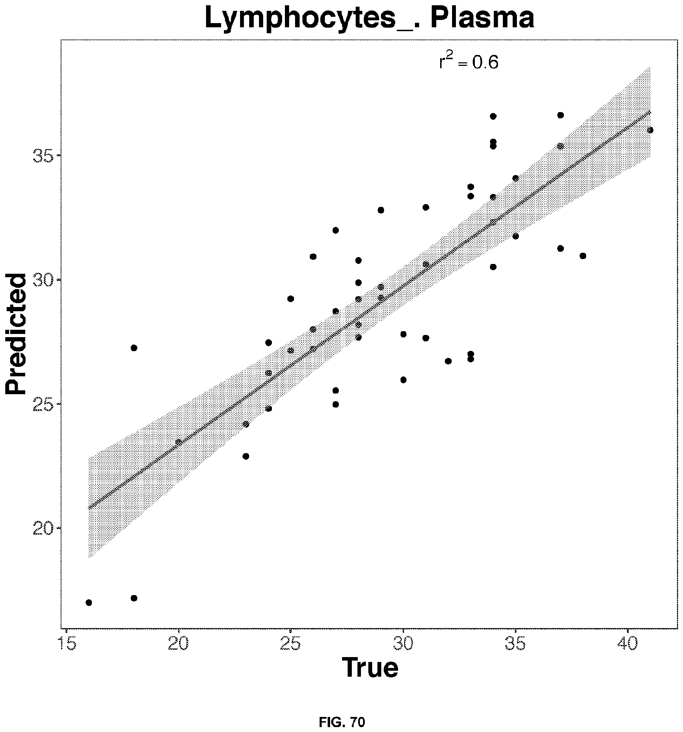

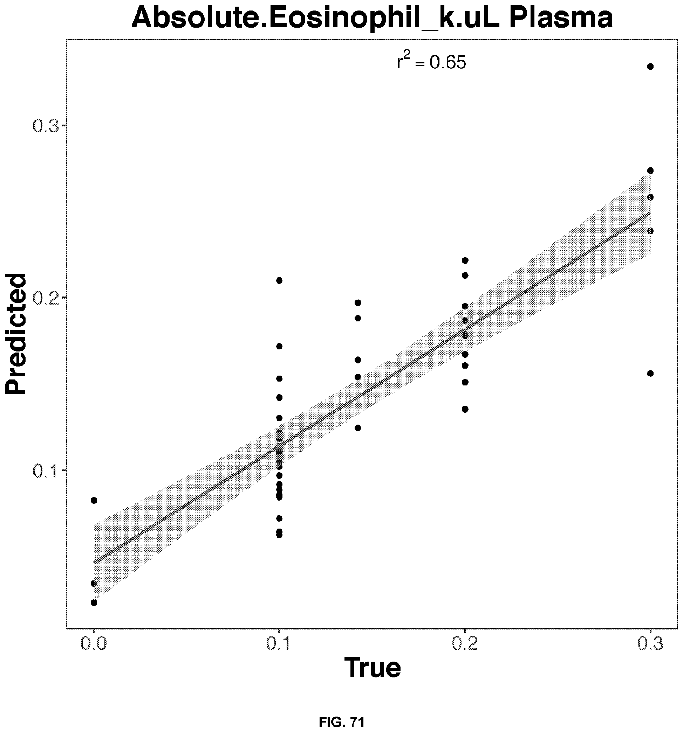

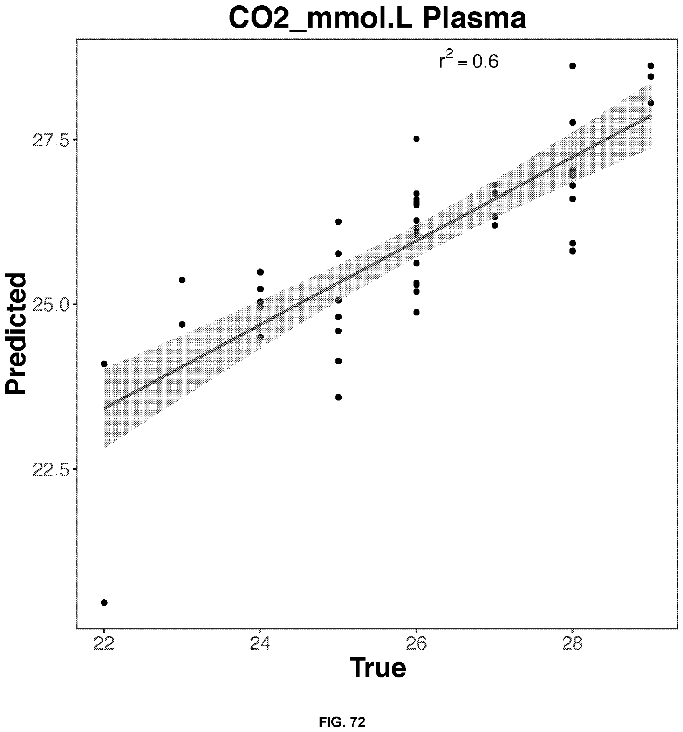

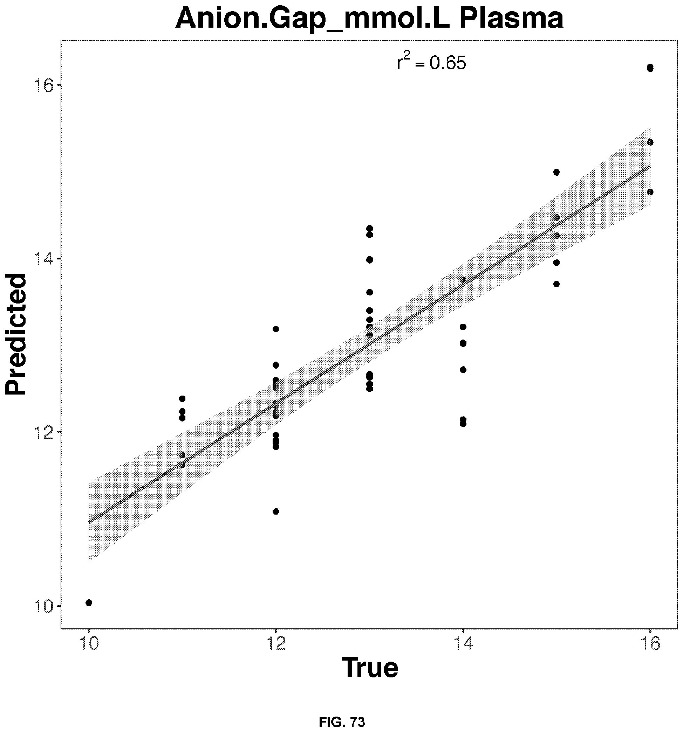

FIGs. 69-73 depict the 2D representation of the multiple regression graphs of the RNA expression of a combination of genes in plasma samples with the results of a blood test. The R2 value (correlations score) shown are for the real analysis rather than the line of best fit for the 2D representation. The genes used in the multiple regression analysis for each blood result test is identified in Table 5.

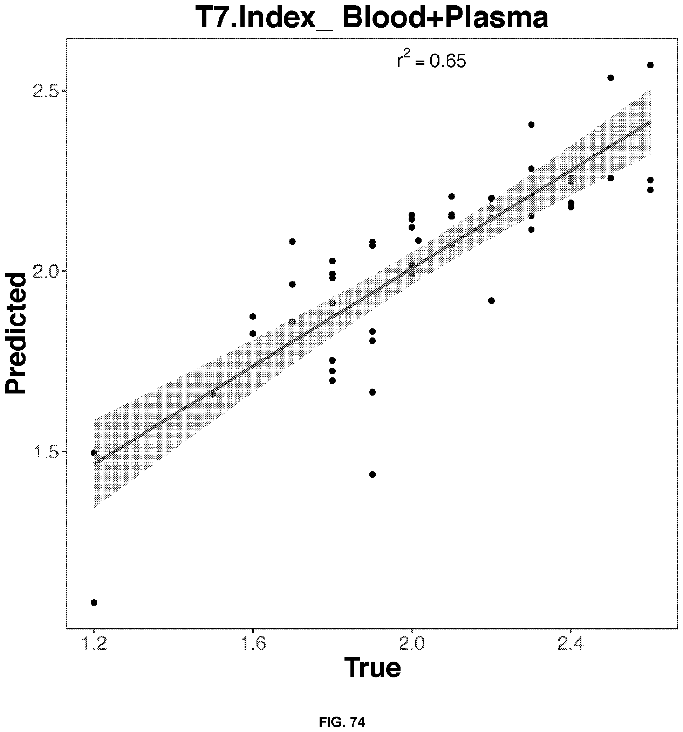

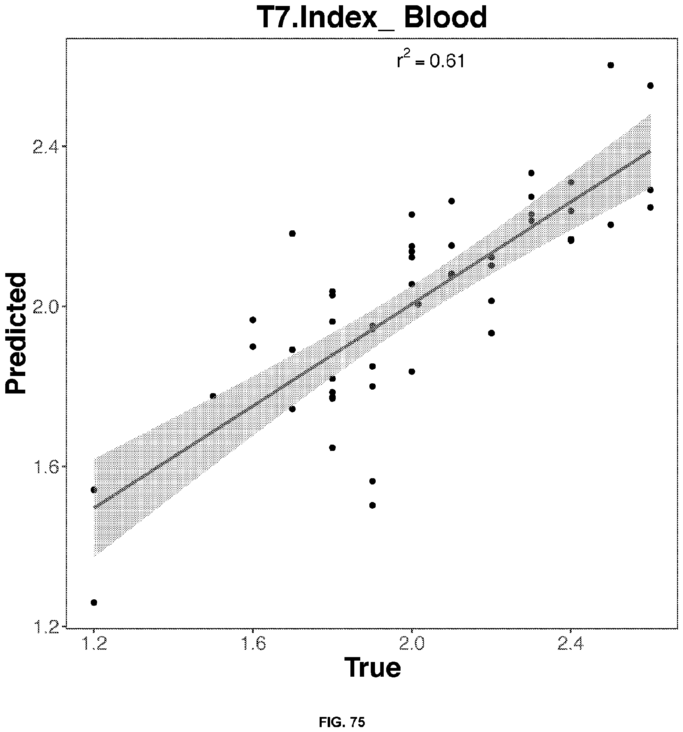

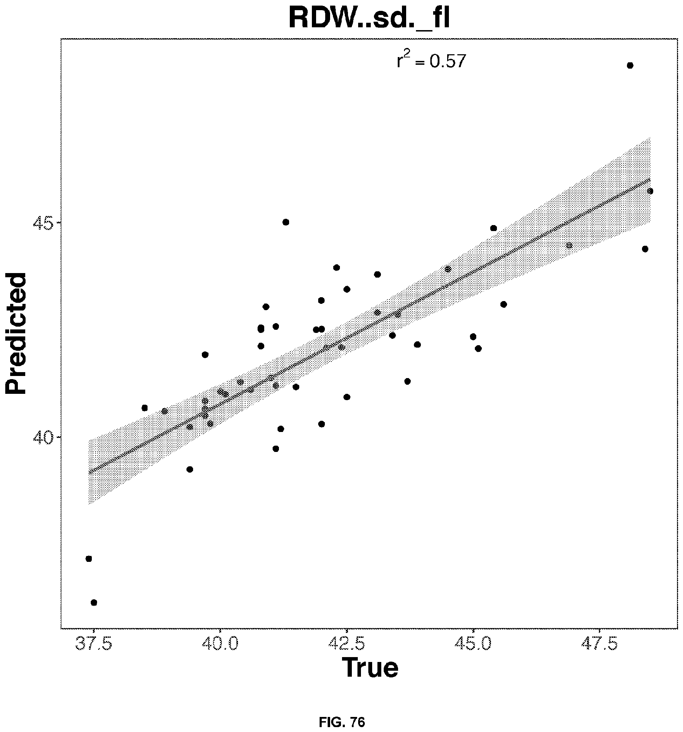

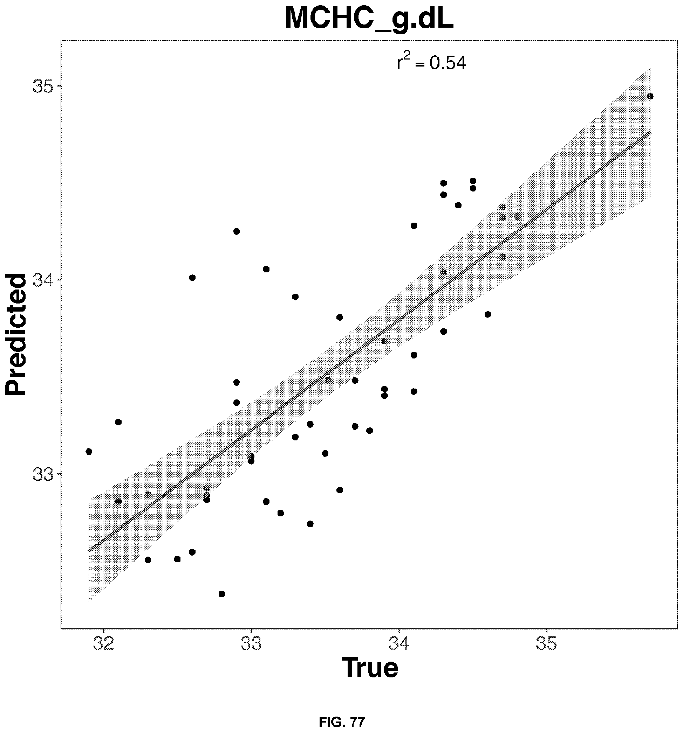

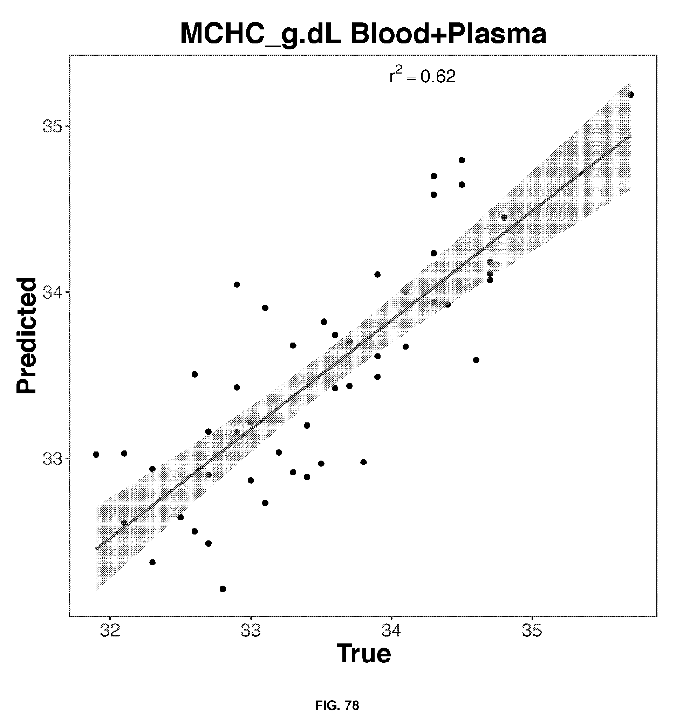

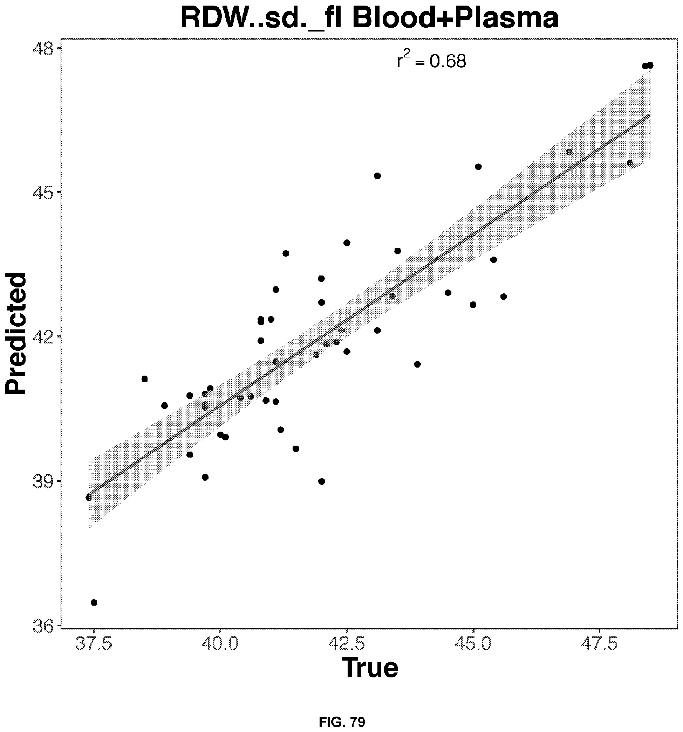

FIGs. 74-79 depict the 2D representation of the multiple regression graphs of the RNA expression of a combination of genes in either whole blood or plasma samples with the results of a blood test. The R2 value (correlations score) shown are for the real analysis rather than the line of best fit for the 2D representation. The genes used in the multiple regression analysis for each blood result test is identified in Table 6.

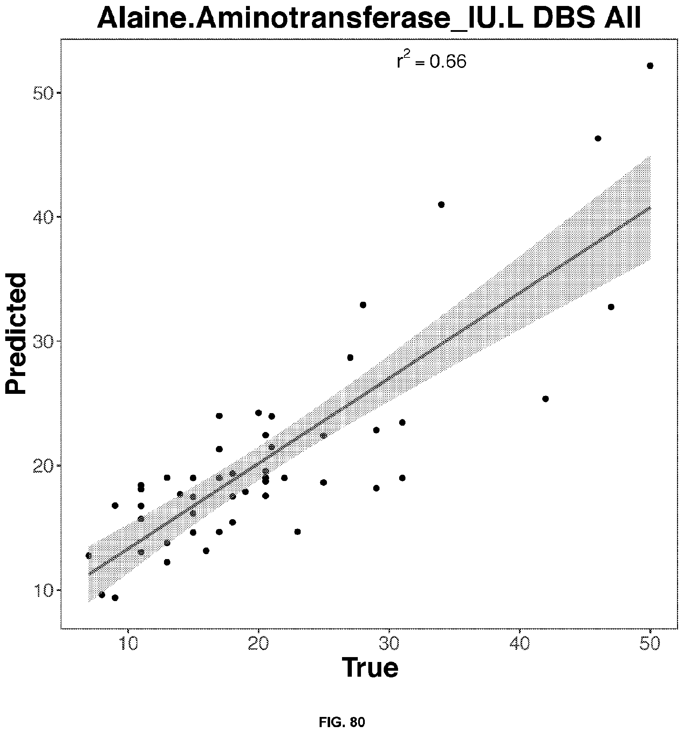

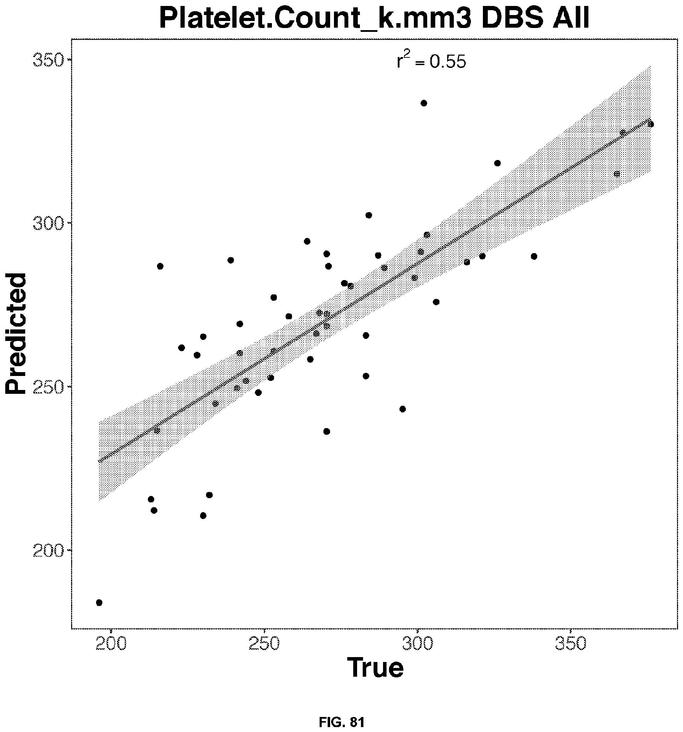

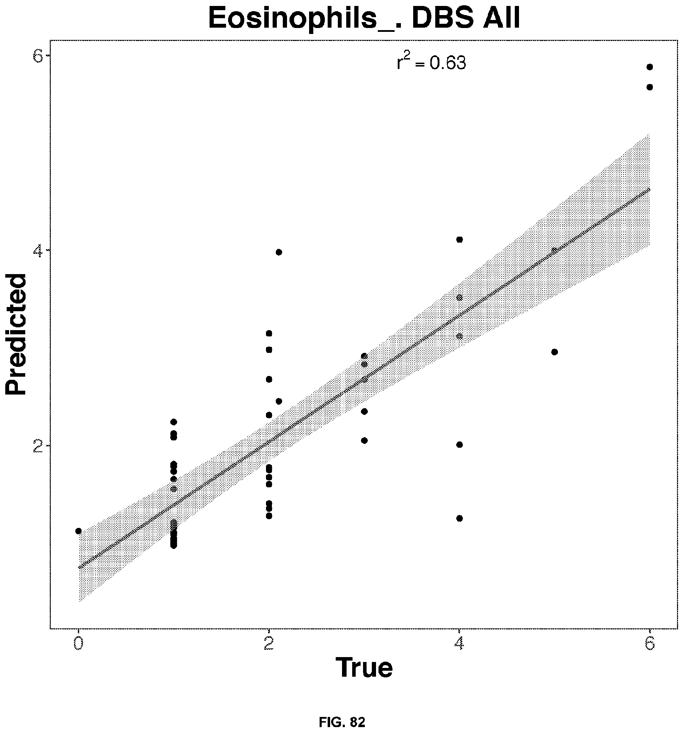

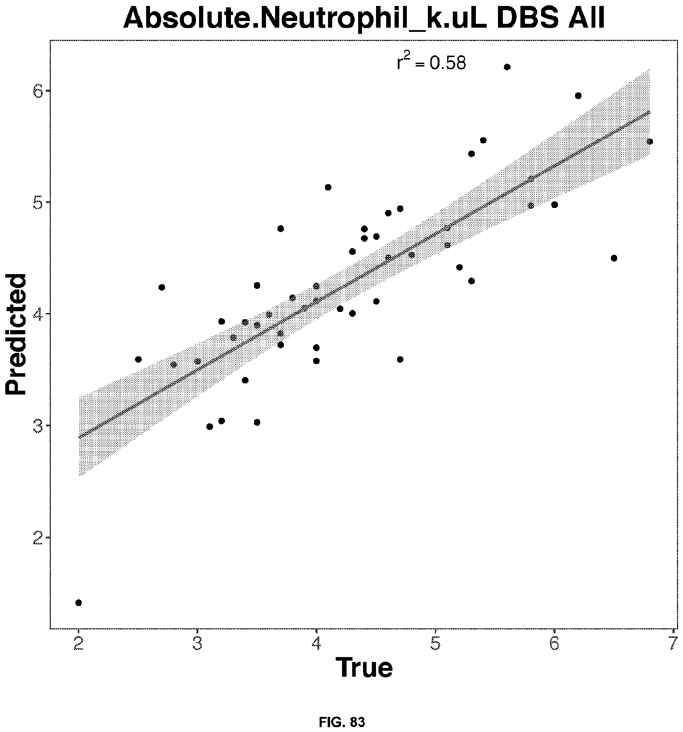

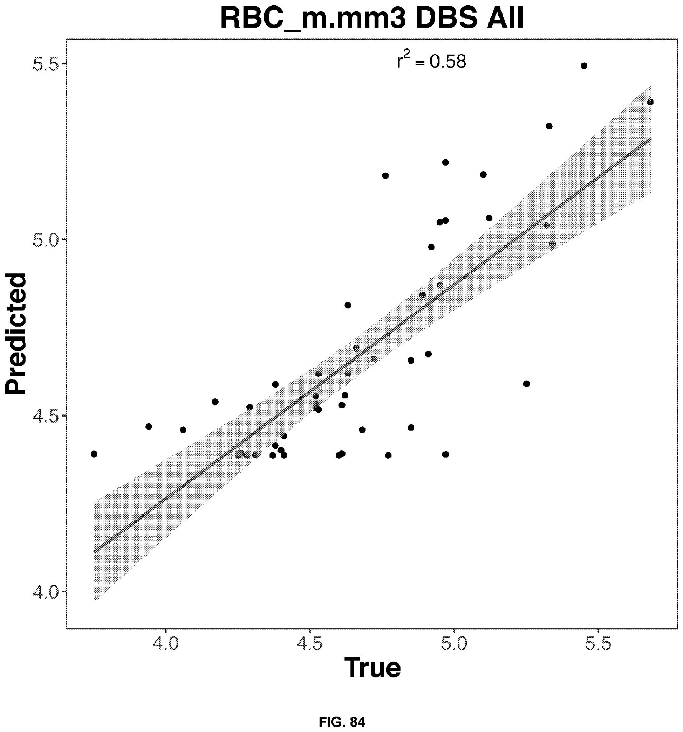

FIGs. 80-84 depict the 2D representation of the multiple regression graphs of the RNA expression of a combination of genes in all dried blood spot samples with the results of a blood test. The R2 value (correlations score) shown are for the real analysis rather than the line of best fit for the 2D representation. The genes used in the multiple regression analysis for each blood result test is identified in Table 7.

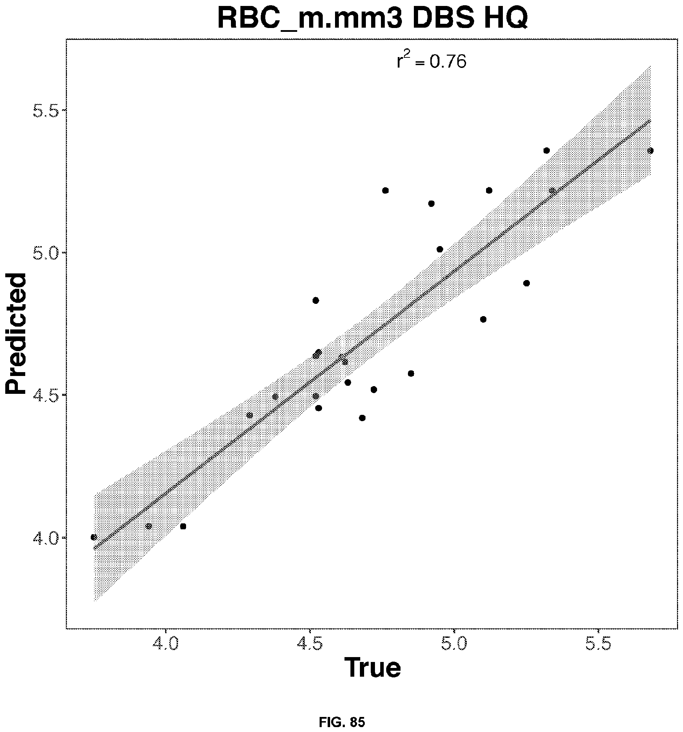

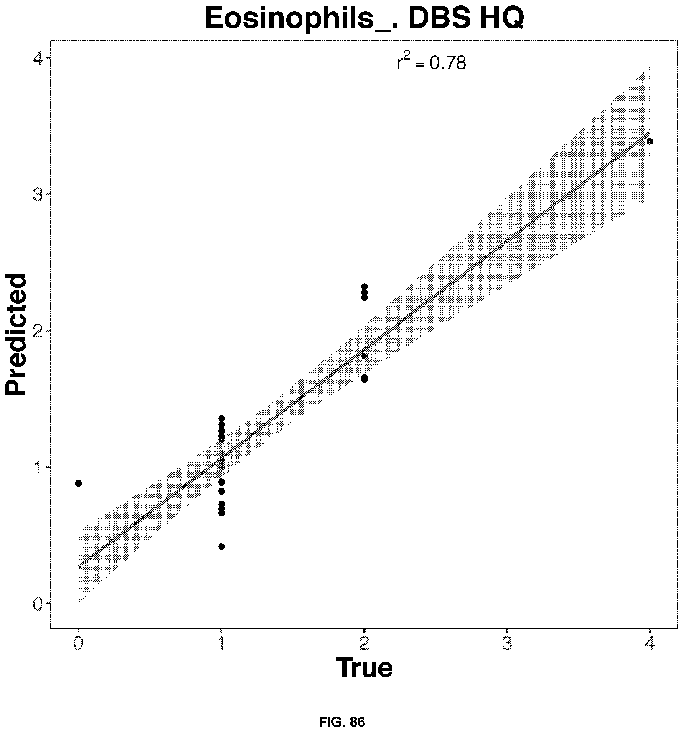

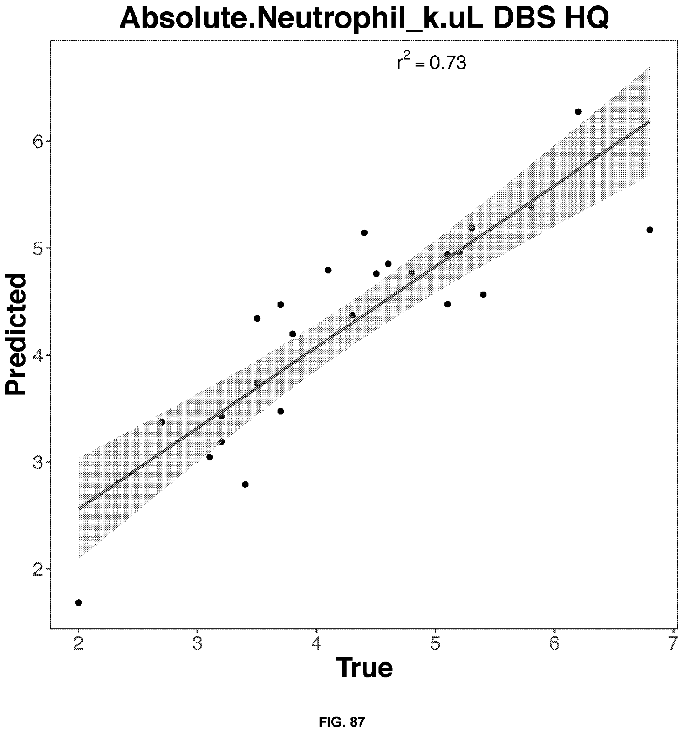

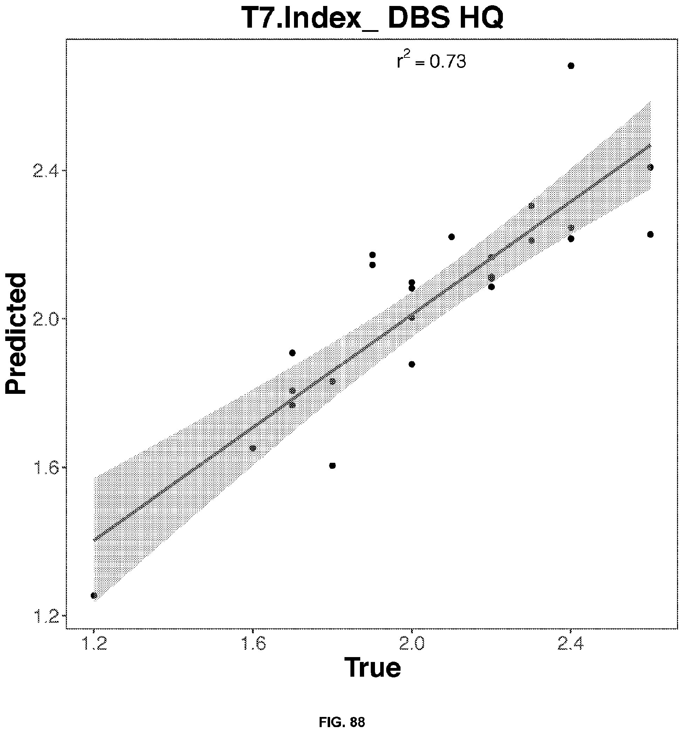

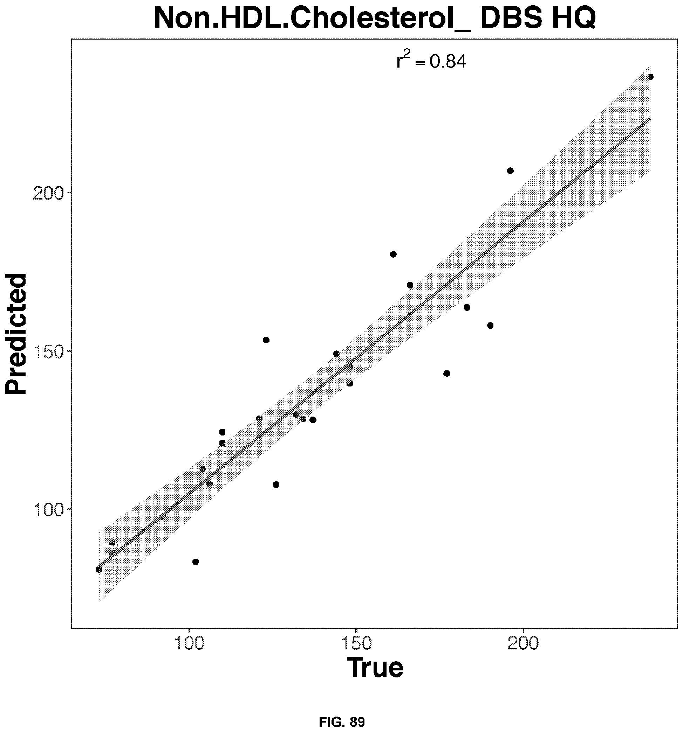

FIGs. 85-89 depict 2D representation of the multiple regression graphs of the RNA expression of a combination of genes in high-quality dried blood spot samples with the results of a blood test. The R2 value (correlations score) shown are for the real analysis rather than the line of best fit for the 2D representation. The genes used in the multiple regression analysis for each blood result test is identified in Table 8.

The headings used in the figures should not be interpreted to limit the scope of the claims.

### DETAILED DESCRIPTION

The disclosure is directed to methods of using biomarker proxies (predictive gene(s)) in predicting the results of standard blood tests based on hematology or chemistry, for example, the results from a complete blood count panel, a comprehensive metabolic panel, a chemistry panel, or an endocrine panel (such as levels of thyroxine, T3, and TSH). Instead of collecting multiple tubes of blood for conducting a variety of tests, a simple blood sample collection, for example of whole blood, plasma, or a dried spot, will enable a determination that correlates to the results of a standard blood test. Accordingly, some embodiments are directed to blood tests for measuring the RNA expression of the biomarker proxies, while other embodiments are directed to methods for determining a blood test result based on the RNA expression of the biomarkers.

In the following description, and for the purposes of explanation, numerous specific details are set forth in order to provide a thorough understanding of the various aspects of the invention. It will be understood, however, by those skilled in the relevant arts, that the present invention may be practiced without these specific details. It should be noted that there are many different and alternative configurations, devices and technologies to which the disclosed inventions may be applied. The full scope of the disclosure is not limited to the examples that are described below.

The singular forms "a," "an," and "the" include plural referents unless the context clearly dictates otherwise. Thus, for example, a reference to "a step" includes reference to one or more of such steps. Unless specifically noted, it is intended that the words and phrases in the specification and the claims be given their plain, ordinary, and accustomed meaning to those of ordinary skill in the applicable arts.

As used herein, the term "subject" refers to any mammal, for example, mice, rats, primates, or humans.

The present disclosure is directed to the discovery of a predictive gene (biomarkers), the expression of which relates to a result of a standard blood test, for example, results for a complete blood count with differential and platelet, a basic chemistry panel, a lipid panel, thyroid tests (such as the levels of thyroxine, T3, and thyroid-stimulating hormone (TSH)), or a prostate-specific antigen (PSA) test.

The inventors disclose a method of performing a blood test. The method typically comprises the steps of: extracting an RNA (total RNA or mRNA) from a blood sample; quantifying a mRNA level of the predictive gene in the blood sample from the extracted RNA; and converting the mRNA level of the predictive gene in the blood sample into a blood test result. In some aspects, the method further comprising selecting a predictive gene or a set of predictive genes, for example, from Tables 1-9. In some implementations, the mRNA level of the predictive gene relates to a target blood component.

As used herein, the term "blood test" or "standard blood tests" refers to tests conducted that directly measure chemical or hematological components found in blood. The chemical components include T3, T3 uptake, Thyroxine (T4), T7 Index, TSH, PSA, cholesterol (HDL, non-HDL, LDL, and VLDL), cholesterol/HDL ratio, triglyceride, glucose, blood urea nitrogen (BUN), creatinine, BUN/creatine ratio, uric acid, sodium, potassium, chloride, CO2, anion gap, osmolality, total protein, albumin, globulin, albumin/ globulin ratio, calcium, phosphorus (inorganic), alkaline phosphatase, gamma-glutamyl transferase (GGT), alanine aminotransferase, aspartate aminotransferase, lactic dehydrogenase, and bilirubin. The hematological components include white blood cell (WBC), red blood cell (RBC), hemoglobin, hematocrit, mean corpuscular volume (MCV), mean corpuscular hemoglobin (MCH), mean corpuscular hemoglobin concentration (MCHC), platelet count, mean platelet volume, segmented neutrophils, lymphocytes, monocytes, eosinophils, basophils, absolute neutrophil, absolute lymphocyte, absolute monocyte, absolute eosinophil, absolute basophil, immature granulocyte, and absolute granulocyte. Table 10 lists some of the standard blood tests and how they may belong in blood test panels.

The term "blood test result," as used herein, refers to the results from conducting the blood test or standard blood test. The third and fourth columns in Table 10 list the specific blood test and the units of the results of the specific blood test.

In some aspects, the blood test is reported as: an amount of the target blood component; a concentration of the target blood component; a volume of the target blood component; a distribution of the target blood component; a ratio of the target blood component to a second blood component; or combinations thereof. In other aspects, the blood test is reported as a volume ratio of red blood cells to total blood (hematocrit level). In other aspects, the blood test is reported as a volume ratio of mean corpuscular hemoglobin (MCH) to mean corpuscle (cell) (MCV) (mean corpuscular hemoglobin concentration (MCHC)).

Non-limiting examples of the blood tests or target blood components include: Absolute Basophils, Absolute Eosinophil, Absolute Lymphocyte, Absolute Monocyte, Absolute Neutrophil, Alanine Aminotransferase, Albumin, Alkaline Phosphatase, Anion Gap, Aspartate Aminotransferase, Total Bilirubin, Blood Urea Nitrogen (BUN), Calcium, Chloride, Cholesterol, CO2, Creatinine, Eosinophils, Gamma-Glutamyl Transferase (GGT), Globulin, Glucose, HDL Cholesterol, Hemoglobin, Immature Granulocyte, Lactic Dehydrogenase, LDL Cholesterol, Lymphocytes, mean corpuscular hemoglobin (MCH), mean corpuscle (cell) volume (MCV), Monocytes, mean platelet volume (MPV), Non-HDL Cholesterol, Osmolality, Inorganic Phosphorus, Platelet Count, Potassium, Total Protein, Red Blood Cell (RBC), red cell distribution width (RDW), Segmented Neutrophils, Sodium, Total T3, T3 Uptake, T7 Index, Thyroxine (T4), Triglycerides, Thyroid Stimulating Hormone (TSH), Uric Acid, VLDL Cholesterol, and White Blood Cell (WBC).

In preferred embodiments, the blood sample is whole blood, plasma, dried blood spot, or combinations thereof. Non-limiting examples of target blood component include: Segmented Neutrophils, Eosinophils, Prostate-Specific Antigen, red blood cells, monocytes, creatinine, lymphocytes, eosinophil, alanine aminotransferase, electrolytes, or non-HDL cholesterol, *etc.* Non-limiting examples of blood test include: red blood Cell count (RBC_m.mm3), Absolute Eosinophil, red cell distribution width (RDW_sd), Thyroid Index (T7), or Anion Gap (AG), *etc.*

In one aspect, the present disclosure is directed to a method of determining a blood test result, *e.g.,* an amount of a target blood component, a concentration of a target blood component, a volume of a target blood component, a distribution of a target blood component, and a ratio between a target blood component and a second target blood component.

The present disclosure is also directed to methods of quantifying a target blood component in a blood sample. Typically, the methods comprising the steps of: extracting an RNA from a blood sample; selecting a predictive gene from Tables 1-9; measuring an mRNA level of the predictive gene (from the extracted RNA of the blood sample) in the blood sample; and converting the mRNA level of the predictive gene in the blood sample into an amount or ratio of the target blood component in the blood sample. In some embodiments, the target blood component is a chemical component, while in other embodiments, the target blood component is a hematological component.

As used herein, the term "blood sample" refers to a sample collected using blood, for example, a whole blood sample, a plasma sample, or a dried blood spot (DBS). The methodologies of the present invention can be used in conjunction with a small quantity of a blood sample. In some implementations, the volume of the blood sample is less than 1 ml (cubic centimeter, cc). In preferred implementations, the volume of the blood sample is less than 0.1 ml (cc), e.g., about 30 µl.

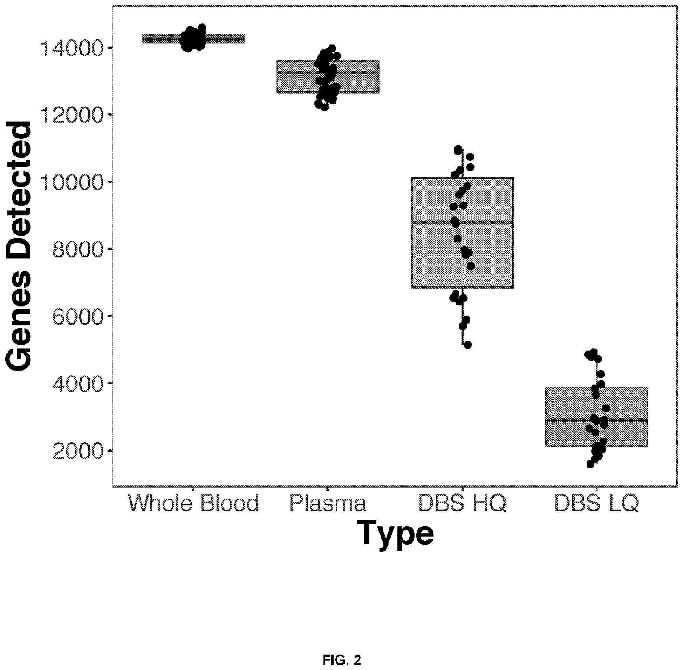

Not all dried blood spots are quality samples for providing predictive RNA expression levels (see FIG. 2), as some dried blood spots (referenced as low-quality dried blood spots, "DBS LQ") can only provide information for less than half the number of genes than other dried blood spots (referenced as high-quality dried blood spots, "DBS HQ"). Accordingly, if RNA expression from dried blood spots is used to predict blood test results, the dried blood spot is preferably analyzed for the number of genes detectable from the sample. If at least 5,000 genes can be detected from the dried blood spot sample, then the dried blood spot is a high-quality sample and provides a more accurate prediction of the blood test results.

In some aspects, the quality of the dried blood spot is determined by assessing the quality of the extracted RNA, for example, by capillary electrophoresis (*e.g*., using an Agilent Bioanalyzer). In some aspects, the RNA quality is quantified as a RIN, wherein the RIN is calculated by an algorithmic assessment of the number of various RNAs presented within the extracted RNA. High-quality cellular RNA generally exhibits an RNA value approaching 10. In yet further aspects, the predictive gene is selected based on the quality of the blood sample. For example, if a dried blood sample is determined to be of high-quality, the predictive gene can be selected from Table 8.

The term "extraction" as used herein refers to any method for separating or isolating the nucleic acids from a sample, more particularly from a biological sample, such as a blood sample. Nucleic acids such as RNA or DNA may be released, for example, by cell lysis. Moreover, in some aspects, extraction may encompass the separation or isolation of coding RNA (mRNA).

Some embodiments of the invention include the extraction of one or more forms of nucleic acids from one or more samples. In some aspects, the extraction of the nucleic acids can be provided using one or more techniques known in the art. For example, in some aspects, the extraction steps can be accomplished using the QIAAMP^{®} RNA Blood Kit from QIAGEN^{®} (*e.g*., for the isolation of total RNA) or EXORNEASY^{®} Serum/Plasma Kit from QIAGEN^{®} (*e.g*., for the isolation of intracellular and/or extracellular RNA). In other embodiments, methodologies of the invention can use any other conventional methodology and/or product intended for the isolation of intracellular and/or extracellular nucleic acids *(e.g.,* RNA).

The term "nucleic acid" or "polynucleotide" as referred to herein comprises all forms of RNA (mRNA, miRNA, rRNA, tRNA, piRNA, ncRNA), DNA (genomic DNA or mtDNA), as well as recombinant RNA and DNA molecules or analogs of DNA or RNA generated using nucleotide analogues. The nucleic acids may be single-stranded or doublestranded. The nucleic acids may include the coding or non-coding strands. The term also comprises fragments of nucleic acids, such as naturally occurring RNA or DNA which may be recovered using one or more extraction methods disclosed herein. "Fragment" refers to a portion of nucleic acid *(e.g.,* RNA or DNA).

The term "library," as used herein refers to a library of genome/transcriptome-derived sequences. The library may also have sequences allowing amplification of the "library" by the polymerase chain reaction or other in vitro amplification methods well known to those skilled in the art. In various embodiments, the library may have sequences that are compatible with next-generation high throughput sequencing platforms. In some embodiments, as a part of the sample preparation process, "barcodes" may be associated with each sample. In this process, short oligonucleotides are added to primers, where each different sample uses a different oligo in addition to a primer.

In certain embodiments, primers and barcodes are ligated to each sample as part of the library generation process. Thus during the amplification process associated with generating the ion amplicon library, the primer and the short oligo are also amplified. As the association of the barcode is done as part of the library preparation process, it is possible to use more than one library, and thus more than one sample. Synthetic nucleic acid barcodes may be included as part of the primer, where a different synthetic nucleic acid barcode may be used for each library. In some embodiments, different libraries may be mixed as they are introduced to a flow cell, and the identity of each sample may be determined as part of the sequencing process.

The term "expression" or "expression level" is used broadly to include a genomic expression profile, *e.g.,* an expression profile of nucleic acids. Profiles may be generated by any convenient means for determining a level of a nucleic acid sequence, e.g., quantitative hybridization of nucleic acid, labeled nucleic acid, amplified nucleic acid, cDNA, *etc.,* quantitative PCR, ELISA for quantitation, sequencing (*e.g*., RNA sequencing) and the like. According to some embodiments, the term "expression level" means measuring the abundance of the nucleic acid in the measured samples.

Expression level or other determinable traits regarding nucleic acids may function as one or more markers or biomarkers. As described herein, the expression level of the one or more biomarkers may be correlated with a blood test result and may be indicative of or predictive of a presence or stage of a disease, condition, or medical state. As such, embodiments of the invention can be employed in medically related analyses to diagnose, assess, provide prognostic information, and make therapeutic decisions regarding any biologically related state.

The expression of these RNA markers from a blood sample determine blood test results with an accuracy of at least 80% when comparing the predicted blood test result based on the RNA markers to the actual blood test result. In particular, these RNA markers determine results in a complete blood count, a comprehensive metabolic panel, and a chemistry panel, and the levels of thyroxine, T3, and TSH an accuracy of at least 80%. In some aspects, accuracy is determined based on regression analysis from the R²-value.

The mRNA level is determined, for example, using RNA sequencing, quantitative PCR *(e.g.,* real-time RT-PCR), or hybridization *(e.g.,* DNA microarray), *etc.* In preferred embodiments, the mRNA level is determined using next-generation sequencing. The methods of determining the expression of RNA from a dried blood spot is explained in PCT Application No. PCT/US2016/038243.

In some implementations, the methods further comprise standardizing the level of RNA expression of the predictive gene.

In other implementations, the methods further comprise normalizing the mRNA level of the predictive gene. In some embodiments, the mRNA level of the predictive gene is normalized according to a method of differential analysis. In some aspects, the count data from next-generation sequencing is normalized using an algorithm. Any normalization algorithm normalization that normalizes library size may be used to normalize the mRNA level of the predictive gene. Non-limiting examples include a DESeq2 algorithm, or edgeR algorithm, *etc.* In some aspects, the mRNA level of the predictive gene is expressed as a normalized gene count. In these aspects, the normalized gene count is used to report the blood test result *(e.g.,* an amount of the target component in the blood sample).

In some embodiments, the methods encompass converting a mRNA level of a single predictive gene in a blood sample into a blood test result using the formula: blood test result = C + C₁*(gene). C and C₁ are constants, and (gene) represents the mRNA level of the predictive gene. In some aspects, (gene) represents normalized gene count. In other aspects, a normalized gene count of a single predictive gene in a blood sample is converted into a blood test result according to a formula set forth in Tables 1-3. In some embodiments, the range of C and C₁ are ±30% of the disclosed value. For example, for formula 0.153698762623272+2.5434273948207*SMPD3, C is between 0.11 and 0.20, and C₁ is between 1.8 and 3.3. In preferred embodiments, the range of C and C₁ are ±20% of the disclosed value. For the same formula, C is between 0.12 and 0.18, and C₁ is between 2.0 and 3.1. In the most preferred embodiments, the range of C and C₁ are ±10% of the disclosed value. For the same formula, C is between 0.14 and 0.17, and C₁ is between 2.3 and 2.8.

In other embodiments, the methods encompass converting a mRNA level of each of a set of predictive genes in a blood sample into a blood test result using the formula: blood test result = C + C₁*(gene₁) + C₂*(gene₂) + ... + Cₙ*(geneₙ), n is 1, 2, 3, 4, or 5, C, C₁, C₂, ... and Cₙ are constants, and (gene₁), (gene₂), ..., and (geneₙ) represent the mRNA level of gene₁, gene₂ ..., and geneₙ. In some aspects, (gene₁), (gene₂), ..., and (geneₙ) represents the normalized gene count for each predictive gene within the set. C and Cₙ may be positive or negative. In certain non-limiting aspects, the blood sample is a dried blood spot, and n is 1, 2, or 3. In some aspects, a set of normalized gene counts of a set of predictive genes in a blood sample is converted into a blood test result according to a formula set forth in Tables 4-9. In some aspects, C, C₁, ... Cₙ is ±30% of the disclosed value. In other aspects, C, C₁, ... Cₙ is ±20% of the disclosed value. In further aspects, C, C₁, ... Cₙ is ±10% of the disclosed value.

In some implementations, a range in the mRNA level of the predictive gene corresponds to the normal range in the results of a blood test. Accordingly, detecting the mRNA level of genes listed in Tables 1-9 below replaces the need for conducting standard blood tests. Whereas conventional blood tests usually require a visit to a laboratory to get blood drawn as each blood test may have particular requirements for the blood collection process, the methods of the invention simplify the process of monitoring of a subject's state of health. One such benefit is that a single sample collection where a relatively small amount of blood is collected replaces the need to collect multiple tubes of blood by a visit to a laboratory. In the examples, a total of 1 cc of blood was collected for the whole blood sample and the generation of the plasma sample, whereas the typical collection volume for blood tests is 8 cc per tube of blood. In some implementations, less than 1 cc blood needs to be collected. In the case of the dried blood sample, a blood smear or the amount of blood released from a typical finger prick (for example, for blood sugar monitoring) is sufficient. Dried blood spot samples may also be easily kept in storage in case other blood tests analysis needs to be conducted on the sample, for example, if additional analysis is needed weeks, months, or years after collection of the dried blood sample. Another exemplary benefit of the invention is that one can track health status without the need to visit a laboratory or blood collection site. Instead, the subject may collect his or her own sample and send the sample for analysis in a laboratory. This is particularly convenient for subjects who cannot make the required visits to a laboratory, for example, ailing house-bound subjects or those residing far from a laboratory. Often, the former group of subjects has the most need for careful monitoring of their health status.

Tables 1-3 list the blood test results and the single most predictive genes based on the gene's mRNA level in whole blood, dried blood spot, and plasma samples respectively. In some aspects, the mRNA level of one or more of the genes listed in Table 1 in a subject's whole blood sample is used to determine the amount of eosinophils, absolute eosinophils, segmented neutrophils, lymphocytes, monocytes, or prostate-specific antigen (PSA) in the subject. In other aspects, the mRNA level of one or more of the genes listed in Table 2 in a subject's dried blood spot sample is used to determine the amount of eosinophils, absolute eosinophils, or PSA in the subject. In yet other aspects, the mRNA level of one or more of the genes listed in Table 3 in a subject's plasma sample is used to determine the amount of creatinine, PSA, red blood cell (RBC), or the mean corpuscular hemoglobin concentration (MCHC) in the subject.

**Table 1. Top predictive gene based on the gene's expression in whole blood samples for each blood test result according to linear regression analysis**

| **Correlation Score** | **Blood Test Result** | **Gene Name** | **Ensemble ID** | **Formula** |
|---|---|---|---|---|
| 0.81 | Eosinophils_. | SLC29A1¹ | ENSG00000112759 | -0.436114553980279+3.12697159781888*SLC29A1 |
| 0.79 | Eosinophils_. | SIGLEC8¹ | ENSG00000105366 | 0.478995513524416+2.26645036634396*SIGLEC8 |
| 0.77 | Eosinophils_. | IL5RA | ENSG00000091181 | -0.0955461742354181+2.81222141861621*IL5RA |
| 0.74 | Eosinophils_. | TMIGD3¹ | ENSG00000121933 | -0.000801764004280439+2.63814405484868*TMIGD3 |
| 0.70 | Eosinophils_. | SMPD3 | ENSG00000103056 | 0.153698762623272+2.5434273948207*SMPD3 |
| 0.80 | Seqmented.Neutrophils_. | MNDA² | ENSG00000163563 | 30.985358159929+30.5084860077407*MNDA |
| 0.78 | Seqmented.Neutrophils_. | STX3 | ENSG00000166900 | 33.0607692672898+28.4228215061986*STX3 |
| 0.77 | Seqmented.Neutrophils_. | TNFRSF1A | ENSG00000067182 | 29.7291893891555+31.7745523363709*TNFRSF1A |
| 0.76 | Seqmented.Neutrophils_. | MSL1 | ENSG00000188895 | 28.7271661674218+32.7254991645035*MSL1 |
| 0.75 | Seqmented.Neutrophils_. | TLR1 | ENSG00000174125 | 35.631442374894+25.9402923721921*TLR1 |
| 0.79 | Lymphocytes_. | EVI2B | ENSG00000185862 | 56.1863937273014+-27.7092017568931*EVI2B |
| 0.77 | Lymphocytes_. | GRB2 | ENSG00000177885 | 66.2749627281548+-37.7780282518198*GRB2 |
| 0.77 | Lymphocytes_. | LAMP2 | ENSG00000005893 | 54.9921800155255+-26.5119940169167*LAMP2 |
| 0.77 | Lymphocytes_. | MNDA² | ENSG00000163563 | 53.8657745533577+-25.3929463761467*MNDA |
| 0.77 | Lymphocytes_. | NFAM1 | ENSG00000235568 | 52.358694909343+-23.8995935882078*NFAM1 |
| 0.71 | PSA..total._ | C9orf142 | ENSG00000148362 | -0.917861007929147+1.69760056628958*C9orf142 |
| 0.65 | PSA..total._ | ARHGEF28 | ENSG00000214944 | 0.357399121217485+0.338880229114067*ARHGEF28 |
| 0.65 | PSA..total._ | SSBP4 | ENSG00000130511 | -0.576221661574983+1.25786772240861*SSBP4 |
| 0.64 | PSA..total._ | ADAM22 | ENSG00000008277 | -0.0422931052522241+0.8800693985004*ADAM22 |
| 0.63 | PSA..total._ | GZMH | ENSG00000100450 | 0.325075313876093+0.32428066183067*GZMH |
| 0.74 | Monocytes_. | CECR1 | ENSG00000093072 | -0.396158811208197+7.73352673027494*CECR1 |
| 0.72 | Monocytes_. | PLXNB2 | ENSG00000196576 | 1.04851193227865+6.31022452456754*PLXNB2 |
| 0.71 | Monocytes_. | NAGA | ENSG00000198951 | -0.427809486988276+7.72986242198722*NAGA |
| 0.67 | Monocytes_. | RIN2 | ENSG00000132669 | 1.30473405937088+5.96955611215279*RIN2 |
| 0.67 | Monocytes_. | CST3 | ENSG00000101439 | 0.523411654697532+6.85269617206023*CST3 |
| 0.68 | Absolute.Eosinophil_k.uL | SLC29A1 | ENSG00000112759 | -0.00476612865203703+0.197930659524045*SLC29A1 |
| 0.65 | Absolute.Eosinophil_k.uL | SIGLEC8 | ENSG00000105366 | 0.0535915596920504+0.142306293239556*SIGLEC8 |
| 0.63 | Absolute.Eosinophil_k.uL | IL5RA | ENSG00000091181 | 0.026206273258456+0.16193658999382*IL5RA |
| 0.60 | Absolute.Eosinophil_k.uL | TMIGD3 | ENSG00000121933 | 0.0201738288843809+0.170369927489743*TMIGD3 |
| 0.58 | Absolute Eosinophil_k.uL | SMPD3 | ENSG00000103056 | 0.04135790555759+0.143797323307533*SMPD3 |

| | | | | |
|---|---|---|---|---|
| ¹ Transmembrane proteins ² The myeloid cell nuclear differentiation antigen (MNDA) is detected only in nuclei of cells of the granulocyte-monocyte lineage. MNDA was correlated with the amount of both lymphocytes and neutrophils. However, for lymphocytes, the correlation is negative. | | | | |

**Table 2. Top predictive gene based on the gene's expression in dried blood spot samples for each blood test result according to linear regression analysis**

| **Correlation Score** | **Blood Test Result** | **Gene Name** | **Ensemble ID** | **Formula** |
|---|---|---|---|---|
| 0.81 | PSA..total._ | CTC-265F19.1 | ENSG00000267749 | 0.432690717089027+0.526112710280575*CTC-265F19.1 |
| 0.81 | PSA.total._ | ADAM9 | ENSG00000168615 | 0.43193992452492+1.68403340939593*ADAM9 |
| 0.78 | PSA.total._ | RAB11FIP5 | ENSG00000135631 | 0.444522689514033+0.593999903134511*RAB 11FIP5 |
| 0.76 | PSA..total._ | SNAPC4 | ENSG00000165684 | 0.444889943948596+0.612746005772941*SNAPC4 |
| 0.76 | PSA..total._ | LMNA | ENSG00000160789 | 0.409986470208812+0.348402891412522*LMNA |
| 0.64 | Eosinophils_. | SCARNA22 | ENSG00000249784 | 1.29455961910828+1.51157194408083*SCARNA22 |
| 0.57 | Eosinophils_. | SNORA36C | ENSG00000207016 | 1.32106746570246+1.4949289970043*SNORA36C |
| 0.54 | Eosinophils_. | SNORA11 | ENSG00000221716 | 1.24052900576161+1.44230554450022*SNORA11 |
| 0.54 | Eosinophils_. | RN7SL4P | ENSG00000263740 | 1.05935580726772+1.57417742477499*RN7SL4P |
| 0.53 | Eosinophils_. | SNHG15 | ENSG00000232956 | 1.40294345290673+ 1.36081 043128595*SNHG15 |
| 0.45 | Absolute.Eosinophil_k.uL | TMSB4X | ENSG00000205542 | 0.0722050887230592+0.102186450139369*TMSB4X |
| 0.41 | Absolute.Eosinophil_k.uL | CCT3 | ENSG00000163468 | 0.215519649778949+-0.085289845232217*CCT3 |
| 0.40 | Absolute.Eosinophil_k.uL | TRIM37 | ENSG00000108395 | 0.195256420982459+-0.0697165663394102*TRIM37 |
| 0.38 | Absolute.Eosinophil_k.uL | C6orf120 | ENSG00000185127 | 0.186400956788973+-0.0636136758785107*C6orf120 |
| 0.38 | Absolute.Eosinophil_k.uL | SCARNA22 | ENSG00000249784 | 0.102654265156325+0.104862734769039*SCARNA22 |

**Table 3. Top predictive gene based on the gene's expression in plasma samples for each blood test result according to linear regression analysis**

| **Correlation Score** | **Blood Test Result** | **Gene Name** | **Ensemble ID** | **Formula** |
|---|---|---|---|---|
| 0.45 | Creatinine_mg.dL | DDX3Y | ENSG00000067048 | 0.793889595070931+0.111042880176709*DDX3Y |
| 0.45 | Creatinine_mg.dL | ZFY | ENSG00000067646 | 0.794717048177349+0.110912224291987*ZFY |
| 0.44 | Creatinine_mg.dL | RPS4Y1 | ENSG00000129824 | 0.797691770712918+0.1063974025239*RPS4Y1 |
| 0.43 | Creatinine_mg.dL | UTY | ENSG00000183878 | 0.79536615038728+0.108780857628159*UTY |
| 0.40 | Creatinine_mg.dL | EIF1AY | ENSG00000198692 | 0.80259827969781+0.102254210816211*EIF1AY |
| 0.48 | RBC_m.mm3 | UTY | ENSG00000183878 | 4.48521231457716+0.344360244986884*UTY |
| 0.45 | RBC_m.mm3 | DDX3Y | ENSG00000067048 | 4.49212644733203+0.333391370781157*DDX3Y |
| 0.45 | RBC_m.mm3 | ZFY | ENSG00000067646 | 4.49582762495249+0.329309620007345*ZFY |
| 0.44 | RBC_m.mm3 | TXLNGY | ENSG00000131002 | 4.50282284950475+0.324347929232679*TXLNGY |
| 0.43 | RBC_m.mm3 | RPS4Y1 | ENSG00000129824 | 4.50659391938372+0.319801899143571*RPS4Y 1 |
| 0.51 | MCHC_g.dL | XRCC5 | ENSG00000079246 | 28.4745390044457+5.10566513836356*XRCC5 |
| 0.42 | MCHC_g.dL | RAD50 | ENSG00000113522 | 31.2775402234416+2.31530729768107*RAD50 |
| 0.38 | MCHC_g.dL | SMARCAD1 | ENSG00000163104 | 31.4895542606002+2.10410631998518*SMARCAD1 |
| 0.38 | MCHC_g.dL | TOP2B | ENSG00000077097 | 29.7561062495169+3.81519551882321*TOP2B |
| 0.38 | MCHC_g.dL | UTRN | ENSG00000152818 | 30.6829018688001+2.89159349570542*UTRN |
| 0.61 | PSA..total._ | HNRNPA3P3 | ENSG00000214653 | 0.210294096516657+0.469934269166632*HNRNPA3P3 |
| 0.58 | PSA..total._ | GTF3A | ENSG00000122034 | -0.372586762800658+0.97034975245291*GTF3A |
| 0.57 | PSA..total._ | RP11-342M1.6 | ENSG00000237090 | 0.396515609660464+0.324918619671395*RP11-342M1.6 |
| 0.55 | PSA..total._ | HNRNPLP2 | ENSG00000259917 | 0.328107495935833+0.317193096343151*HNRNPLP2 |
| 0.54 | PSA..total._ | RPS11P5 | ENSG00000232888 | 0.24545455693342+0.491684664852536*RPS11P5 |

Tables 4-8 list the blood test results with the most predictive set of genes of based on the genes' mRNA level in whole blood samples, plasma samples, the combination of results from whole blood and plasma samples, all dried blood spot samples, and dried blood spot samples with RNA expression of a high number of genes detected (high-quality dried blood spot samples), respectively. Accordingly, some implementations of the disclosure are directed to kits comprising reagents to measuring the RNA expression of the specific sets of genes listings in Tables 1-8 in whole blood samples, plasma samples, the combination of results from whole blood and plasma samples, any dried blood spot samples, or high-quality dried blood spot samples. Other implementations of the disclosure are directed to methods of using the mRNA level of genes in the specific combinations listed in Tables 4-9 to predict corresponding blood test results. The formulas shown in Tables 1-9 transform the mRNA level into the typically presented blood test results.

In some implementations, the method comprises determining the subject's blood test result is in the normal range based on the RNA expression count of a gene, which may be determined from the conversion formula. Accordingly, the methods comprise quantifying the RNA expression of a set of genes, for example, the set of genes described listed Tables 1-8 for each combination of blood test and sample type, in the whole blood, plasma, or dried blood spot sample from a subject; and determining the subject has normal results for the corresponding blood test based on the RNA expression count of the set of genes.

For example, the subject is determined to have a normal percentage of segmented neutrophils if the subject's whole blood has gene counts of between 508 and 574 for RNF24, between 21829 and 22878 for MNDA, and between 9031 and 10757 for WIPF1. In another example, the subject is determined to have a normal percentage of lymphocytes if the subject's whole blood has gene counts of between 4345 and 4583 for GRB2, between 17569 and 19699 for MNDA, and between 3862 and 4492 for NFAM1. In still another example, the subject is determined to have a normal percentage of monocytes if the subject's whole blood has gene counts of between 1311 and 1642 for NAGA, between 629 and 828 for RIN2, between 2773 and 3436 for ADA2, between 3220 and 4087 for PLXNB2, and between 3907 and 5210 for ANXA2. Also from the whole blood sample, a subject may be determined to have a normal level of cholesterol if the subject's whole blood has gene counts of between 13 and 20 for RP5-1139B12.2, between 466 and 794 for GOLGA8A, between 83 and 99 for ENSG00000233280, and between 1186 and 1445 for SMC5. A subject may also be determined to have normal concentration of Aspartate Aminotransferase if the gene count in the whole blood sample for NEFM is between 9 and 52, for THUMPD1 is between 438 and 584, for LDLR is between 570 and 630, for CRTAM is between 66 and 97, and for CHCHD1 is between 35 and 37. Accordingly, if the gene counts for the set of the genes are not within the aforementioned range, the subject may be determined to have abnormal percentage of segmented neutrophils, lymphocytes, or monocytes, abnormal level of cholesterol, or abnormal concentration of Aspartate Aminotransferase.

**Table 4. Predictive combination of genes for a blood test result based on the genes' expression in whole blood samples according to multiple regression analysis**

| **Blood Test Result** | **R² value** | **Combination of Genes** | **Conversion Formula** |
|---|---|---|---|
| Lymphocytes_. | 0.87 | GRB2 \| MNDA \| NFAM1 \| | 61.368 + GRB2*-14.435 + MNDA*-8.518 + NFAM1*-10.035 |
| Monocytes_. | 0.79 | NAGA \| RIN2 \| ADA2 \| PLXNB2 \| ANXA2 \| | -1.485 + NAGA*2.640 + RIN2*3.203 + ADA2*4.201 + PLXNB2*-2.979 + ANXA2* 1.699 |
| Segmented.Neutrophils_. | 0.77 | RNF24 \| MNDA \| WIPF1 \| | 28.186 + RNF24*6.599 + MNDA*10.596 + WIPF1*16.517 |
| Apartate.Aminotransferase_IU.L | 0.74 | NEFM \| THUMPD 1 \| LDLR \| CRTAM \| CHCHD1 \| | 29.107 + NEFM*2.117 + THUMPD1*8.922 + LDLR*-6.926 + CRTAM*-4.987 + CHCHD1*-8.460 |
| Cholesterol_ | 0.72 | RP5-1139B12.2 \| GOLGA8A \| ENSG00000233280 \| SMC5 \| | -10.889 + RP5-1139B12.2*29.644 + GOLGA8A*51.591 + ENSG00000233280*71.333 + SMC5*71.353 |
| Eosinophils_. | 0.72 | PRSS33 \| CYSLTR2 \| FBN1 \| | -0.130 + PRSS33*0.793 + CYSLTR2* 1.035 + FBN1*0.816 |
| VLDL.Cholesterol_ | 0.69 | MAP3K15 \| SPDYE5 \| KL \| CDK15 \| | 17.169 + MAP3K15*6.338 + SPDYE5*3.931 + KL*-5.687 + CDK15*3.963 |
| Triglyceride_ | 0.69 | MAP3K15 \| SPDYE5 \| KL \| CDK15 \| | 86.793 + MAP3K15*31.417 + SPDYE5*19.132 + KL*-28.689 + CDK15*19.605 |
| LDL.Cholesterol..Calculated_ | 0.68 | ENSG00000233280 \| GOLGA8A \| HGSNAT \| PTMAP5 \| | -79.067 + ENSG00000233280*60.257 + GOLGA8A*66.896 + HGSNAT*64.237 + PTMAP5 * 14.510 |
| WBC_K.mm3 | 0.68 | GYPE \| SAP30BP \| MINPP1 \| IGHV2-5 \| | 3.618 + GYPE*-0.635 + SAP30BP*4.597 + MINPP1*-1.280 + IGHV2-5*0.654 |
| Absolute.Neutrophil_k.uL | 0.68 | SRPK1 \| ZFP36L1 \| DHRS12 \| | 0.599 + SRPK1*1.290 + ZFP36L1*1.508 + DHRS12*1.049 |
| TSH..High.Sensitivity_mU.L | 0.67 | ZNF100 \| SNHG8 \| TMCO6 \| MYO15B \| | 2.512 + ZNF100*0.672 + SNHG8*-0.852 + TMCO6*-1.367 + MYO15B*0.775 |
| Anion.Gap_mmol.L | 0.66 | PGLS \| CPSF7 \| CXorf65 \| COX18 \| | 17.595 + PGLS*-6.162 + CPSF7*3.144 + CXorf65*0.882 + COX18*-2.475 |
| Immature. Granulocyte_. | 0.66 | RPSAP46 \| NUP155 \| PCYT2 \| ERCC3 \| NFE2L1 \| | -1.755 + RPSAP46*0.235 + NUP155* 1.306 + PCYT2*0.832 + ERCC3*0.971 + NFE2L1 *-1.244 |
| Alkaline.Phosphatase_IU.L | 0.64 | SH3YL1 \| NAA38 \| SYNM \| FLJ21408 \| YBEY \| | 51.866 + SH3YL1*-18.148 + NAA38* 15.025 + SYNM*8.467 + FLJ21408*4.472 + YBEY*5.892 |
| Chloride_mmol.L | 0.63 | GNPDA1 \| NBR2 \| HUS1 \| IGHJ3 \| SPA17 \| | 104.120 + GNPDA1*-1.814 + NBR2*1.105 + HUS1*-1.884 + IGHJ3*0.496 + SPA17*-0.735 |
| MCH_pg | 0.63 | SMIM5 \| IL1RAP \| C10orf128 \| PLBI \| | 33.051 + SMIM5*-1.319 + IL1RAP*-1.036 + C10orf128*0.970 + PLB1*-1.183 |
| CO2_mmol.L | 0.62 | FAM157A \| NFKB2 \| IDI1 \| BTBD19 \| | 32.847 + FAM157A*-1.026 + NFKB2*-2.175 + IDI1*-2.866 + BTBD19*-1.263 |
| Calcium_mg.dL | 0.61 | MIOS \| SREBF1 \| NAA20 \| ITSN1 \| | 7.973 + MIOS* 1.222 + SREBF1*-0.414 + NAA20*0.407 + ITSN1*0.325 |
| T3.Uptake_. | 0.61 | ZNF469 \| ING2 \| RP11-22B23.1 \| EXTL2 \| XYLB \| | 29.359 + ZNF469*3.708 + ING2*-3.657 + RP11-22B23.1*-1.311 + EXTL2*0.914 + XYLB*-1.030 |
| T7.Index_ | 0.61 | IGHV3-33 \| ZNF266 \| CCDC183-AS1 \| GALK1 \| | 3.030 + IGHV3-33*-0.153 + ZNF266*-0.781 + CCDC183-AS1*0.272 + GALK1*-0.382 |
| Protein..Total_g.dL | 0.6 | ITM2A \| CDK2 \| SNORA80A \| DLG3 \| | 6.270 + ITM2A*0.435 + CDK2*0.469 + SNORA80A*-0.278 + DLG3*0.297 |
| Phosphorus..inorganic._mg.dL | 0.6 | IL18RAP \| SMPD2 \| KANSL2 \| CLCN1 \| SNORA20 \| | 3.756 + IL18RAP*-0.273 + SMPD2*-0.606 + KANSL2*0.688 + CLCN1*-0.188 + SNORA20*-0.177 |
| GGT_IU.L | 0.6 | SERPINE1 \| OTUD3 \| SORBS2 \| TMEM189 \| TFF3 \| | -9.959 + SERPINE1*4.791 + OTUD3*14.382 + SORBS2*3.572 + TMEM189*5.399 + TFF3*3.206 |
| MCV_fl | 0.59 | TMEM183A \| DTX3 \| RPL36AL \| COCH \| | 89.752 + TMEM183A*-8.108 + DTX3*4.893 + RPL36AL*6.015 + COCH*-1.233 |
| Non.HDL.Cholesterol_ | 0.59 | HGSNAT \| ENSG00000233280 \| PKDIP5 \| SMC5 \| | -102.769 + HGSNAT*93.920 + ENSG00000233280*46.891 + PKD1P5* 17.329 + SMC5*96.950 |
| Sodium_mmol.L | 0.59 | BTRC \| AMD1P3 \| WASHC2C \| ZNF575 \| RP11-156P1.3 \| | 137.476 + BTRC*2.416 + AMD1P3*0.793 + WASHC2C*-1.945 + ZNF575*0.861 + RP11-156P1.3*0.777 |
| Globulin_g.dL | 0.59 | MYH3 \| IL18BP \| ENSG00000196533 \| FASLG \| | 3.455 + MYH3*-0.292 + IL18BP*-0.619 + ENSG00000196533*-0.178 + FASLG*0.266 |
| Absolute.Lymphocyte_k.uL | 0.59 | OAZ2 \| KCNE3 \| RRP1B \| | 2.699 + OAZ2*-0.986 + KCNE3*-0.580 + RRP1B*0.778 |
| Platelet.Count_k.mm3 | 0.59 | SLC37A2 \| ERG \| IGLV3-12 \| RASSF8 \| | 295.099 + SLC37A2*-62.377 + ERG*13.913 + IGLV3-12* 15.408 + RASSF8* 18.805 |
| MPV_fl | 0.59 | TMCO3 \| GKAP1 \| LRRN1 \| SEPT7P8 \| | 11.973 + TMCO3*-1.361 + GKAP1*0.850 + LRRN1*-0.438 + SEPT7P8*-0.194 |
| T3.Total_ng.dL | 0.58 | MTPAP \| EBPL \| NRROS \| PMS2P5 \| KIF17 \| | 113.871 + MTPAP*-23.347 + EBPL*16.072 + NRROS*-15.683 + PMS2P5*18.738 + KIF17*8.796 |
| BUN_mg.dL | 0.58 | RFX2 \| HIST2H2BA \| ALG1L10P \| CDK16 \| MEIS2 \| | 19.980 + RFX2*-2.309 + HIST2H2BA*1.226 + ALG1L10P*0.784 + CDK16*-7.209 + MEIS2*0.785 |
| Cholesterol.HDL.Ratio_ | 0.57 | NCBP2L \| ENSG00000157828 \| BRWD1 \| NOS3 \| | 0.238 + NCBP2L*0.458 + ENSG00000157828*0.205 + BRWD1*3.580 + NOS3*-0.658 |
| RDW..sd._fl | 0.57 | PLEKHA5 \| DNAL1 \| ENSG00000197262 \| HNRNPCP2 \| IGHV1-69 \| | 40.743 + PLEKHA5* 1.542 + DNAL1*2.069 + ENSG00000197262*-0.857 + HNRNPCP2*-2.781 + IGHV1-69*1.463 |
| Thyroxine..T4._ug.dL | 0.56 | IQCE \| PNLDC1 \| RP1-34B20.4 \| GTF2H2B \| RBM3 \| | 6.393 + IQCE*1.833 + PNLDC1 *0.717 + RP1-34B20.4*0.793 + GTF2H2B*-0.514 + RBM3*-1.689 |
| BUN.Creatine.Ratio_ | 0.55 | HIST2H2BA \| ENSG00000235999 \| USF2 \| LOC652276 \| | 17.177 + HIST2H2BA* 1.583 + ENSG00000235999*2.443 + USF2*-7.711 + LOC652276*2.267 |
| Albumin..Globulin.Ratio_ | 0.55 | IL18BP \| SYCE1 \| SNORA80A \| CCZ1 \| | 0.842 + IL18BP*0.386 + SYCE1*0.069 + SNORA80A*0.192 + CCZ1*0.257 |
| Absolute.Monocyte_k.uL | 0.55 | NAGA \| ADA2 \| | -0.012 + NAGA*0.322 + ADA2*0.202 |
| Bilirubin..Total_mg.dL | 0.55 | CH13L2 \| ATXN7L1 \| INTS4P1 \| ZNF853 \| EGFL7 \| | 0.742 + CHI3L2*0.091 + ATXN7L1*-0.359 + INTS4P1*0.056 + ZNF853*-0.139 + EGFL7*0.090 |
| Uric.Acid_mg.dL | 0.54 | PARVB \| ST7 \| | 2.013 + PARVB*1.483 + ST7*1.231 |
| RDW..cv._. | 0.54 | PLEKHH2 \| NMT2 \| HNRNPLP2 \| | 11.820 + PLEKHH2*0.422 + NMT2*0.964 + HNRNPLP2*-0.516 |
| MCHC_g.dL | 0.54 | DTX4 \| SCOC \| PCMTDL1 \| | 29.803 + DTX4*1.328 + SCOC*1.293 + PCMTD1*1.242 |
| HDL.Cholesterol_ | 0.54 | SCARB1 \| FLYWCH1 \| NDUFS6 \| RPSAP14 \| ZNF442 \| | 67.537 + SCARB1*-10.340 + FLYWCH1*7.970 + NDUFS6*-17.096 + RPSAP14*2.312 + ZNF442*5.841 |
| Potassium_mmol.L | 0.53 | LRRC28 \| RP11-167N42 \| CLEC11A \| | 3.919 + LRRC28*0.614 + RP11-167N4.2*-0.270 + CLEC11A*0.165 |
| Albumin_g.dL | 0.5 | KANSL3 \| FNBP4 \| PGM1 \| | 3.347 + KANSL3*0.837 + FNBP4*0.724 + PGM1*-0.376 |
| Glucose _mg.dL | 0.49 | HMGB1P1 \| EXT2 \| SCAMP5 \| GUSBP3 \| | 62.755 + HMGB1P1 *6.262 + EXT2*23.950 + SCAMP5*-4.129 + GUSBP3*4.837 |
| Absolute.Basophil_k.uL | 0.49 | GATA2 \| SLC45A3 \| | -0.047 + GATA2*0.084 + SLC45A3*0.024 |
| Absolute.Eosinophil_k.uL | 0.49 | PRSS41 \| CLC \| ACOT11 \| | 0.051 + PRSS41*0.031 + CLC*0.031 + ACOT11*0.052 |
| Creatinine_mg.dL | 0.48 | USP9Y \| | 0.789 + USP9Y*0.122 |
| Lactic. Dehydrogenase_IU.L | 0.47 | PITPNM3 \| RAB31 \| ZNF138 \| CLEC9A \| | 173.513 + PITPNM3*7.515 + RAB31*-35.371 + ZNF138* 19.571 + CLEC9A*-8.565 |
| RBC_m.mm3 | 0.47 | DDX3Y \| | 4.476 + DDX3Y*0.343 |
| Hematocrit_. | 0.46 | NFYA \| PRKY \| | 51.088 + NFYA*-9.748 + PRKY* 1.422 |
| Alaine.Aminotransferase_IU.L | 0.46 | RNASE3 \| DEFA4 \| | 14.551 + RNASE3*5.042 + DEFA4*4.534 |
| Osmolality..Calculated,_mOsm.kg | 0.39 | EIF1AY \| | 284.603 + EIF1AY*2.352 |
| Hemoglobin_g.dL | 0.37 | USP9Y \| | 13.649 + USP9Y*0.878 |

**Table 5. Predictive combination of genes for a blood test result based on the genes' expression in plasma samples according to multiple regression analysis**

| **Blood Test Result** | **R² value** | **Combination of Genes** | **Conversion Formula** |
|---|---|---|---|
| Absolute.Eosinophil_k.uL | 0.65 | CLC \| ADAT1 \| SNRPEP4 \| GPC6 \| | 0.003 + CLC*0.058 + ADAT1*0.111 + SNRPEP4*-0.027 + GPC6*0.017 |
| Anion.Gap_mmol.L | 0.65 | DHX40 \| SLC1A4 \| IMPA2 \| KATNA1 \| MEIS3P1 \| | 8.466 + DHX40*2.429 + SLC1A4*-1.006 + IMPA2*1.263 + KATNA1*1.667 + MEIS3P1*0.506 |
| Lymphocytes_. | 0.6 | LAMB1 \| IRF6 \| RXFP1 \| FPGT \| CLECL1 \| | 25.096 + LAMB1*-1.424 + IRF6*-2.601 + RXFP1*-1.102 + FPGT*4.816 + CLECL1*3.539 |
| CO2_mmol.L | 0.6 | C8orf58 \| CFL2 \| EPHX2 \| AHDC1 \| | 25.672 + C8orf58*-1.275 + CFL2*-1.462 + EPHX2*1.095 + AHDC1*1.706 |
| Absolute.Basophil_k.uL | 0.58 | RRM1 \| SLC7A8 \| CCSER2 \| | 0.189 + RRM1*-0.097 + SLC7A8*0.031 + CCSER2*-0.070 |
| MCV_fl | 0.57 | CLCNKB \| OTUD4P1 \| PIKFYVE \| MFN2 \| | 96.222 + CLCNKB*-1.520 + OTUD4P1*1.489 + PIKFYVE*-6.486 + MFN2* 1.711 |
| RDW..cv._. | 0.57 | SKIL \| RAMP3) \| KDM8 \| SOCS4 \| | 13.499 + SKIL*-1.011 + RAMP3*0.205 + KDM8*0.663 + SOCS4*-0.688 |
| BUN_mg.dL | 0.57 | C5orf66 \| BLOC1S5 \| MRPL54 \| | 12.909 + C5orf66*1.426 + BLOC1S5*1.948 + MRPL54*-2.900 |
| MCHC_g.dL | 0.56 | XRCC5 \| RAD50 \| SPRTN \| | 29.328 + XRCC5*2.587 + RAD50*0.909 + SPRTN*0.805 |
| T7.Index_ | 0.55 | AXDND 1 \| ENSG00000232745 \| FAM117A \| C9orf172 \| | 1.737 + AXDND1*-0.119 + ENSG00000232745*-0.171 + FAM117A*0.409 + C9orf172*0.113 |
| Segmented.Neutrophils_. | 0.53 | RXFP1 \| POLR3GL \| FOXK2 \| LAMB1 \| | 58.500 + RXFP1*2.118 + POLR3GL*-5.441 + FOXK2*5.098 + LAMB1*2.226 |
| Cholesterol.HDL.Ratio_ | 0.52 | DES \| TUG1 \| KIAA1217 \| MFSD9 \| | 2.370 + DES*-0.389 + TUG1*0.348 + KIAA1217*0.422 + MFSD9*0.968 |
| Uric. Acid_mg.dL | 0.52 | ZFY \| C9orf78 \| CDH26 \| | 2.179 + ZFY*0.432 + C9orf78* 1.823 + CDH26*0.476 |
| Osmolality.. Calculated mOsm.kg | 0.52 | ZFY \| SLC15A2 \| | 289.077 + ZFY* 1.786 + SLC15A2*-4.292 |
| BUN.Creatine.Ratio_ | 0.51 | CHN1 \| ENSG00000205021 \| KDM1B \| C5orf66 \| | 7.190 + CHN1 * 1.694 + ENSG00000205021*1.256 + KDM1B*4.564 + C5orf66*1.421 |
| Globulin_g.dL | 0.51 | HNRNPLP2 \| HLA-G \| SETP14 \| | 2.218 + HNRNPLP2*0.177 + HLA-G*0.242 + SETP14*0.142 |
| Alkaline.Phosphatase_IU.L | 0.51 | LNX2 \| CH17-12M21.1 \| TTC26 \| | 30.327 + LNX2*25.914 + CH17-12M21.1*4.929 + TTC26*7.892 |
| Lactic.Dehydrogenase_IU.L | 0.51 | AFF2 \| MERTK \| AXDND1 \| RP11-603B24.1 \| | 149.778 + AFF2*17.419 + MERTK*-11.816 + AXDND1*7.350 + RP11-603B24.1*-4.700 |
| Absolute.Neutrophil_k.uL | 0.5 | MYO1A \| ENSG00000140181 \| ROBO1 \| | 5.258 + MYO1A*-0.566 + ENSG00000140181*-0.969 + ROBO1*0.348 |
| T3.Uptake_. | 0.5 | AP3S2 \| RPL23AP52 \| PSMC3IP \| SAXO2 \| | 29.044 + AP3S2*-1.168 + RPL23AP52*-1.337 + PSMC3IP* 1.603 + SAXO2*-0.715 |
| Alaine.Aminotransferase_IU.L | 0.5 | MMP8 \| CRISP3 \| RPL39L \| | 12.355 + MMP8*5.526 + CRISP3*3.331 + RPL39L*3.528 |
| Bilirubin..Total mg.dL | 0.5 | TGM2 \| NEK6 \| XCL2 \| BZW2 \| | 0.547 + TGM2*0.096 + NEK6*-0.171 + XCL2*-0.104 + BZW2*0.087 |
| Thyroxine..T4._ug.dL | 0.49 | CDCA8 \| RPS12P23 \| GTSE1 \| N4BP3 \| | 7.359 + CDCA8*-0.532 + RPS12P23*0.582 + GTSE1*-0.552 + N4BP3*0.563 |
| Triglyceride_ | 0.49 | RP11-516A11.1 \| CHSY1 \| ZNF816 \| | 56.067 + RP11-516A11.1*-27.741 + CHSY1*57.871 + ZNF816*30.405 |
| VLDL. Chole sterol_ | 0.49 | RP11-516A11.1 \| CHSY1 \| ZNF816 \| | 11.166 + RP11-516A11.1*-5.595 + CHSY1*11.697 + ZNF816*6.044 |
| Sodium_mmol.L | 0.49 | MRRF \| PDCD6IP \| SMN2 \| ERRFI1 \| | 144.248 + MRRF*-1.755 + PDCD61P*-1.584 + SMN2*-0.501 + ERRFI1*-0.598 |
| Albumin_g.dL | 0.49 | SNF8 \| TWF2 \| PAQR4 \| FAM26F \| | 3.950 + SNF8*0.254 + TWF2*0.118 + PAQR4*0.122 + FAM26F*0.154 |
| Albumin..Globulin.Ratio_ | 0.49 | SEPT10 \| HLA-G \| GPR146 \| HNRNPLP2 \| | 1.591 + SEPT10*0.165 + HLA-G*-0.071 + GPR146*0.109 + HNRNPLP2*-0.084 |
| RBC_m.mm3 | 0.48 | UTY \| | 4.485 + UTY*0.344 |
| Platelet. Count k.mm3 | 0.48 | JADE3 \| ZMIZ1 \| VNN 1 \| ENSG00000214982 \| | 328.471 + JADE3* 16.865 + ZMIZ1*-50.872 + VNN1* 16.123 + ENSG00000214982*-37.657 |
| MPV_fl | 0.48 | CBRI \| CHIC2 \| FANK1 \| BAIAP2 \| | 10.962 + CBR1*0.549 + CHIC2*-1.242 + FANK1*0.300 + BAIAP2*0.475 |
| TSH..High.Sensitivity_mU.L | 0.47 | SLC26A8 \| ITGB8 \| WNK4 \| | 0.947 + SLC26A8*0.530 + ITGB8*0.292 + WNK4*0.229 |
| Creatinine_mg.dL | 0.47 | ZFY \| | 0.793 + ZFY*0.120 |
| Calcium_mg.dL | 0.47 | IGLV3-19 \| HSPA1B \| JCHAIN \| | 10.138 + IGLV3-19*-0.153 + HSPA1B*-0.458 + JCHAIN*-0.118 |
| Potassium_mmol.L | 0.46 | SYK \| BAK1 \| SCIN \| ANO5 \| | 4.995 + SYK*-0.265 + BAK1*-0.206 + SCIN*-0.113 + ANO5*-0.103 |
| RDW..sd._fl | 0.45 | EOGT \| ABHD 13 \| NUDCD 1 \| | 42.919 + EOGT*2.466 + ABHD13*-1.634 + NUDCD1*-1.817 |
| Cholesterol_ | 0.45 | AURKB \| RNF103 \| C3orf79 \| | 169.209 + AURKB*-22.224 + RNF103*43.571 + C3orf79* 17.506 |
| Protein.Total_g.dL | 0.45 | RP11-516A11.1 \| SLC4A11 \| UBR7 \| BFSP2\| | 6.619 + RP11-516A11.1*0.136 + SLC4A11*0.102 + UBR7*0.334 + BFSP2*0.099 |
| Absolute. Lymphocyte_k.uL | 0.44 | AC138623.1 \| DPPA4 \| ZNF688 \| | 1.450 + AC138623.1*0.177 + DPPA4*0.241 + ZNF688*0.221 |
| Absolute.Monocyte k.uL | 0.44 | TMED7 \| ADSSL1 \| PSMB6 \| RP11-832N8.1 \| | 0.422 + TMED7*-0.067 + ADSSL1*0.045 + PSMB6*0.136 + RP11-832N8.1*-0.054 |
| LDL.Cholesterol..Calculated_ | 0.43 | ENG \| MERTK \| PSMD9 \| NFIC \| | 214.896 + ENG*-21.550 + MERTK*-26.626 + PSMD9*-14.801 + NFIC*-42.993 |
| Eosinophils_. | 0.42 | SUPT3H \| ZNF662 \| ZSCAN30 \| | -0.566 + SUPT3H*1.584 + ZNF662*0.474 + ZSCAN30*0.965 |
| Non.HDL.Cholesterol_ | 0.41 | SBDSP1 \| RNF103 \| DES \| | 80.611 + SBDSP1*36.140 + RNF103*39.399 + DES*-11.264 |
| Glucose_mg.dL | 0.41 | ENSG00000138297 \| JAG2 \| DNAJB4 \| | 75.380 + ENSG00000138297* 12.952 + JAG2*-3.585 + DNAJB4*7.969 |
| Hematocrit_. | 0.4 | UTY \| C9orf40 \| | 39.838 + UTY*1.450 + C9orf40*2.060 |
| Phosphorus. inorganic._mg.dL | 0.4 | CNRIP1 \| NSL1 \| MUC4 \| | 3.928 + CNRIP1*-0.181 +NSL1*-0.488 + MUC4*-0.128 |
| GGT_IU.L | 0.4 | CD84 \| SCIN \| UCP2 \| | 10.005 + CD84*4.097 + SCIN*3.028 + UCP2*4.275 |
| WBC_K.mm3 | 0.39 | HAVCR2 \| SLC24A1 \| | 8.561 + HAVCR2*-0.990 + SLC24A1*-0.934 |
| Monocytes_. | 0.38 | CADM2 \| MTCL1 \| SAMD10 \| | 7.869 + CADM2*-0.751 + MTCL1*-0.837 + SAMD10*0.597 |
| T3.Total_ng.dL | 0.38 | OPA1 \| FDX1\| TRDV1 \| | 70.678 + OPA1*28.331 + FDX1* 14.297 + TRDV1*5.626 |
| MCH_pg | 0.37 | SH2B2 \| MIPEP \| TPMT \| | 30.413 + SH2B2*-0.591 + MIPEP*-0.528 + TPMT* 1.203 |
| Immature. Granulocyte_. | 0.37 | CDCA7L \| TMEM99 \| FUT10 \| | -0.454 + CDCA7L*0.384 + TMEM99*0.229 + FUT10*0.213 |
| HDL.Cholesterol_ | 0.37 | SBNO1 \| ACTR8 \| ZFY \| | 87.733 + SBNO1*-20.008 + ACTR8*-10.976 + ZFY*-3.830 |
| Hemoglobin_g.dL | 0.33 | ANOS2P \| ZFY \| | 13.685 + ANOS2P*0.458 + ZFY*0.428 |
| Chloride_mmol.L | 0.18 | HBEGF \| IL1RAPL1 \| | 101.368 + HBEGF*-0.524 + IL1RAPL1*0.585 |
| Apartate.Aminotransferase_IU.L | 0.11 | HHEX \| | 14.976 + HHEX*5.171 |

**Table 6. Predictive combination of genes for a blood test result based on the genes' expression in either whole blood or plasma samples according to multiple regression analysis**

| **Blood Test Result** | **R² value** | **Combination of Genes and Source of Expression Information** | **Conversion Formula** |
|---|---|---|---|
| RDW..sd._fl | 0.68 | CHCHD2P6 (Plasma) \| SEC63P1 (Plasma) \| DNAL1 (Blood) \| ENSG00000197262 (Blood) \| | 37.446 + CHCHD2P6*1.489 + SEC63P1*1.463 + DNAL1*3.237 + ENSG00000197262*1.214 |
| T7.Index_ | 0.65 | IGHV3-33 (Blood) \| ZNF266 (Blood) \| CCDC183-AS1 (Blood) \| ENSG00000232745 (Plasma) \| | 2.706 + IGHV3-33*-0.152 + ZNF266*-0.762 + CCDC183-AS1*0.296 + ENSG00000232745*-0.125 |
| MCHC_g.dL | 0.62 | DTX4 (Blood) \| LRIF1 (Plasma) \| SCOC (Blood) \| PCMTD1 (Blood) \| | 29.423 + DTX4*0.884 + LRIF1*0.755 + SCOC*1.191 + PCMTD1*1.435 |
| Thyroxine..T4._ug.dL | 0.57 | CDCA8 (Plasma) \| IQCE (Blood) \| PNLDC1 (Blood) \| RP1-34B20.4 (Blood) \| | 4.553 + CDCA8*-0.693 + IQCE*2.069 + PNLDC1*0.730 + RP1-34B20.4*0.943 |

**Table 7. Predictive combination of genes for a blood test result based on the genes' expression in all dried blood spot samples according to multiple regression analysis**

| **Blood Test Result** | **R² value** | **Combination of Genes** | **Conversion Formula** |
|---|---|---|---|
| Alaine.Aminotransferase_IU.L | 0.66 | EIF1AY \| SRXN1 \| NDUFAF2 \| TBCE \| | 19.003 + EIF1AY*3.710 + SRXN1*2.987 + NDUFAF2*4.296 + TBCE*-5.521 |
| Eosinophils_. | 0.63 | SCARNA22 \| TET3 \| | 0.850 + SCARNA22*0.942 + TET3*0.868 |
| Absolute.Neutrophil_k.uL | 0.58 | MMP25 \| KAT2B \| DOK3 \| | 3.574 + MMP25*0.681 + KAT2B*-0.558 + DOK3*0.610 |
| RBC_m.mm3 | 0.58 | EIF1AY \| DDX3Y \| BCL2L13 \| | 4.387 + EIFIAY*0.215 + DDX3Y*0.147 + BCL2L13*0.174 |
| Platelet. Count k.mm3 | 0.55 | SNORA19 \| SNCA \| FCHO2 \| ARHGAP10 \| | 280.688 + SNORA19*12.416 + SNCA*-19.917 + FCHO2*-21.947 + ARHGAP10*19.487 |
| RDW..sd._fl | 0.55 | JAM3 \| PEX10 \| N4BP2L2 \| NCAPG \| | 43.901 + JAM3*0.830 + PEX10*1.106 + N4BP2L2*-2.563 + NCAPG*-0.876 |
| Anion.Gap_mmol.L | 0.54 | FOLR3 \| SNORD116-15 \| TIMELESS \| ANKRD54 \| | 13.470 + FOLR3*-0.512 + SNORD116-15*-0.446 + TIMELESS*0.816 + ANKRD54*-0.661 |
| CO2_mmol.L | 0.54 | PPP1R12C \| TPT1 \| IK \| | 26.246 + PPP1R12C*-1.447 + TPT1* 1.727 + IK*-0.807 |
| HDL.Cholesterol_ | 0.53 | GNAQ \| SCARNA9 \| DDX3Y \| INTS13 \| | 49.432 + GNAQ*3.596 + SCARNA9*3.748 + DDX3Y*-3.237 + INTS13*4.036 |
| Osmolality..Calculated_mOsm.kg | 0.52 | EIF1AY \| CCNF \| DDX41 \| TRAPPC8 \| | 286.876 + EIF1AY*1.130 + CCNF*1.099 + DDX41*-1.006 + TRAPPC8*-1.648 |
| Creatinine_mg.dL | 0.51 | EIF1AY \| PRKY \| XIST \| RPS4Y1 \| | 0.835 + EIF1AY*0.046 + PRKY*0.042 + XIST*-0.042 + RPS4Y1*0.034 |
| Hematocrit_. | 0.51 | DDX3Y \| TTC8 \| UTY \| SPDL1 \| | 41.765 + DDX3Y*1.047 + TTC8*-1.199 + UTY*0.827 + SPDL1* 1.051 |
| Lymphocytes_. | 0.5 | RPL23A \| LPIN1 \| MRPS11 \| RGS6 \| | 25.554 + RPL23A*4.075 + LPIN1*2.745 + MRPS11*3.109 + RGS6*-5.810 |
| Absolute.Eosinophil_k.uL | 0.5 | CCT3 \| C6orf120 \| RHOG \| | 0.211 + CCT3*-0.042 + C6orf120*-0.032 + RHOG*-0.022 |
| MCV_fl | 0.49 | ISPD \| MARK3 \| CHD3 \| STRN3 \| | 92.995 + ISPD*-1.413 + MARK3*-1.332 + CHD3*2.159 + STRN3*-1.626 |
| Absolute.Lymphocyte_k.uL | 0.49 | CAMP \| ENDOD1 \| NEDD4L \| ALS2CR12 \| | 2.180 + CAMP*0.123 + ENDOD1*0.154 + NEDD4L*-0.259 + ALS2CR12*-0.230 |
| TSH..High.Sensitivity_mU.L | 0.49 | KIF21A \| MIA3 \| | 1.216 + KIF21A*0.402 + MIA3*0.475 |
| LDL.Cholesterol..Calculated_ | 0.48 | SNORD116-26 \| PTMAP5 \| ECT2 \| IL31RA \| NAP1L2 \| | 98.596 + SNORD116-26*10.898 + PTMAP5*14.280 + ECT2*11.341 + IL31RA*-17.483 + NAP1L2*7.391 |
| Calcium_mg.dL | 0.46 | RPS11 \| TLK2P1 \| UBTD1 \| | 9.444 + RPS11*0.225 + TLK2P1*-0.122 + UBTD1*-0.171 |
| Segmented.Neutrophils_. | 0.45 | HMGB1P1 \| CSRNP1 \| CCNJL \| | 55.794 + HMGB1P1*2.817 + CSRNP1*2.822 + CCNJL*2.452 |
| RDW..cv._. | 0.44 | RGS10 \| N4BP2L2 \| MMD \| | 12.957 + RGS10*0.370 + N4BP2L2*-0.695 + MMD*0.139 |
| Sodium_mmol.L | 0.44 | PGBD2 \| PRPF18 \| TATDN3 \| KRT1 \| | 140.710 + PGBD2*-0.385 + PRPF18*0.595 + TATDN3*-0.333 + KRT1*-0.536 |
| WBC_K.mm3 | 0.43 | CDK8 \| EPB41 \| RAB11B \| | 8.013 + CDK8*-0.483 + EPB41*-0.709 + RAB11B*-0.318 |
| Bilirubin..Total_mg.dL | 0.43 | LRRC37A4P \| DNAJC2 \| PIK3CB \| PDP2 \| | 0.423 + LRRC37A4P*0.077 + DNAJC2*0.077 + PIK3CB*-0.055 + PDP2*-0.048 |
| T7.Index_ | 0.42 | UBBP4 \| LUC7L \| GIT2 \| COA5 \| | 1.950 + UBBP4*0.127 + LUC7L*0.103 + GIT2*-0.097 + COA5*-0.101 |
| Immature.Granulocyte_. | 0.42 | TRAF3IP1 \| NOC3L \| CFAP161 \| | -0.007 + TRAF3TP1 *0.173 + NOC3L*0.236 + CFAP161*0.112 |
| BUN_mg.dL | 0.42 | FSDIL \| C6orf48 \| ZC3H15 \| LRRK2 \| RP11-632K20.7 \| | 10.116 + FSD1L*1.476 + C6orf48*0.902 + ZC3H15*-1.686 + LRRK2*1.607 + RP11-632K20.7*1.534 |
| Albumin_g.dL | 0.42 | ORC1 \| BICDL2 \| PSMC3IP \| | 4.527 + ORC1*0.067 + BICDL2*-0.110 + PSMC3IP*0.076 |
| Non.HDL.Cholesterol_ | 0.41 | IL31RA \| PARL \| BLOC1S6 \| | 143.318 + IL31RA*-14.229 + PARL*-18.019 + BLOC1S6*24.533 |
| Hemoglobin_g.dL | 0.41 | DDX3Y \| IFNGR2 \| FBXW7 \| | 14.050 + DDX3Y* 1.140 + IFNGR2*0.577 + FBXW7*-0.947 |
| Absolute.Monocyte_k.uL | 0.4 | UBBP4 \| GGA1 \| KLF7 \| FARSA \| | 0.471 + UBBP4*-0.073 + GGA1*0.041 + KLF7*0.041 + FARSA*0.040 |
| Monocytes_. | 0.39 | CCDC115 \| RECQL4 \| SASS6 \| | 8.259 + CCDC115*-1.082 + RECQL4*-0.505 + SASS6*-0.432 |
| MCH_pg | 0.39 | MIR15A \| ClGALT1 \| SAMD9 \| SNCA \| | 30.097 + MIR15A*0.372 + C1GALT1*-0.498 + SAMD9*0.275 + SNCA*0.434 |
| Uric.Acid_mg.dL | 0.38 | EIF1AY \| IFNGR2 \| WHAMMP2 \| | 4.400 + EIF1AY*0.449 + IFNGR2*0.274 + WHAMMP2*-0.377 |
| Absolute.Basophil_k.uL | 0.38 | POLB \| A TRIP \| DIP2A \| | 0.091 + POLB*-0.018 + ATRIP*-0.023 + DIP2A*-0.016 |
| Phosphorus..inorganic._mg.dL | 0.38 | RECK \| HIKESHI \| CMC1 \| | 3.554 + RECK*-0.146 + HIKESHI*-0.125 + CMCl *-0.197 |
| Cholesterol.HDL.Ratio_ | 0.37 | GOLGA2 \| UTY \| ARIH1 \| | 4.056 + GOLGA2*-0.432 + UTY*0.447 + ARIH1*-0.532 |
| VLDL.Cholesterol_ | 0.37 | GOS2 \| ZHX3 \| | 24.116 + GOS2*4.296 + ZHX3*-4.787 |
| MPV_fl | 0.37 | IMPDH1 \| FCHO1 \| | 11.584 + IMPDH1*-0.514 + FCHO1*-0.359 |
| T3.Total_ng.dL | 0.37 | RP11-707023.5 \| UQCC1 \| BEX3 I | 113.352 + RP11-707023.5*7.613 + UQCC1*5.937 + BEX3*-9.050 |
| GGT_IU.L | 0.36 | EIF1AY \| SEPT2 \| MTMR3 \| | 20.448 + EIF1AY*4.166 + SEPT2*-3.769 + MTMR3*-2.967 |
| Potassium_mmol.L | 0.36 | STAG3 \| SREBF1 \| HSP90AA1 \| | 4.615 + STAG3*-0.128 + SREBF1*-0.105 + HSP90AA1*-0.103 |
| Globulin_g.dL | 0.36 | ABCG2 \| LSM2\| | 2.524 + ABCG2*0.157 + LSM2*0.129 |
| Lactic.Dehydrogenase_IU.L | 0.35 | NSUN6 \| ENSG00000211953 \| SMIM13 \| | 161.058 + NSUN6*-5.722 + ENSG00000211953*8.385 + SMIM13*-7.785 |
| Protein.Total_g.dL | 0.35 | VAMP4 \| TREML1 \| SHMT1 \| | 6.992 + VAMP4*0.166 + TREML1*0.098 + SHMT1*0.096 |
| Albumin..Globulin.Ratio_ | 0.33 | ABCG2 \| ACOT8 \| | 1.815 + ABCG2*-0.103 + ACOT8*-0.108 |
| MCHC_g.dL | 0.33 | DDX3Y \| AURKA \| | 33.101 + DDX3Y*0.361 + AURKA*0.390 |
| Glucose_mg.dL | 0.31 | AKIRIN1 \| ENSG00000196331 \| | 87.285 + AKIRIN1*4.455 + ENSG00000196331*4.462 |
| Alkaline.Phosphatase_IU.L | 0.31 | SCAF8 \| POLE4 \| | 58.928 + SCAF8*5.435 + POLE4*6.309 |
| T3.Uptake_. | 0.3 | SRF \| ZNF736 \| | 29.281 + SRF*-1.142 + ZNF736*-0.955 |
| Apartate.Aminotransferase_IU.L | 0.29 | RASSF4 \| MECP2 \| ACTR6 \| | 21.744 + RASSF4*11.652 + MECP2*-11.011 + ACTR6*-3.542 |
| BUN.Creatine.Ratio_ | 0.29 | MTMR11 \| ZNF865 \| | 12.749 + MTMR11* 1.415 + ZNF865* 1.554 |
| Chloride_mmol.L | 0.28 | SPC24 \| IL17RA \| | 100.088 + SPC24*0.557 + IL17RA* 1.141 |
| Cholesterol_ | 0.27 | BLOC1S6 \| ARPP19 \| | 181.520 + BLOC1S6*20.047 + ARPP19* 15.122 |
| Triglyceride_ | 0.25 | RN7SL5P \| G0S2 \| | 98.336 + RN7SL5P*18.347 + G0S2*13.272 |
| Thyroxine..T4._ug.dL | 0.23 | DIRC2 \| LDLR \| | 6.827 + DIRC2*0.526 +LDLR*0.589 |

**Table 8 Predictive combination of genes for a blood test result based on the genes' expression in high-quality dried blood spot samples according to multiple regression analysis**

| **Blood Test Result** | **R² value** | **Combination of Genes** | **Conversion Formula** |
|---|---|---|---|
| Non.HDL.Cholesterol_ | 0.84 | BMT2 \| PKD1P5 \| ARIH1 \| | 190.187 + BMT2*-39.633 + PKD1P5*24.799 + ARIH1*-36.288 |
| Eosinophils_. | 0.78 | NDUFA5 \| MCM8 \| | 1.637 + NDUFA5*0.652 + MCM8*-0.888 |
| RBC_m.mm3 | 0.76 | PRKY \| OARD1 \| | 5.018 + PRKY*0.306 + OARD1*-0.552 |
| Absolute.Neutrophil_k.uL | 0.76 | AC079140.2 \| RAP1GAP \| | 1.295 + AC079140.2*0.434 + RAP1GAP*0.191 |
| T7.Index_ | 0.74 | C7orf50 \| ARHGAP 10 \| | 169.734 + C7orf50*60.129 + ARHGAP10*45.009 |
| Lymphocytes_. | 0.73 | C7orf73 \| ATG16L2 \| | 3.708 + C7orf73*-1.104 + ATG16L2* 1.673 |
| Creatinine_mg.dL | 0.73 | RPS6KA5 \| HAL \| MYO6 \| | 2.205 + RPS6KA5*-0.307 + HAL*-0.158 + MYO6*0.314 |
| Absolute.Eosinophil_k.uL | 0.71 | SEPT7 \| DDX11L5 \| ODF2L \| | 137.493 + SEPT7*2.407 + DDX11L5*0.771 + ODF2L*-0.574 |
| Platelet.Count_k.mm3 | 0.71 | RPL4P5 \| RN7SL396P \| | 11.287 + RPL4P5*8.000 + RN7SL396P*2.881 |
| Cholesterol_ | 0.7 | CENPE \| PLEC \| NEK1 \| | 13.118 + CENPE*-0.541 + PLEC*0.542 + NEK1*-0.622 |
| CO2_mmol.L | 0.69 | UTRN \| CD247 \| FAM133B \| | 21.612 + UTRN*7.280 + CD247*3.664 + FAM133B*-2.998 |
| Globulin_g.dL | 0.69 | TOPORS \| CHD3 \| LCMT2 \| | 37.378 + TOPORS*-3.897 + CHD3*-4.481 + LCMT2*-1.476 |
| Albumin..Globulin.Ratio_ | 0.69 | BLOC1S2 \| PRPF18 \| PRKY \| | 281.269 + BLOC1S2*2.260 + PRPF18*1.743 + PRKY*1.671 |
| GGT_IU.L | 0.68 | GSK3A \| RHOBTB1 \| TMEM64 \| | 35.870 + GSK3A*2.889 + RHOBTB1*2.033 + TMEM64*2.648 |
| Osmolality..Calculated_mOsm.kg | 0.68 | EPSTI1 \| CIR1 \| PMS2P1 \| | 217.443 + EPSTI1*-62.074 + CIR1 *-43.229 + PMS2P1*-20.816 |
| RDW..sd._fl | 0.68 | EPSTI1 \| PMS2P1 \| CIR1 \| | 43.728 + EPSTI1*-12.414 + PMS2P1*-4.321 + CIR1*-8.806 |
| Absolute.Basophil_k.uL | 0.68 | SPDL1 \| XIST \| | 0.868 + SPDL1*0.125 + XIST*-0.133 |
| T3.Total_ng.dL | 0.67 | BEND2 \| METTL9 \| ARHGAP10 \| | -0.012 + BEND2*0.040 + METTL9*0.060 + ARHGAP10*0.045 |
| Anion.Gap_mmol.L | 0.67 | RAD18 \| MTFMT \| | 7.714 + RAD18*3.284 + MTFMT*2.233 |
| MPV_fl | 0.67 | SPIDR \| SCARNA8 \| MRPL1 \| | 0.700 + SPIDR*-0.225 + SCARNA8*0.075 + MRPL1*-0.122 |
| BUN.Creatine.Ratio_ | 0.66 | SENP6 \| PTPN9 \| | 31.454 + SENP6*1.849 + PTPN9*0.407 |
| Absolute.Monocyte_k.uL | 0.66 | BMT2 \| ZNF561 \| | 205.331 + BMT2*-45.826 + ZNF561*32.029 |
| WBC_K.mm3 | 0.66 | ISPD \| RHOBTB1 \| | 22.903 + ISPD*2.134 + RHOBTB1*1.206 |
| Segmented.Neutrophils_. | 0.65 | AKAP12 \| APP \| | 3.478 + AKAP12*1.431 + APP*2.342 |
| HDL.Cholesterol_ | 0.64 | C1GALT1 \| SSX2IP \| | 2.335 + C1GALT1*0.276 + SSX2IP*0.245 |
| Uric.Acid_mg.dL | 0.63 | ATG16L2 \| EPB41 \| | 5.763 + ATG16L2*2.012 + EPB41*-1.000 |
| Lactic.Dehydrogenase_IU.L | 0.63 | CASP8AP2 \| PIN1 \| | 1.676 + CASP8AP2*0.190 + PIN1*-0.177 |
| Cholesterol.HDL.Ratio_ | 0.63 | FBXO28 \| UNC13B \| PAFAH1B2 \| | 76.189+FBXO28*-7.825+UNC13B*9.059 + PAFAH1B2*-11.419 |
| Albumin_g.dL | 0.62 | KPNA5 \| MTCH2 \| SIRT5 \| | 18.205 + KPNA5*10.349 + MTCH2*-6.415 + SIRT5*-6.980 |
| LDL.Cholesterol..Calculated_ | 0.6 | BRIX1 \| BABAM1 \| GSK3A \| | 11.947 + BRIX1*0.980 + BABAM1*0.632 + GSK3A*0.885 |
| BUN_mg.dL | 0.59 | GNG11 \| NCOA2 \| | 44.944 + GNG11* 1.759 + NCOA2*-4.385 |
| Immature.Granulocyte_. | 0.59 | NUDT3 \| YEATS4 \| ANP32B \| | -0.026 + NUDT3*0.045 + YEATS4*-0.020 + ANP32B*0.041 |
| Phosphorus..inorganic._mg.dL | 0.59 | RMND1 \| TRAF4 \| | 98.038 + RMND1*14.283 + TRAF4*9.635 |
| Hematocrit_. | 0.59 | MARC1 \| SREK1IP1 \| PF4V1 \| | 13.017 + MARC1*-0.711 + SREK1IP1*1.181 + PF4V1*-0.778 |
| Potassium_mmol.L | 0.58 | AP001004.1 \| COX11 \| | 94.483 + AP001004.1*-1.813 + COX11*-2.265 |
| Calcium_mg.dL | 0.58 | PEX5 \| RPL26 \| | 10.234 + PEX5*-0.526 + RPL26* 1.150 |
| Absolute. Lymphocyte k.uL | 0.58 | ZNF155 \| PRDM8 \| | 10.759 + ZNF155*2.778 + PRDM8*2.815 |
| Protein.Total_g.dL | 0.57 | PLXNB2 \| APP \| APOL1 \| | 0.191 + PLXNB2*0.118 + APP*0.109 + APOL1*0.098 |
| Triglyceride_ | 0.56 | ST13 \| CCT3 \| | 3.280 + ST13*-0.932 + CCT3*-0.486 |
| VLDL.Cholesterol_ | 0.55 | C7orf73 \| SMAD4 \| | 71.124 + C7orf73*-5.658 + SMAD4*-5.247 |
| T3.Uptake_. | 0.54 | MAPK6 \| BMP6 \| | 83.258 + MAPK6*6.674 + BMP6*8.335 |
| Thyroxine..T4._ug.dL | 0.54 | MAP1LC3B \| HPF1 \| | 3.184 + MAP1LC3B* 1.204 + HPF1*0.472 |
| Alkaline.Phosphatase_IU.L | 0.54 | GAD1 \| PDP2 \| | 158.447 + GAD1*-16.450 + PDP2*12.011 |
| Sodium_mmol.L | 0.53 | UTY \| PKDIP5 \| | 2.954 + UTY*0.599 + PKD1P5*0.485 |
| Alaine.Aminotransferase_IU.L | 0.53 | QRICH2 \| SLC25A1 \| | 4.761 + QRICH2*-0.148 + SLC25A1*-0.160 |
| Bilirubin..Total_mg.dL | 0.52 | DLEU2 \| KIF14 \| | 29.102 + DLEU2*0.725 + KIF14*1.098 |
| MCHC_g.dL | 0.52 | SCARNA9 \| UTY \| | 54.066 + SCARNA9*6.618 + UTY*-5.407 |
| Hemoglobin_g.dL | 0.5 | DIP2C \| CCDC137 \| | 6.071 + DIP2C*0.899 + CCDC137*0.892 |
| MCV_fl | 0.5 | ARIH1 \| BMT2 \| | 179.700 + ARIH1*-45.553 + BMT2*-32.071 |
| MCH_pg | 0.48 | FAM228B \| LINC00969 \| | 0.228 + FAM228B*0.432 + LINC00969*-0.346 |
| TSH..High.Sensitivity_mU.L | 0.48 | RECK \| FAM76A \| | 3.635 + RECK*-0.315 + FAM76A*-0.271 |
| Apartate.Aminotransferase_IU.L | 0.47 | PVALB \| ABCB7 \| | 6.799 + PVALB*0.262 + ABCB7*0.244 |
| Monocytes_. | 0.44 | ENSG00000254184 \| TMEM18 \| | 4.780 + ENSG00000254184*-0.230 + TMEM18*-0.218 |
| Chloride_mmol.L | 0.44 | ABCB7 \| SPDL1 \| | 9.065 + ABCB7*0.319 + SPDL1*0.143 |
| RDW..cv._. | 0.41 | SLAIN1 \| CCDC115 \| | 10.961 + SLAIN1*4.311 + CCDC115*4.465 |
| Glucose_mg.dL | 0.17 | LOC100506302 \| | 101.975 + LOC100506302*-0.769 |

**Table 9 Predictive combination of genes for a blood test result based on the genes' expression in whole blood, plasma samples, or dried blood-spot samples according to multiple regression analysis**

| **Test** | **R² value** | **genes** | **forumla** |
|---|---|---|---|
| Lymphocytes_. | 0.84 | EVI2B (Blood) \| NFAM1 (Blood) \| | 56.7246715975982 +EVI2B (Blood) *-15.8825524318068 + NFAM1 (Blood) *-12.3596561059337 |
| Monocytes_. | 0.8 | RIN2 (Blood) \| ADA2 (Blood) \| | -0.158183662335998 +RIN2 (Blood) *3.93278986538093 + ADA2 (Blood) *3.5561768635177 |
| Seqmented.Neutrophils_. | 0.74 | RNF24 (Blood) \| MNDA (Blood) \| TLR1 (Blood) | 35.6931236896115 +RNF24 (Blood) *8.85994146801373 + MNDA (Blood) *9.76462219780331 + TLR1 (Blood) *7.46277245596464 |
| Eosinophils_. | 0.72 | SIGLEC8 (Blood) \| FBN1 (Blood) \| | 0.239650247438389 +SIGLEC8 (Blood) *1.07664315020184 + FBN1 (Blood) * 1.24348775920263 |
| Anion.Gap_mmol.L | 0.67 | PGLS (Blood) \| BTBD19 (Blood) \| LUC7L (Blood) | 13.497493355259 +PGLS (Blood) *-5.33321876906759 + BTBD19 (Blood) * 1.20890323544764 + LUC7L (Blood) *3.80083623295323 |
| LDL.Cholesterol..Calculated_ | 0.62 | ENSG00000233280 (Blood) \| GOLGA8A (Blood) \| SMC5 (Blood) | -88.1273832988103 +ENSG00000233280 (Blood) *76.7777518719049 + GOLGA8A (Blood) *72.0126373761645 + SMC5 (Blood) *64.9019008350544 |
| VLDL. Chole sterol_ | 0.6 | MAP3K15 (Blood) \| SPDYE5 (Blood) \| KL (Blood) | 18.0359463299043 +MAP3K15 (Blood) *8.01564614798221 + SPDYE5 (Blood) *4.51098808994539 + KL (Blood) *-5.79001413042372 |
| Calcium_mg.dL | 0.6 | MIOS (Blood) \| METTL27 (Blood) \| SREBF1 (Blood) | 9.04429378541027 +MIOS (Blood) *0.975925061845625 + METTL27 (Blood) *0.176272133075835 + SREBF1 (Blood) *-0.658990356157586 |
| Absolute.Neutrophil_k.uL | 0.59 | NTNG2 (Blood) \| TLE3 (Blood) \| | 0.207173484373919 +NTNG2 (Blood) * 1.2255813525448 + TLE3 (Blood) *2.95112923413471 |
| Cholesterol_ | 0.59 | AL353593.1 (Blood) \| GOLGA8A (Blood) \| SMC5 (Blood) | -8.89616538539591 +AL353593.1 (Blood) *35.1472237736851 + GOLGA8A (Blood) *40.9974581099568 + SMC5 (Blood) * 144.75178829258 |
| Triglyceride_ | 0.59 | MAP3K15 (Blood) \| SPDYE5 (Blood) \| KL (Blood) | 90.9925979507341 +MAP3K15 (Blood) *39.696698822413 + SPDYE5 (Blood) *22.0302935559495 + KL (Blood) *-29.0497443084864 |
| Alkaline.Phosphatase_IU.L | 0.59 | SH3YL1 (Blood) \| NAA38 (Blood) \| SYNM (Blood) | 58.2398378742039 +SH3YL1 (Blood) *-22.3047606118879 + NAA38 (Blood) *19.9891213359575 + SYNM (Blood) * 10.4119638636418 |
| MCHC_g.dL | 0.58 | DTX4 (Blood) \| LRIF1 (Plasma) \| DDX3Y (DBS) | 31.5919289067614 +DTX4 (Blood) *1.22187120717758 + LRIF1 (Plasma) *0.677253108836186 + DDX3Y (DBS) *0.344965207259182 |
| MPV_fl | 0.57 | TMCO3 (Blood) \| GKAP1 (Blood) \| LRRN1 (Blood) | 11.9329246913931 +TMCO3 (Blood) *-1.540745991263 + GKAP1 (Blood) *0.990929913508873 + LRRN1 (Blood) *-0.501948155611101 |
| Absolute.Lymphocyte_k.uL | 0.57 | OAZ2 (Blood) \| KCNE3 (Blood) \| | 3.86546727874129 +OAZ2 (Blood) *-1.270425943019 + KCNE3 (Blood) *-0.713214848990538 |
| T3.Uptake_. | 0.57 | ZNF469 (Blood) \| AC009533.1 (Blood) \| ING2 (Blood) | 29.793090781956 +ZNF469 (Blood) *4.25243087247019 + AC009533.1 (Blood) *-1.74462338232437 + ING2 (Blood) *-4.27258736309824 |
| TSH..High.Sensitivity_mU.L | 0.57 | ZNF100 (Blood) \| SNHG8 (Blood) \| TMCO6 (Blood) | 3.19874435967558 +ZNF100 (Blood) *0.885504781564805 + SNHG8 (Blood) *-0.895518070606725 + TMCO6 (Blood) *-1.47539482145919 |
| Absolute.Monocyte_k.uL | 0.56 | NAGA (Blood) \| ADA2 (Blood) \| | -0.0132838072590899 +NAGA (Blood) *0.31303328641343 + ADA2 (Blood) *0.212312268574206 |
| Protein..Total_g.dL | 0.55 | ITM2A (Blood) \| CDK2 (Blood) \| SNORA80A (Blood) | 6.42969507497666 +ITM2A (Blood) *0.48446965070971 + CDK2 (Blood) *0.578526892265614 + SNORA80A (Blood) *-0.297860482683532 |
| WBC_K.mm3 | 0.54 | GYPB (Blood) \| CDK8 (DBS) \| GYPE (Blood) | 8.75731192981126 +GYPB (Blood) *-1.19669632836611 + CDK8 (DBS) *-0.555271823408771 + GYPE (Blood) *-0.497538891339749 |
| RDW..sd._fl | 0.54 | CHCHD2P6 (Plasma) \| JAM3 (DBS) \| PLEKHA5 (Blood) | 36.4961904978741 +CHCHD2P6 (Plasma) * 1.18804830985846 + JAM3 (DBS) *1.40776378934959 + PLEKHA5 (Blood) *3.71863771779343 |
| Potassium_mmol.L | 0.54 | LRRC28 (Blood) \| AP003717.1 (Blood) \| CLEC11A (Blood) | 3.91480059363952 +LRRC28 (Blood) *0.615635316747977 + AP003717.1 (Blood) *-0.268337277773096 + CLEC11A (Blood) *0.168320177486421 |
| Absolute.Eosinophil_k.uL | 0.53 | CCT3 (DBS) \| CLC (Blood) \| TRIM37 (DBS) | 0.158025801580431 +CCT3 (DBS) *-0.0357543172038057 + CLC (Blood) *0.0420117960778139 + TRIM37 (DBS) *-0.0291472504478695 |
| Cholesterol.HDL.Ratio_ | 0.53 | NCBP2L (Blood) \| CNPY4 (Blood) \| | 4.91205323233742 +NCBP2L (Blood) *0.723951587039121 + CNPY4 (Blood) *-1.85894964453232 |
| Phosphorus..inorganic._mg.dL | 0.53 | IL18RAP (Blood) \| SMPD2 (Blood) \| SNORA20 (Blood) | 4.6242871887666 +IL18RAP (Blood) *-0.334249474991653 + SMPD2 (Blood) *-0.789192760531163 + SNORA20 (Blood) *-0.304324426243099 |
| GGT_IU.L | 0.53 | SERPINE1 (Blood) \| OTUD3 | -8.20472237821403 +SERPINE1 (Blood) *9.65320012119823 + |
| | | (Blood) \| SORBS2 (Blood) | OTUD3 (Blood) *15.2619055752769 + SORBS2 (Blood) *3.6777083142664 |
| MCV_fl | 0.52 | TMEM183A (Blood) \| AC092490.1 (Blood) \| DTX3 (Blood) | 96.407560101612 +TMEM183A (Blood) *-9.17520928654472 + AC092490.1 (Blood) *-1.40669741860889 + DTX3 (Blood) *5.29603408779261 |
| Non.HDL.Cholesterol_ | 0.51 | HGSNAT (Blood) \| ENSG00000233280 (Blood) \| AC027309.2 (Blood) | -33.7319304845757 +HGSNAT (Blood) *104.791759023732 + ENSG00000233280 (Blood) *59.6894397836695 + AC027309.2 (Blood) *20.267866956693 |
| Albumin_g.dL | 0.51 | KANSL3 (Blood) \| FNBP4 (Blood) \| COL9A2 (Blood) | 2.76592669611056 +KANSL3 (Blood) *0.886820024932328 + FNBP4 (Blood) *0.655112995219229 + COL9A2 (Blood) *0.256841598168667 |
| BUN_mg.dL | 0.5 | RFX2 (Blood) \| ALG1L10P (Blood) \| HIST2H2BA (Blood) | 14.8385962751296 +RFX2 (Blood) *-3.97673032999744 + ALG1L10P (Blood) *0.975798500220197 + HIST2H2BA (Blood) * 1.31765775405616 |
| Albumin..Globulin.Ratio_ | 0.5 | IL18BP (Blood) \| SNORA80A (Blood) \| SYCE1 (Blood) | 1.00809686565828 +IL18BP (Blood) *0.43690587433331 + SNORA80A (Blood) *0.213881752825225 + SYCE1 (Blood) *0.0879911614032978 |
| RDW..cv._. | 0.49 | NMT2 (Blood) \| PLEKHH2 (Blood) \| TMEM245 (Blood) | 9.68380709762748 +NMT2 (Blood) * 1.1639957216907 + PLEKHH2 (Blood) *0.286892303141383 + TMEM245 (Blood) *1.61864203340345 |
| MCH_pg | 0.48 | TMEM273 (Blood) \| IL1RAP (Blood) \| SMIM5 (Blood) | 32.3291274277572 +TMEM273 (Blood) *1.07159539254098 + IL1RAP (Blood) *-1.68802662235095 + SMIM5 (Blood) *-1.15265411525787 |
| Creatinine_mg.dL | 0.48 | USP9Y (Blood) | 0.791262056112685 +USP9Y (Blood) *0.121419890322682 |
| Globulin_g.dL | 0.48 | MYH3 (Blood) \| IL18BP (Blood) \| ABCG2 (DBS) | 3.55573198888647 +MYH3 (Blood) *-0.461465147890139 + IL18BP (Blood) *-0.518152000005631 +ABCG2 (DBS) *0.128213641962725 |
| RBC_m.mm3 | 0.47 | DDX3Y (Blood) | 4.48514538508006 +DDX3Y (Blood) *0.340768206800471 |
| Platelet.Count_k.mm3 | 0.47 | SLC37A2 (Blood) \| IGLV3-13 (Blood) \| | 337.931177295631 +SLC37A2 (Blood) *-87.7079726147655 + IGLV3-13 (Blood) *22.5377387049476 |
| T7.Index | 0.47 | IGHV3-33 (Blood) \| ZNF266 (Blood) \| ABHD17AP4 (Blood) | 2.87096861063423 +IGHV3-33 (Blood) *-0.224132194657099 + ZNF266 (Blood) *-0.612950925919992 + ABHD17AP4 (Blood) *-0.0997566798097242 |
| Sodium_mmol.L | 0.47 | BTRC (Blood) \| WASHC2C (Blood) \| AMD1P3 (Blood) | 138.883276573839 +BTRC (Blood) *3.39463427490849 + WASHC2C (Blood) *-2.77858809739364 + AMD1P3 (Blood) *0.798496161860985 |
| CO2_mmol.L | 0.47 | FAM157A (Blood) \| NFKB2 (Blood) \| | 31.4496060708057 +FAM157A (Blood) *-2.21544050441395 + NFKB2 (Blood) *-3.56645987757677 |
| Alaine.Aminotransferase_IU.L | 0.47 | RNASE3 (Blood) \| DEFA4 (Blood) \| | 14.4248577378265 +RNASE3 (Blood) *5.0882012572435 + DEFA4 (Blood) *4.65136914688879 |
| Hematocrit_. | 0.44 | NFYA (Blood) \| USP9Y (Blood) \| | 50.7828595935722 +NFYA (Blood) *-9.37767350069941 + USP9Y (Blood) * 1.43978771568548 |
| HDL.Cholesterol_ | 0.44 | SCARB1 (Blood) \| FLYWCH1 (Blood) \| NDUFS6 (Blood) | 82.7610301362919 +SCARB1 (Blood) *-15.370144223283 + FLYWCH1 (Blood) *8.21837672821879 + NDUFS6 (Blood) *-20.8961962050542 |
| Uric.Acid_mg.dL | 0.44 | PROS1 (Blood) | 3.14682258245291 +PROS1 (Blood) *1.61277932298865 |
| Glucose _mg.dL | 0.42 | SAR1B (DBS) \| HMGB1P1 (Blood) \| MPC2 (Blood) | 63.2945313509948 +SAR1B (DBS) *4.07249192713006 + HMGB1P1 (Blood) *5.43121538759866 + MPC2 (Blood) *22.1820621067802 |
| T3.Total_ng.dL | 0.41 | EBPL (Blood) \| MTPAP (Blood) \| NRROS (Blood) | 157.005257929307 +EBPL (Blood) * 19.3280952080184 + MTPAP (Blood) *-34.6142095073691 + NRROS (Blood) *-27.3385865176937 |
| Thyroxine..T4._ug.dL | 0.4 | CDCA8 (Plasma) \| PHKA1P1 (Plasma) \| IQCE (Blood) | 5.56382805834608 +CDCA8 (Plasma) *-0.828628892995684 + PHKA1P1 (Plasma) *0.380736434918838 + IQCE (Blood) *2.31407501016819 |
| Lactic.Dehydrogenase_IU.L | 0.4 | PITPNM3 (Blood) \| NUMA1 (Blood) \| RAB31 (Blood) | 108.780505731206 +PITPNM3 (Blood) *10.4819435495866 + NUMA1 (Blood) *64.8667532767841 + RAB31 (Blood) *-25.1238658063081 |
| Bilirubin..Total_mg.dL | 0.4 | ATXNT7L1 (Blood) \| MAGI2 (Blood) \| CH13L2 (Blood) | 0.832953190599145 +ATXN7L1 (Blood) *-0.547061215778317 + MAGI2 (Blood) *0.0638082975470622 + CHI3L2 (Blood) *0.132047306873859 |
| BUN.Creatine.Ratio_ | 0.39 | ALG1L10P (Blood) \| HIST2H2BA (Blood) \| | 12.4257435214487 +ALG1L10P (Blood) *1.91216977539833 + HIST2H2BA (Blood) * 1.63567334248823 |
| Osmolality..Calculated_mOsm .kg | 0.39 | EIF1AY (Blood) | 284.712173286834 +EIF1AY (Blood) *2.31346985184965 |
| Hemoglobin_g.dL | 0.37 | USP9Y (Blood) | 13.6628105965376 +USP9Y (Blood) *0.878879525161311 |
| Chloride_mmol.L | 0.33 | RMRP (Blood) \| PDK4 (Blood) \| SIRT7 (DBS) | 100.285307143487 +RMRP (Blood) *1.56514051132095 + PDK4 (Blood) *-1.10764371640888 + SIRT7 (DBS) *0.734799171039523 |
| Apartate.Aminotransferase_IU .L | 0.24 | PLIN5 (Blood) \| FBXO48 (Blood) \| | 20.1666477248498 +PLIN5 (Blood) *-4.53574445013183 + FBXO48 (Blood) *3.83345216930802 |

### EXAMPLES

It should be understood that while particular embodiments have been illustrated and described, various modifications can be made thereto without departing from the scope of the invention as will be apparent to those skilled in the art. Such changes and modifications are within the scope and teachings of this invention as defined in the claims appended hereto.

### 1: Sample Collection

Whole blood, plasma, and dried blood spot (DBS) samples were collected from 50 non-fasting individuals. Two sets of blood samples were collected on the same day. The set to be sent for analysis by Sonora Quest Laboratories contained collections of whole blood and plasma according to standard procedure. The set for analysis of RNA expression contained collections of whole blood, plasma, and DBS. Instead of collecting 8 ccs of blood, the total amount of blood for the second section was 1 cc. Blood was collected in blood collection tubes with K2EDTA. Plasma samples were produced by centrifuging the whole blood collected in K2EDTA tubes according to standard procedure.

Dried blood spot samples may be obtained using a finger-puncture technique in which a single drop of blood from the subject's finger was applied to a sample collection apparatus (i.e., RNA collection paper from FORTIUSBIO^{®}). The blood spot is allowed to dry on the FORTIUSBIO^{®} sample collection apparatus. A portion of the sample that has dried on the sample collection apparatus is then removed for nucleic acid extraction.

### 2: Measurement of Genes Detected in the Sample and Quantification of RNA Expression in the Sample

RNA, including mRNA, may be extracted using commercially available kits. RNA was extracted from whole blood, plasma, and dried blood spot samples using exoRNeasy (QIAGEN ^{®}, Germantown, MD) according to the manufacturer's instructions. The extracted RNA or mRNA was sequenced using the ILLUMINA^{®} system (San Diego, CA) to determine the RNA or mRNA expression level of each predictive gene. In various embodiments, mRNA may be sequenced using next-generation sequencing (NGS) to obtain raw sequencing data.

After the mRNA from the blood sample is sequenced, some embodiments provide methods of analyzing the data. For example, the analyzing steps of the methodology include steps such as processing the raw sequencing data/reads to remove information related to barcodes and adapters using technologies provided by Cutadapt and AlienTrimmer. Thereafter, the sequences can be aligned to a reference sequence using technologies such as STAR or Tophat. After alignment, the data can be quantitated to generate numerical estimates of each gene's expression or "counts" provided by technologies like FeatureCounts or htseq-count. For example, a number of copies or reads of a predictive gene in the sequencing data can be quantified or counted to determine a gene count. A gene count represents a relative expression level of the predictive gene in the blood sample and is independent of the volume of the blood sample. The gene count is a value that can then be used as an input into one or more bioinformatic analysis steps used to correlate the gene count to an output value of a blood test result.

Gene counts were obtained and normalized within each sample type for sequencing depth and then standardized for performing linear regression.

The normalization of gene counts reduces the impact of different sequencing length on the gene count. For example, when the total gene count of sample A is 1 million counts, and the total gene count for sample B is 1.3 million counts, the difference may mainly be attributed to technical variation and not a true biological difference. Accordingly, normalization is applied to the total gene counts of these samples so that the sequencing results of sample A can be compared to the sequencing results of sample B. A variety of algorithms for normalizing library size exist in the prior art, for example, DESeq2, and they may all be used for normalization the gene count in the methods of the invention.

The standardization of the gene count is a mathematical correction applied to ensure the variables of comparison are on the same scale. This step helps stabilize the results of any kind of machine learning. While gene counts do not need to be standardized, the step increases the accuracy of the blood test result determination. Any method of standardizing variables may be used. In one implementation, the gene counts are standardized by dividing each value by the root mean square of all the samples values for the given gene.

### 3: Blood Tests Results

The samples were sent to Sonora Quest for the analysis of the specific blood tests listed in Table 10.

**Table 10.**

| ***Test Category*** | **Panel** | **Test** | **Units** |
|---|---|---|---|
| ***Chemistry*** | **Thyroid** | T3 Uptake | % |
| | | Thyroxine (T4) | ug/dL |
| | | T7 Index | |
| | | T3 Total | ng/dL |
| | | TSH, High Sensitivity | mU/L |
| | **PSA (Males Only)** | PSA (total) | |
| | **Lipid Panel** | Cholesterol | |
| | | Triglyceride | |
| | | Cholesterol/HDL Ratio | |
| | | HDL Cholesterol | |
| | | Non-HDL Cholesterol | |
| | | LDL Cholesterol, | |
| | | Calculated | |
| | | VLDL Cholesterol | |
| | **Chemistry Panel, Basic** | Glucose | mg/dL |
| | | BUN | mg/dL |
| | | Creatinine | mg/dL |
| | | BUN/Creatine Ratio | |
| | | Uric Acid | mg/dL |
| | | Sodium | mmol/L |
| | | Potassium | mmol/L |
| | | Chloride | mmol/L |
| | | CO₂ | mmol/L |
| | | Anion Gap | mmol/L |
| | | Osmolality, Calculated | mOsm/kg |
| | | Protein, Total | g/dL |
| | | Albumin | g/dL |
| | | Globulin | g/dL |
| | | Albumin/ Globulin Ratio | |
| | | Calcium | mg/dL |
| | | Phosphorus (inorganic) | mg/dL |
| | | Alkaline Phosphatase | IU/L |
| | | GGT | IU/L |
| | | Alanine Aminotransferase | IU/L |
| | | Aspartate Aminotransferase | IU/L |
| | | Lactic Dehydrogenase | IU/L |
| | | Bilirubin, Total | mg/dL |
| ***Hematology*** | **CBC with Differential, with Platelet** | WBC | K/mm³ |
| | | RBC | m/mm³ |
| | | Hemoglobin | g/dL |
| | | Hematocrit | % |
| | | MCV | fl |
| | | MCH | pg |
| | | MCHC | g/dL |
| | | Platelet Count | k/mm³ |
| | | RDW (sd) | fl |
| | | RDW (cv) | % |
| | | MPV | fl |
| | | Segmented Neutrophils | % |
| | | Lymphocytes | % |
| | | Monocytes | % |
| | | Eosinophils | % |
| | | Basophils | % |
| | | Absolute Neutrophil | k/uL |
| | | Absolute Lymphocyte | k/uL |
| | | Absolute Monocyte | k/uL |
| | | Absolute Eosinophil | k/uL |
| | | Absolute Basophil | k/uL |
| | | Immature Granulocyte | % |
| | | Absolute Granulocyte | k/uL |

### 4: Regression Analysis

Simple linear regression was performed after removing any outliers by regressing each gene individually on each blood test. The single genes whose expression levels are most highly correlated values of standard blood chemistry tests (as measured by R² values) were noted in Tables 1-3.

Multiple linear regression was performed by considering up to 5 genes that could be used on the regression model. Outliers were imputed with the mean, and two rounds of feature selection were performed to identify genes of interest for each blood test. The first round selected the 5 highest (3 for DBS) scoring genes based on a univariate F-test followed by a second round where genes were potentially removed based on the Akaike information criterion. This process was performed for each sample type separately and in combination by considering whole blood with plasma as well as all three sample types together. A combination of sample types was created by allowing genes from any of the included sample types to be selected during the first round of feature selection.

## Claims

1. A method of performing a blood test, comprising:
extracting an RNA from a dried blood spot or blood plasma;
selecting a predictive gene, wherein an mRNA level of the predictive gene in the dried blood spot or blood plasma relates to a target blood component;
determining the mRNA level of the predictive gene in the dried blood spot or blood plasma; and
converting the mRNA level into a blood test result of the target blood component;
wherein the mRNA level is determined using RNA sequencing, the target blood component is Prostate-Specific Antigen (PSA_total); and the predictive gene for the dried blood spot is selected from the group consisting of: CTC-265F19.1, ADAM9, RAB11FIP5, SNAPC4, and LMNA; and the predictive gene for the blood plasma is selected from the group consisting of: HNRNPA3P3, GTF3A, RP11-342M1.6, HNRNPLP2, and RPS11P5.

2. The method of Claim 1, further comprising determining the quality of the dried blood spot and selecting the predictive gene based on the quality.

3. The method of any one of Claims1 or 2, wherein the mRNA level is determined using next-generation sequencing and normalized to a normalized gene count using a DESeq2 algorithm.

4. The method of any one of Claims 1-3, wherein the blood test result is reported as: an amount of the target blood component, a concentration of the target blood component; a volume of the target blood component, a distribution of the target blood component; a ratio between the target blood component and a second target blood component; or combinations thereof.

5. The method of any one of Claims 1-4, wherein converting the mRNA level into the blood test result uses the following formula: blood test result = C + C₁*(gene), C and C₁ are constants, and (gene) represents the normalized gene count of the predictive gene.

6. The method of any of on Claims 1-5, wherein the predictive gene for the dried blood spot is selected from the group consisting of: CTC-265F19.1, ADAM9, RAB11FIP5, SNAPC4, and LMNA; and C is between 0.39 and 0.48, and C₁ is between 0.47 and 0.58 for CTC-265F19.1; C is between 0.39 and 0.48, and C₁ is between 1.5 and 1.9 for ADAM9; C is between 0.40 and 0.49, and C₁ is between 0.53 and 0.65 for RAB11FIP5; C is between 0.40 and 0.49, and C₁ is between 0.55 and 0.67 for SNAPC4; and C is between 0.37 and 0.45, and C₁ is between 0.31 and 0.38 for LMNA.

7. The method of any of on Claims 1-5, wherein the predictive gene for the blood plasma is selected from the group consisting of: HNRNPA3P3, GTF3A, RP11-342M1.6, HNRNPLP2, and RPS11P5; and C is between 0.19 and 0.23, and C₁ is between 0.42 and 0.52 for HNRNPA3P3; C is between -0.41 and -0.34, and C₁ is between 0.9 and 1.1 for GTF3A; C is between 0.36 and 0.44, and C₁ is between 0.29 and 0.36 for RP11-342M1.6; C is between 0.30 and 0.36, and C₁ is between 0.29 and 0.35 for HNRNPLP2, and C is between 0.22 and 0.27, and C₁ is between 0.44 and 0.54 for RPS11P5.

## Patentansprüche

1. Verfahren zum Durchführen eines Bluttests, umfassend:
Extraktion einer RNA aus einem getrockneten Blutfleck oder Blutplasma;
Auswählen eines prädiktiven Gens, wobei ein mRNA-Spiegel des prädiktiven Gens in dem getrockneten Blutfleck oder Blutplasma mit einer Zielblutkomponente in Beziehung steht;
Bestimmen des mRNA-Spiegels des prädiktiven Gens in dem getrockneten Blutfleck oder Blutplasma; und
Umwandeln des mRNA-Spiegels in ein Bluttestergebnis der Zielblutkomponente;
wobei der mRNA-Spiegel durch RNA-Sequenzierung bestimmt wird, die Zielblutkomponente Prostata-spezifisches Antigen (PSA_total) ist und das prädiktive Gen für den getrockneten Blutfleck aus der Gruppe ausgewählt ist, bestehend aus: CTC-265F19.1, ADAM9, RAB11FIP5, SNAPC4 und LMNA; und das prädiktive Gen für das Blutplasma ausgewählt ist aus der Gruppe, bestehend aus: HNRNPA3P3, GTF3A, RP11-342M1.6, HNRNPLP2 und RPS11P5.

2. Verfahren nach Anspruch 1, ferner umfassend das Bestimmen der Qualität des getrockneten Blutflecks und das Auswählen des prädiktiven Gens basierend auf der Qualität.

3. Verfahren nach einem der Ansprüche 1 oder 2, wobei der mRNA-Spiegel unter Verwendung der Sequenzierung der nächsten Generation bestimmt und unter Verwendung eines DESeq2-Algorithmus auf eine normalisierte Genanzahl normiert wird.

4. Verfahren nach einem der Ansprüche 1 bis 3, wobei das Bluttestergebnis angegeben wird als: eine Menge der Zielblutkomponente, eine Konzentration der Zielblutkomponente, ein Volumen der Zielblutkomponente, eine Verteilung der Zielblutkomponente, ein Verhältnis zwischen der Zielblutkomponente und einer zweiten Zielblutkomponente, oder Kombinationen davon.

5. Verfahren nach einem der Ansprüche 1 bis 4, wobei das Umwandeln des mRNA-Niveaus in das Bluttestergebnis die folgende Formel verwendet: Bluttestergebnis = C + C₁*(Gen), wobei C und C₁ Konstanten sind und (Gen) die normalisierte Genanzahl des prädiktiven Gens darstellt.

6. Verfahren nach einem der Ansprüche 1 bis 5, wobei das prädiktive Gen für den getrockneten Blutfleck ausgewählt ist aus der Gruppe, bestehend aus: CTC-265F19.1, ADAM9, RAB11FIP5, SNAPC4 und LMNA; und C zwischen 0,39 und 0,48 liegt und C₁ zwischen 0,47 und 0,58 für CTC-265F19.1 liegt; C zwischen 0,39 und 0,48 liegt und C₁ zwischen 1,5 und 1,9 für ADAM9 liegt; C zwischen 0,40 und 0,49 und C₁ zwischen 0,53 und 0,65 für RAB11FIP5; C zwischen 0,40 und 0,49 und C₁ zwischen 0,55 und 0,67 für SNAPC4; und C zwischen 0,37 und 0,45 und C₁ zwischen 0,31 und 0,38 für LMNA.

7. Verfahren nach einem der Ansprüche 1 bis 5, wobei das prädiktive Gen für das Blutplasma ausgewählt ist aus der Gruppe, bestehend aus: HNRNPA3P3, GTF3A, RP11-342M1.6, HNRNPLP2 und RPS11P5; und C zwischen 0,19 und 0,23 liegt und C₁ zwischen 0,42 und 0,52 für HNRNPA3P3 liegt; C zwischen -0,41 und -0,34 liegt und C₁ zwischen 0,9 und 1,1 für GTF3A liegt; C zwischen 0,36 und 0,44, und C₁ zwischen 0,29 und 0,36 für RP11-342M1.6; C zwischen 0,30 und 0,36, und C₁ zwischen 0,29 und 0,35 für HNRNPLP2, und C zwischen 0,22 und 0,27, und C₁ zwischen 0,44 und 0,54 für RPS11P5.

## Revendications

1. Procédé de réalisation d'une analyse de sang, comprenant :
l'extraction d'un ARN à partir d'une goutte de sang séché ou d'un plasma sanguin ;
la sélection d'un gène prédictif, dans lequel un niveau d'ARNm du gène prédictif dans la goutte de sang séché ou le plasma sanguin se rapporte à un composant sanguin cible ;
la détermination du niveau d'ARNm du gène prédictif dans la goutte de sang séché ou le plasma sanguin ; et
la conversion du niveau d'ARNm en un résultat d'analyse de sang du composant sanguin cible ;
dans lequel le niveau d'ARNm est déterminé à l'aide du séquençage de l'ARN, le composant sanguin cible est l'antigène prostatique spécifique (PSA_total) ; et le gène prédictif pour la goutte de sang séché est sélectionné dans le groupe constitué de : CTC-265F19.1, ADAM9, RAB11FIP5, SNAPC4, et LMNA ; et le gène prédictif pour le plasma sanguin est sélectionné dans le groupe consistant de : HNRNPA3P3, GTF3A, RP11-342M1.6, HNRNPLP2, et RPS11P5.

2. Procédé selon la revendication 1, comprenant en outre la détermination de la qualité de la goutte de sang séché et la sélection du gène prédictif sur la base de cette qualité.

3. Procédé selon l'une quelconque des revendications 1 ou 2, dans lequel le niveau d'ARNm est déterminé à l'aide d'un séquençage de nouvelle génération et normalisé par rapport à un nombre normalisé de gènes à l'aide d'un algorithme DESeq2.

4. Procédé selon l'une quelconque des revendications 1 à 3, dans lequel le résultat de l'analyse de sang est rapporté sous la forme : d'une quantité du composant sanguin cible, d'une concentration du composant sanguin cible ; d'un volume du composant sanguin cible, d'une distribution du composant sanguin cible ; d'un rapport entre le composant sanguin cible et un second composant sanguin cible ; ou des combinaisons de ceux-ci.

5. Procédé selon l'une quelconque des revendications 1 à 4, dans lequel la conversion du niveau d'ARNm en résultat d'analyse de sang utilise la formule suivante : résultat d'analyse de sang = C + C₁*(gène), C et C₁ sont des constantes, et (gène) représente le nombre normalisé de gènes du gène prédictif.

6. Procédé selon l'une quelconque des revendications 1 à 5, dans lequel le gène prédictif pour la goutte de sang séché est sélectionné dans le groupe constitué de : CTC-265F19.1, ADAM9, RAB11FIP5, SNAPC4, et LMNA ; et C est compris entre 0,39 et 0,48, et C₁ est compris entre 0,47 et 0,58 pour CTC-265F19.1 ; C est compris entre 0,39 et 0,48, et C₁ est compris entre 1,5 et 1,9 pour ADAM9 ; C est compris entre 0.40 et 0,49, et C₁ est compris entre 0,53 et 0,65 pour RAB11FIP5 ; C entre 0,40 et 0,49, et C₁ est compris entre 0,55 et 0,67 pour SNAPC4 ; et C entre 0,37 et 0,45, et C₁ est compris entre 0,31 et 0,38 pour LMNA.

7. Procédé selon l'une quelconque des revendications 1 à 5, dans lequel le gène prédictif pour le plasma sanguin est sélectionné dans le groupe constitué de : HNRNPA3P3, GTF3A, RP11-342M1.6, HNRNPLP2, et RPS11P5 ; et C est compris entre 0,19 et 0,23, et C₁ est compris entre 0,42 et 0,52 pour HNRNPA3P3 ; C est compris entre -0,41 et -0,34, et C₁ est compris entre 0,9 et 1,1 pour GTF3A ; C est compris entre 0.36 et 0,44, et C₁ est compris entre 0,29 et 0,36 pour RP11-342M1.6 ; C est compris entre 0,30 et 0,36, et C₁ est compris entre 0,29 et 0,35 pour HNRNPLP2, et C est compris entre 0,22 et 0,27, et C₁ est compris entre 0,44 et 0,54 pour RPS11P5.
